# EUROPEAN PATENT SPECIFICATION

(11) **EP 1 651 166 B1**
(45) Date of publication and mention of the grant of the patent: **17.02.2010**
(21) Application number: 04778847.6
(22) Date of filing: 22.07.2004
(51) Int. Cl.: A61K 39/085, C07K 14/31, C12N 15/31, C12N 15/81

(54) **POLYPEPTIDES FOR INDUCING A PROTECTIVE IMMUNE RESPONSE AGAINST STAPHYLOCOCCUS AUREUS**
POLYPEPTIDE ZUR INDUKTION EINER SCHÜTZENDEN IMMUNANTWORT GEGEN STAPHYLOCOCCUS AUREUS
POLYPEPTIDES POUR L'INDUCTION D'UNE REPONSE IMMUNITAIRE DE PROTECTION CONTRE LE STAPHYLOCOCCUS AUREUS

(30) Priority: 24.07.2003 US 489840 P; 14.11.2003 US 520115 P
(43) Date of publication of application: 03.05.2006
(73) Proprietor: Merck & Co., Inc., Rahway, New Jersey 07065-0907 (US)
(72) Inventor: ANDERSON, Annaliesa, S., Rahway, New Jersey 07065-0907 (US); JANSEN, Kathrin Ute, Rahway, New Jersey 07065-0907 (US); KELLY, Rosemarie, Rahway, New Jersey 07065-0907 (US); SCHULTZ, Loren, D., Rahway, New Jersey 07065-0907 (US); MONTGOMERY, Donna, L., Rahway, New Jersey 07065-0907 (US); MCCLEMENTS, William, L., Rahway, New Jersey 07065-0907 (US)
(74) Representative: Horgan, James Michael Frederic
(86) International application number: PCT/US2004/023523
(87) International publication number: WO 2005/009379

(56) References cited:
- WO-A-02/059148
- WO-A-02/102829
- WO-A2-02/059148
- TAYLOR J.M. ET AL: 'Transferrin binding in Staphylococcus aureus: involvement of a cell wall-anchored protein' MOL. MICROBIOL. vol. 43, no. 6, 2002, pages 1603 - 1614, XP003004471

### SEQUENCE LISTING

<110> Merck & Co, Inc,
<120> POLYPEPTlDES FOR INDUCING A PROTECTIVE
   IMMUNE RESPONSE AGAINST STAPHYLOCOCCUS AURUS
<130> 21669Y PCT
<150> 60/489, 840
   <151> 2003-07-24
<150> 60/520, 115
   <151> 2003-11-14
<160> 107
<170> FastSEQ for windows version 4.0
<210> 1
   <211> 446
   <212> PRT
   <213> Artificial Sequence
<220>
   <223> ORF0657nI with amino terminus methionine
<400> 1
   Met Ala Glu Glu Thr Gly Gly Thr Asn Thr Glu Ala Gln Pro Lys Thr
    1               5                  10                  15
   Glu Ala Val Ala Ser Pro Thr Thr Thr Ser Glu Lys Ala Pro Glu Thr
               20                  25                  30
   Lys Pro Val Ala Asn Ala Val Ser Val Ser Asn Lys Glu Val Glu Ala
           35                  40                  45
   Pro Thr Ser Glu Thr Lys Glu Ala Lys Glu Val Lys Glu Val Lys Ala
       50                  55                  60
   Pro Lys Glu Thr Lys Glu Val Lys Pro Ala Ala Lys Ala Thr Asn Asn
   65                  70                  75                  80
   Thr Tyr Pro Ile Leu Asn Gln Glu Leu Arg Glu Ala Ile Lys Asn Pro
                   85                  90                  95
   Ala Ile Lys Asp Lys Asp His Ser Ala Pro Asn Ser Arg Pro Ile Asp
               100                 105                 110
   Phe Glu Met Lys Lys Lys Asp Gly Thr Gln Gln Phe Tyr His Tyr Ala
           115                 120                 125
   Ser Ser Val Lys Pro Ala Arg Val Ile Phe Thr Asp Ser Lys Pro Glu
       130                 135                 140
   Ile Glu Leu Gly Leu Gln Ser Gly Gln Phe Trp Arg Lys Phe Glu Val
   145                 150                 155                 160
   Tyr Glu Gly Asp Lys Lys Leu Pro Ile Lys Leu Val Ser Tyr Asp Thr
                   165                 170                 175
   Val Lys Asp Tyr Ala Tyr Ile Arg Phe Ser Val Ser Asn Gly Thr Lys
               180                 185                 190
   Ala Val Lys Ile Val Ser Ser Thr His Phe Asn Asn Lys Glu Glu Lys
           195                 200                 205
   Tyr Asp Tyr Thr Leu Met Glu Phe Ala Gln Pro Ile Tyr Asn Ser Ala
   210 215 220
   Asp Lys Phe Lys Thr Glu Glu Asp Tyr Lys Ala Glu Lys Leu Leu Ala
   225 230 235 240
   Pro Tyr Lys Lys Ala Lys Thr Leu Glu Arg Gln Val Tyr Glu Leu Asn
   245 250 255
   Lys Ile Gln Asp Lys Leu Pro Glu Lys Leu Lys Ala Glu Tyr Lys Lys
   260 265 270
   Lys Leu Glu Asp Thr Lys Lys Ala Leu Asp Glu Gln Val Lys Ser Ala
   275 280 285
   Ile Thr Glu Phe Gln Asn Val Gln Pro Thr Asn Glu Lys Met Thr Asp
   290 295 300
   Leu Gln Asp Thr Lys Tyr Val Val Tyr Glu Ser Val Glu Asn Asn Glu
   305 310 315 320
   Ser Met Met Asp Thr Phe Val Lys His Pro Ile Lys Thr Gly Met Leu
   325 330 335
   Asn Gly Lys Lys Tyr Met Val Met Glu Thr Thr Asn Asp Asp Tyr Trp
   340 345 350
   Lys Asp Phe Met Val Glu Gly Gln Arg Val Arg Thr Ile Ser Lys Asp
   355 360 365
   Ala Lys Asn Asn Thr Arg Thr Ile Ile Phe Pro Tyr Val Glu Gly Lys
   370 375 380
   Thr Leu Tyr Asp Ala Ile Val Lys Val His Val Lys Thr Ile Asp Tyr
   385 390 395 400
   Asp Gly Gln Tyr His Val Arg Ile Val Asp Lys Glu Ala Phe Thr Lys
   405 410 415
   Ala Asn Thr Asp Lys Ser Asn Lys Lys Glu Gln Gln Asp Asn Ser Ala
   420 425 430
   Lys Lys Glu Ala Thr Pro Ala Thr Pro Ser Lys Pro Thr Pro
   435 440 445
<210> 2
   <211> 645
   <212> PRT
   <213> S. aureus
<400> 2
   Met Asn Lys Gln Gln Lys Glu Phe Lys Ser Phe Tyr Ser Ile Arg Lys
   1 5 10 15
   Ser Ser Leu Gly Val Ala Ser Val Ala Ile Ser Thr Leu Leu Leu Leu
   20 25 30
   Met Ser Asn Gly Glu Ala Gln Ala Ala Ala Glu Glu Thr Gly Gly Thr
   35 40 45
   Asn Thr Glu Ala Gln Pro Lys Thr Glu Ala Val Ala Ser Pro Thr Thr
   50 55 60
   Thr Ser Glu Lys Ala Pro Glu Thr Lys Pro Val Ala Asn Ala Val Ser
   65 70 75 80
   Val Ser Asn Lys Glu Val Glu Ala Pro Thr Ser Glu Thr Lys Glu Ala
   85 90 95
   Lys Glu Val Lys Glu Val Lys Ala Pro Lys Glu Thr Lys Glu Val Lys
   100 105 110
   Pro Ala Ala Lys Ala Thr Asn Asn Thr Tyr Pro Ile Leu Asn Gln Glu
   115 120 125
   Leu Arg Glu Ala Ile Lys Asn Pro Ala Ile Lys Asp Lys Asp His Ser
   130 135 140
   Ala Pro Asn Ser Arg Pro Ile Asp Phe Glu Met Lys Lys Lys Asp Gly
   145                 150                 155                 160
   Thr Gln Gln Phe Tyr His Tyr Ala Ser Ser Val Lys Pro Ala Arg Val
                   165                 170                 175
   Ile Phe Thr Asp Ser Lys Pro Glu Ile Glu Leu Gly Leu Gln Ser Gly
               180                 185                 190
   Gln Phe Trp Arg Lys Phe Glu Val Tyr Glu Gly Asp Lys Lys Leu Pro
           195                 200                 205
   Ile Lys Leu Val Ser Tyr Asp Thr Val Lys Asp Tyr Ala Tyr Ile Arg
       210                 215                 220
   Phe Ser Val Ser Asn Gly Thr Lys Ala Val Lys Ile Val Ser Ser Thr
   225                 230                 235                 240
   His Phe Asn Asn Lys Glu Glu Lys Tyr Asp Tyr Thr Leu Met Glu Phe
                   245                 250                 255
   Ala Gln Pro Ile Tyr Asn Ser Ala Asp Lys Phe Lys Thr Glu Glu Asp
               260                 265                 270
   Tyr Lys Ala Glu Lys Leu Leu Ala Pro Tyr Lys Lys Ala Lys Thr Leu
           275                 280                 285
   Glu Arg Gln Val Tyr Glu Leu Asn Lys Ile Gln Asp Lys Leu Pro Glu
       290                 295                 300
   Lys Leu Lys Ala Glu Tyr Lys Lys Lys Leu Glu Asp Thr Lys Lys Ala
   305                 310                 315                 320
   Leu Asp Glu Gln Val Lys Ser Ala Ile Thr Glu Phe Gln Asn Val Gln
                   325                 330                 335
   Pro Thr Asn Glu Lys Met Thr Asp Leu Gln Asp Thr Lys Tyr Val Val
               340                 345                 350
   Tyr Glu Ser Val Glu Asn Asn Glu Ser Met Met Asp Thr Phe Val Lys
           355                 360                 365
   His Pro Ile Lys Thr Gly Met Leu Asn Gly Lys Lys Tyr Met Val Met
       370                 375                 380
   Glu Thr Thr Asn Asp Asp Tyr Trp Lys Asp Phe Met Val Glu Gly Gln
   385                 390                 395                 400
   Arg Val Arg Thr Ile Ser Lys Asp Ala Lys Asn Asn Thr Arg Thr Ile
                   405                 410                 415
   Ile Phe Pro Tyr Val Glu Gly Lys Thr Leu Tyr Asp Ala Ile Val Lys
               420                 425                 430
   Val His Val Lys Thr Ile Asp Tyr Asp Gly Gln Tyr His Val Arg Ile
           435                 440                 445
   Val Asp Lys Glu Ala Phe Thr Lys Ala Asn Thr Asp Lys Ser Asn Lys
       450                 455                 460
   Lys Glu Gln Gln Asp Asn Ser Ala Lys Lys Glu Ala Thr Pro Ala Thr
   465                 470                 475                 480
   Pro Ser Lys Pro Thr Pro Ser Pro Val Glu Lys Glu Ser Gln Lys Gln
                   485                 490                 495
   Asp Ser Gln Lys Asp Asp Asn Lys Gln Leu Pro Ser Val Glu Lys Glu
               500                 505                 510
   Asn Asp Ala Ser Ser Glu Ser Gly Lys Asp Lys Thr Pro Ala Thr Lys
           515                 520                 525
   Pro Thr Lys Gly Glu Val Glu Ser Ser Ser Thr Thr Pro Thr Lys Val
       530                 535                 540
   Val Ser Thr Thr Gln Asn Val Ala Lys Pro Thr Thr Ala Ser Ser Lys
   545                 550                 555                 560
   Thr Thr Lys Asp Val Val Gln Thr Ser Ala Gly Ser Ser Glu Ala Lys
                   565                 570                 575
   Asp Ser Ala Pro Leu Gln Lys Ala Asn Ile Lys Asn Thr Asn Asp Gly
   580 585 590
   His Thr Gln Ser Gln Asn Asn Lys Asn Thr Gln Glu Asn Lys Ala Lys
   595 600 605
   Ser Leu Pro Gln Thr Gly Glu Glu Ser Asn Lys Asp Met Thr Leu Pro
   610 615 620
   Leu Met Ala Leu Leu Ala Leu Ser Ser Ile Val Ala Phe Val Leu Pro
   625 630 635 640
   Arg Lys Arg Lys Asn
   645
<210> 3
   <211> 569
   <212> PRT
   <213> Artificial Sequence
<220>
   <223> ORF0657nH with amino terminus methionine
<400> 3
   Met Ala Glu Glu Thr Gly Gly Thr Asn Thr Glu Ala Gln Pro Lys Thr
   1 5 10 15
   Glu Ala Val Ala Ser Pro Thr Thr Thr Ser Glu Lys Ala Pro Glu Thr
   20 25 30
   Lys Pro Val Ala Asn Ala Val Ser Val Ser Asn Lys Glu Val Glu Ala
   35 40 45
   Pro Thr Ser Glu Thr Lys Glu Ala Lys Glu Val Lys Glu Val Lys Ala
   50 55 60
   Pro Lys Glu Thr Lys Glu Val Lys Pro Ala Ala Lys Ala Thr Asn Asn
   65 70 75 80
   Thr Tyr Pro Ile Leu Asn Gln Glu Leu Arg Glu Ala Ile Lys Asn Pro
   85 90 95
   Ala Ile Lys Asp Lys Asp His Ser Ala Pro Asn Ser Arg Pro Ile Asp
   100 105 110
   Phe Glu Met Lys Lys Lys Asp Gly Thr Gln Gln Phe Tyr His Tyr Ala
   115 120 125
   Ser Ser Val Lys Pro Ala Arg Val Ile Phe Thr Asp Ser Lys Pro Glu
   130 135 140
   Ile Glu Leu Gly Leu Gln Ser Gly Gln Phe Trp Arg Lys Phe Glu Val
   145 150 155 160
   Tyr Glu Gly Asp Lys Lys Leu Pro Ile Lys Leu Val Ser Tyr Asp Thr
   165 170 175
   Val Lys Asp Tyr Ala Tyr Ile Arg Phe Ser Val Ser Asn Gly Thr Lys
   180 185 190
   Ala Val Lys Ile Val Ser Ser Thr His Phe Asn Asn Lys Glu Glu Lys
   195 200 205
   Tyr Asp Tyr Thr Leu Met Glu Phe Ala Gln Pro Ile Tyr Asn Ser Ala
   210 215 220
   Asp Lys Phe Lys Thr Glu Glu Asp Tyr Lys Ala Glu Lys Leu Leu Ala
   225 230 235 240
   Pro Tyr Lys Lys Ala Lys Thr Leu Glu Arg Gln Val Tyr Glu Leu Asn
   245 250 255
   Lys Ile Gln Asp Lys Leu Pro Glu Lys Leu Lys Ala Glu Tyr Lys Lys
   260 265 270
   Lys Leu Glu Asp Thr Lys Lys Ala Leu Asp Glu Gln Val Lys Ser Ala
           275                 280                 285
   Ile Thr Glu Phe Gln Asn Val Gln Pro Thr Asn Glu Lys Met Thr Asp
       290                 295                 300
   Leu Gln Asp Thr Lys Tyr Val Val Tyr Glu Ser Val Glu Asn Asn Glu
   305                 310                 315                 320
   Ser Met Met Asp Thr Phe Val Lys His Pro Ile Lys Thr Gly Met Leu
                   325                 330                 335
   Asn Gly Lys Lys Tyr Met Val Met Glu Thr Thr Asn Asp Asp Tyr Trp
               340                 345                 350
   Lys Asp Phe Met Val Glu Gly Gln Arg Val Arg Thr Ile Ser Lys Asp
           355                 360                 365
   Ala Lys Asn Asn Thr Arg Thr Ile Ile Phe Pro Tyr Val Glu Gly Lys
       370                 375                 380
   Thr Leu Tyr Asp Ala Ile Val Lys Val His Val Lys Thr Ile Asp Tyr
   385                 390                 395                 400
   Asp Gly Gln Tyr His Val Arg Ile Val Asp Lys Glu Ala Phe Thr Lys
                   405                 410                 415
   Ala Asn Thr Asp Lys Ser Asn Lys Lys Glu Gln Gln Asp Asn Ser Ala
               420                 425                 430
   Lys Lys Glu Ala Thr Pro Ala Thr Pro Ser Lys Pro Thr Pro Ser Pro
           435                 440                 445
   Val Glu Lys Glu Ser Gln Lys Gln Asp Ser Gln Lys Asp Asp Asn Lys
       450                 455                 460
   Gln Leu Pro Ser Val Glu Lys Glu Asn Asp Ala Ser Ser Glu Ser Gly
   465                 470                 475                 480
   Lys Asp Lys Thr Pro Ala Thr Lys Pro Thr Lys Gly Glu Val Glu Ser
                   485                 490                 495
   Ser Ser Thr Thr Pro Thr Lys Val Val Ser Thr Thr Gln Asn Val Ala
               500                 505                 510
   Lys Pro Thr Thr Ala Ser Ser Lys Thr Thr Lys Asp Val Val Gln Thr
           515                 520                 525
   Ser Ala Gly Ser Ser Glu Ala Lys Asp Ser Ala Pro Leu Gln Lys Ala
       530                 535                 540
   Asn Ile Lys Asn Thr Asn Asp Gly His Thr Gln Ser Gln Asn Asn Lys
   545                 550                 555                 560
   Asn Thr Gln Glu Asn Lys Ala Lys Ser
                   565
<210> 4
   <211> 570
   <212> PRT
   <213> Artificial Sequence
<220>
   <223> ORF0657nH with amino terminus methionine-glycine
<400> 4
   Met Gly Ala Glu Glu Thr Gly Gly Thr Asn Thr Glu Ala Gln Pro Lys
   1               5                   10                  15
   Thr Glu Ala Val Ala Ser Pro Thr Thr Thr Ser Glu Lys Ala Pro Glu
               20                  25                  30
   Thr Lys Pro Val Ala Asn Ala Val Ser Val Ser Asn Lys Glu Val Glu
           35                  40                  45
   Ala Pro Thr Ser Glu Thr Lys Glu Ala Lys Glu Val Lys Glu Val Lys
   50 55 60
   Ala Pro Lys Glu Thr Lys Glu Val Lys Pro Ala Ala Lys Ala Thr Asn
   65 70 75 80
   Asn Thr Tyr Pro Ile Leu Asn Gln Glu Leu Arg Glu Ala Ile Lys Asn
   85 90 95
   Pro Ala Ile Lys Asp Lys Asp His Ser Ala Pro Asn Ser Arg Pro Ile
   100 105 110
   Asp Phe Glu Met Lys Lys Lys Asp Gly Thr Gln Gln Phe Tyr His Tyr
   115 120 125
   Ala Ser Ser Val Lys Pro Ala Arg Val Ile Phe Thr Asp Ser Lys Pro
   130 135 140
   Glu Ile Glu Leu Gly Leu Gln Ser Gly Gln Phe Trp Arg Lys Phe Glu
   145 150 155 160
   Val Tyr Glu Gly Asp Lys Lys Leu Pro Ile Lys Leu Val Ser Tyr Asp
   165 170 175
   Thr Val Lys Asp Tyr Ala Tyr Ile Arg Phe Ser Val Ser Asn Gly Thr
   180 185 190
   Lys Ala Val Lys Ile Val Ser Ser Thr His Phe Asn Asn Lys Glu Glu
   195 200 205
   Lys Tyr Asp Tyr Thr Leu Met Glu Phe Ala Gln Pro Ile Tyr Asn Ser
   210 215 220
   Ala Asp Lys Phe Lys Thr Glu Glu Asp Tyr Lys Ala Glu Lys Leu Leu
   225 230 235 240
   Ala Pro Tyr Lys Lys Ala Lys Thr Leu Glu Arg Gln Val Tyr Glu Leu
   245 250 255
   Asn Lys Ile Gln Asp Lys Leu Pro Glu Lys Leu Lys Ala Glu Tyr Lys
   260 265 270
   Lys Lys Leu Glu Asp Thr Lys Lys Ala Leu Asp Glu Gln Val Lys Ser
   275 280 285
   Ala Ile Thr Glu Phe Gln Asn Val Gln Pro Thr Asn Glu Lys Met Thr
   290 295 300
   Asp Leu Gln Asp Thr Lys Tyr Val Val Tyr Glu Ser Val Glu Asn Asn
   305 310 315 320
   Glu Ser Met Met Asp Thr Phe Val Lys His Pro Ile Lys Thr Gly Met
   325 330 335
   Leu Asn Gly Lys Lys Tyr Met Val Met Glu Thr Thr Asn Asp Asp Tyr
   340 345 350
   Trp Lys Asp Phe Met Val Glu Gly Gln Arg Val Arg Thr Ile Ser Lys
   355 360 365
   Asp Ala Lys Asn Asn Thr Arg Thr Ile Ile Phe Pro Tyr Val Glu Gly
   370 375 380
   Lys Thr Leu Tyr Asp Ala Ile Val Lys Val His Val Lys Thr Ile Asp
   385 390 395 400
   Tyr Asp Gly Gln Tyr His Val Arg Ile Val Asp Lys Glu Ala Phe Thr
   405 410 415
   Lys Ala Asn Thr Asp Lys Ser Asn Lys Lys Glu Gln Gln Asp Asn Ser
   420 425 430
   Ala Lys Lys Glu Ala Thr Pro Ala Thr Pro Ser Lys Pro Thr Pro Ser
   435 440 445
   Pro Val Glu Lys Glu Ser Gln Lys Gln Asp Ser Gln Lys Asp Asp Asn
   450 455 460
   Lys Gln Leu Pro Ser Val Glu Lys Glu Asn Asp Ala Ser Ser Glu Ser
   465 470 475 480
   Gly Lys Asp Lys Thr Pro Ala Thr Lys Pro Thr Lys Gly Glu Val Glu
                   485                 490                 495
   Ser Ser Ser Thr Thr Pro Thr Lys Val Val Ser Thr Thr Gln Asn Val
               500                 505                 510
   Ala Lys Pro Thr Thr Ala Ser Ser Lys Thr Thr Lys Asp Val Val Gln
           515                 520                 525
   Thr Ser Ala Gly Ser Ser Glu Ala Lys Asp Ser Ala Pro Leu Gln Lys
       530                 535                 540
   Ala Asn Ile Lys Asn Thr Asn Asp Gly His Thr Gln Ser Gln Asn Asn
   545                 550                 555                 560
   Lys Asn Thr Gln Glu Asn Lys Ala Lys Ser
                   565                 570
<210> 5
   <211> 447
   <212> PRT
   <213> Artificial Sequence
<220>
   <223> ORF0657nH with amino terminus methionine-glycine
<400> 5
   Met Gly Ala Glu Glu Thr Gly Gly Thr Asn Thr Glu Ala Gln Pro Lys
    1               5                  10                  15
   Thr Glu Ala Val Ala Ser Pro Thr Thr Thr Ser Glu Lys Ala Pro Glu
               20                  25                  30
   Thr Lys Pro Val Ala Asn Ala Val Ser Val Ser Asn Lys Glu Val Glu
           35                  40                  45
   Ala Pro Thr Ser Glu Thr Lys Glu Ala Lys Glu Val Lys Glu Val Lys
       50                  55                  60
   Ala Pro Lys Glu Thr Lys Glu Val Lys Pro Ala Ala Lys Ala Thr Asn
   65                  70                  75                  80
   Asn Thr Tyr Pro Ile Leu Asn Gln Glu Leu Arg Glu Ala Ile Lys Asn
                   85                  90                  95
   Pro Ala Ile Lys Asp Lys Asp His Ser Ala Pro Asn Ser Arg Pro Ile
               100                 105                 110
   Asp Phe Glu Met Lys Lys Lys Asp Gly Thr Gln Gln Phe Tyr His Tyr
           115                 120                 125
   Ala Ser Ser Val Lys Pro Ala Arg Val Ile Phe Thr Asp Ser Lys Pro
       130                 135                 140
   Glu Ile Glu Leu Gly Leu Gln Ser Gly Gln Phe Trp Arg Lys Phe Glu
   145                 150                 155                 160
   Val Tyr Glu Gly Asp Lys Lys Leu Pro Ile Lys Leu Val Ser Tyr Asp
                   165                 170                 175
   Thr Val Lys Asp Tyr Ala Tyr Ile Arg Phe Ser Val Ser Asn Gly Thr
               180                 185                 190
   Lys Ala Val Lys Ile Val Ser Ser Thr His Phe Asn Asn Lys Glu Glu
           195                 200                 205
   Lys Tyr Asp Tyr Thr Leu Met Glu Phe Ala Gln Pro Ile Tyr Asn Ser
       210                 215                 220
   Ala Asp Lys Phe Lys Thr Glu Glu Asp Tyr Lys Ala Glu Lys Leu Leu
   225                 230                 235                 240
   Ala Pro Tyr Lys Lys Ala Lys Thr Leu Glu Arg Gln Val Tyr Glu Leu
                   245                 250                 255
   Asn Lys Ile Gln Asp Lys Leu Pro Glu Lys Leu Lys Ala Glu Tyr Lys
   260 265 270
   Lys Lys Leu Glu Asp Thr Lys Lys Ala Leu Asp Glu Gln Val Lys Ser
   275 280 285
   Ala Ile Thr Glu Phe Gln Asn Val Gln Pro Thr Asn Glu Lys Met Thr
   290 295 300
   Asp Leu Gln Asp Thr Lys Tyr Val Val Tyr Glu Ser Val Glu Asn Asn
   305 310 315 320
   Glu Ser Met Met Asp Thr Phe Val Lys His Pro Ile Lys Thr Gly Met
   325 330 335
   Leu Asn Gly Lys Lys Tyr Met Val Met Glu Thr Thr Asn Asp Asp Tyr
   340 345 350
   Trp Lys Asp Phe Met Val Glu Gly Gln Arg Val Arg Thr Ile Ser Lys
   355 360 365
   Asp Ala Lys Asn Asn Thr Arg Thr Ile Ile Phe Pro Tyr Val Glu Gly
   370 375 380
   Lys Thr Leu Tyr Asp Ala Ile Val Lys Val His Val Lys Thr Ile Asp
   385 390 395 400
   Tyr Asp Gly Gln Tyr His Val Arg Ile Val Asp Lys Glu Ala Phe Thr
   405 410 415
   Lys Ala Asn Thr Asp Lys Ser Asn Lys Lys Glu Gln Gln Asp Asn Ser
   420 425 430
   Ala Lys Lys Glu Ala Thr Pro Ala Thr Pro Ser Lys Pro Thr Pro
   435 440 445
<210> 6
   <211> 576
   <212> PRT
   <213> Artificial Sequence
<220>
   <223> ORF0657nH
<400> 6
   Ala Glu Glu Thr Gly Val Thr Asn Thr Glu Ala Gln Pro Lys Thr Glu
   1 5 10 15
   Ala Val Ala Ser Pro Thr Thr Thr Thr Thr Glu Lys Ala Pro Glu Ala
   20 25 30
   Lys Pro Val Ala Lys Pro Val Ala Asn Ala Val Ser Val Ser Asn Lys
   35 40 45
   Glu Val Val Ala Pro Thr Thr Glu Thr Lys Glu Ala Lys Glu Val Lys
   50 55 60
   Ala Val Lys Glu Val Lys Ala Pro Lys Glu Ala Lys Glu Glu Lys Pro
   65 70 75 80
   Ala Ala Lys Ala Asp Asn Asn Thr Tyr Pro Ile Leu Asn Gln Glu Leu
   85 90 95
   Arg Glu Ala Ile Lys Asn Pro Ala Ile Lys Asp Lys Asp His Ser Ala
   100 105 110
   Pro Asn Ser Arg Pro Ile Asp Phe Glu Met Lys Lys Lys Asp Gly Thr
   115 120 125
   Gln Gln Phe Tyr His Tyr Ala Ser Ser Val Lys Pro Ala Arg Val Ile
   130 135 140
   Phe Thr Asp Ser Lys Pro Glu Ile Glu Leu Gly Leu Gln Ser Gly Gln
   145 150 155 160
   Phe Trp Arg Lys Phe Glu Val Tyr Glu Gly Asp Lys Lys Leu Pro Ile
                   165                 170                 175
   Lys Leu Val Ser Tyr Asp Thr Val Lys Asp Tyr Ala Tyr Ile Arg Phe
               180                 185                 190
   Ser Val Ser Asn Gly Thr Lys Ala Val Lys Ile Val Ser Ser Thr His
           195                 200                 205
   Phe Asn Asn Lys Glu Glu Lys Tyr Asp Tyr Thr Leu Met Glu Phe Ala
       210                 215                 220
   Gln Pro Ile Tyr Asn Ser Ala Asp Lys Phe Lys Thr Glu Glu Asp Tyr
   225                 230                 235                 240
   Lys Ala Glu Lys Leu Leu Ala Pro Tyr Lys Lys Ala Lys Thr Leu Glu
                   245                 250                 255
   Arg Gln Val Tyr Glu Leu Asn Lys Ile Gln Asp Lys Leu Pro Glu Lys
               260                 265                 270
   Leu Lys Ala Glu Tyr Lys Lys Lys Leu Glu Glu Thr Lys Lys Ala Leu
           275                 280                 285
   Asp Glu Gln Val Lys Ser Ala Ile Thr Glu Phe Gln Asn Val Gln Pro
       290                 295                 300
   Thr Asn Glu Lys Met Thr Asp Leu Gln Asp Thr Lys Tyr Val Val Tyr
   305                 310                 315                 320
   Glu Ser Val Glu Asn Asn Glu Ser Met Met Asp Ala Phe Val Lys His
                   325                 330                 335
   Pro Ile Lys Thr Gly Met Leu Asn Gly Lys Lys Tyr Met Val Met Glu
               340                 345                 350
   Thr Thr Asn Asp Asp Tyr Trp Lys Asp Phe Met Val Glu Gly Gln Arg
           355                 360                 365
   Val Arg Thr Ile Ser Lys Asp Ala Lys Asn Asn Thr Arg Thr Ile Ile
       370                 375                 380
   Phe Pro Tyr Val Glu Gly Lys Thr Leu Tyr Asp Ala Ile Val Lys Val
   385                 390                 395                 400
   His Val Lys Thr Ile Asp Tyr Asp Gly Gln Tyr His Val Arg Ile Val
                   405                 410                 415
   Asp Lys Glu Ala Phe Thr Lys Ala Asn Ala Asp Lys Thr Asn Lys Lys
               420                 425                 430
   Glu Gln Gln Asp Asn Ser Ala Lys Lys Glu Thr Thr Pro Ala Met Pro
           435                 440                 445
   Ser Lys Pro Thr Thr Pro Pro Val Glu Lys Glu Ser Gln Lys Gln Asp
       450                 455                 460
   Ser Gln Lys Asp Asp Asn Lys Gln Ser Pro Ser Val Glu Lys Glu Asn
   465                 470                 475                 480
   Asp Ala Ser Ser Glu Ser Gly Lys Asp Lys Met Pro Val Thr Lys Pro
                   485                 490                 495
   Ala Lys Ala Glu Val Glu Ser Ser Ser Thr Thr Pro Thr Lys Val Val
               500                 505                 510
   Ser Thr Thr Gln Asn Val Ala Lys Pro Thr Thr Ala Ser Ser Glu Thr
           515                 520                 525
   Thr Lys Asp Val Val Gln Thr Ser Ala Gly Ser Ser Glu Ala Lys Asp
       530                 535                 540
   Ser Ala Pro Leu Gln Lys Ala Asn Ile Lys Asn Thr Asn Asp Gly His
   545                 550                 555                 560
   Thr Gln Ser Gln Asn Asn Lys Asn Thr Gln Glu Asn Lys Ala Lys Ser
                   565                 570                 575
<210> 7
   <211> 568
   <212> PRT
   <213> Artificial Sequence
<220>
   <223> ORF0657nH
<400> 7
   Ala Glu Glu Thr Gly Gly Thr Asn Thr Glu Ala Gln Pro Lys Thr Glu
    1               5                  10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Glu Val Lys Pro Ala Ala Lys Ala Thr Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Lys Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Gly Val Thr Leu Ala Thr Lys Pro Thr Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Gly Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys His Thr Asn Asp Gly His Thr Gln Ser Gln Asn Asn Lys Asn
   545                 550                 555                 560
   Thr Gln Glu Asn Lys Ala Lys Ser
                   565
<210> 8
   <211> 568
   <212> PRT
   <213> Artificial Sequence
<220>
   <223> ORF0657nH
<400> 8
   Ala Glu Glu Thr Gly Gly Thr Asn Thr Glu Ala Gln Pro Lys Thr Glu
    1               5                  10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Glu Val Lys Pro Ala Ala Lys Ala Thr Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Lys Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Thr Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 9
   <211> 568
   <212> PRT
   <213> Artificial Sequence
<220>
   <223> ORF0657nH
<400> 9
   Ala Glu Glu Thr Gly Gly Thr Asn Thr Glu Ala Gln Pro Lys Thr Glu
   1               5                   10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Ala Val Lys Pro Ala Ala Lys Ala Thr Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Glu Asn Gly Glu Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385 390 395 400
   Gly Gln Tyr His Val Arg Ile Val Asp Lys Glu Ala Phe Thr Lys Ala
   405 410 415
   Asn Thr Asp Lys Ser Asn Lys Lys Glu Gln Gln Asp Asn Ser Ala Lys
   420 425 430
   Lys Glu Ala Thr Pro Ala Thr Pro Ser Lys Pro Thr Pro Ser Pro Val
   435 440 445
   Glu Lys Glu Ser Gln Lys Gln Asp Ser Gln Lys Asp Asp Asn Lys Gln
   450 455 460
   Leu Pro Ser Val Glu Lys Glu Asn Asp Ala Ser Ser Glu Ser Gly Lys
   465 470 475 480
   Asp Lys Thr Pro Ala Thr Lys Pro Thr Lys Gly Glu Val Glu Ser Ser
   485 490 495
   Ser Thr Thr Pro Thr Lys Val Val Ser Thr Thr Gln Asn Val Ala Lys
   500 505 510
   Pro Thr Thr Ala Ser Ser Lys Thr Thr Lys Asp Val Val Gln Thr Ser
   515 520 525
   Ala Gly Ser Ser Glu Ala Lys Asp Ser Ala Pro Leu Gln Lys Ala Asn
   530 535 540
   Ile Lys Asn Thr Asn Asp Gly His Thr Gln Ser Gln Asn Asn Lys Asn
   545 550 555 560
   Thr Gln Glu Asn Lys Ala Lys Ser
   565
<210> 10
   <211> 568
   <212> PRT
   <213> Artificial Sequence
<220>
   <223> ORF0657nH
<400> 10
   Ala Glu Glu Thr Gly Gly Thr Asn Thr Glu Ala Gln Pro Lys Thr Glu
   1 5 10 15
   Ala Val Ala Ser Pro Thr Thr Thr Ser Glu Lys Ala Pro Glu Thr Lys
   20 25 30
   Pro Val Ala Asn Ala Val Ser Val Ser Asn Lys Glu Val Glu Ala Pro
   35 40 45
   Thr Ser Glu Thr Lys Glu Ala Lys Glu Val Lys Glu Val Lys Ala Pro
   50 55 60
   Lys Glu Thr Lys Glu Val Lys Pro Ala Ala Lys Ala Thr Asn Asn Thr
   65 70 75 80
   Tyr Pro Ile Leu Asn Gln Glu Leu Arg Glu Ala Ile Lys Asn Pro Ala
   85 90 95
   Ile Lys Asp Lys Asp His Ser Ala Pro Asn Ser Arg Pro Ile Asp Phe
   100 105 110
   Glu Met Lys Lys Lys Asp Gly Thr Gln Gln Phe Tyr His Tyr Ala Ser
   115 120 125
   Ser Val Lys Pro Ala Arg Val Ile Phe Thr Asp Ser Lys Pro Glu Ile
   130 135 140
   Glu Leu Gly Leu Gln Ser Gly Gln Phe Trp Arg Lys Phe Glu Val Tyr
   145 150 155 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Lys Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Thr Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 11
   <211> 565
   <212> PRT
   <213> Artificial Sequence
<220>
   <223> ORF0657nH
<400> 11
   Ala Glu Glu Thr Gly Gly Thr Asn Thr Glu Ala Gln Pro Lys Thr Glu
   1               5                   10                  15
   Ala Val Ala Ser Pro Thr Thr Thr Thr Glu Lys Ala Pro Glu Ala Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Ala Pro Lys Glu Thr
       50                  55                  60
   Lys Ala Val Lys Pro Ala Ala Lys Ala Asp Asn Asn Thr Tyr Pro Ile
   65                  70                  75                  80
   Leu Asn Gln Glu Leu Arg Glu Ala Ile Lys Asn Pro Ala Ile Lys Asp
                   85                  90                  95
   Lys Asp His Ser Ala Pro Asn Ser Arg Pro Ile Asp Phe Glu Met Lys
               100                 105                 110
   Lys Glu Asn Gly Glu Gln Gln Phe Tyr His Tyr Ala Ser Ser Val Lys
           115                 120                 125
   Pro Ala Arg Val Ile Phe Thr Asp Ser Lys Pro Glu Ile Glu Leu Gly
       130                 135                 140
   Leu Gln Ser Gly Gln Phe Trp Arg Lys Phe Glu Val Tyr Glu Gly Asp
   145                 150                 155                 160
   Lys Lys Leu Pro Ile Lys Leu Val Ser Tyr Asp Thr Val Lys Asp Tyr
                   165                 170                 175
   Ala Tyr Ile Arg Phe Ser Val Ser Asn Gly Thr Lys Ala Val Lys Ile
               180                 185                 190
   Val Ser Ser Thr His Phe Asn Asn Lys Glu Glu Lys Tyr Asp Tyr Thr
           195                 200                 205
   Leu Met Glu Phe Ala Gln Pro Ile Tyr Asn Ser Ala Asp Lys Phe Lys
       210                 215                 220
   Thr Glu Glu Asp Tyr Lys Ala Glu Lys Leu Leu Ala Pro Tyr Lys Lys
   225                 230                 235                 240
   Ala Lys Thr Leu Glu Arg Gln Val Tyr Glu Leu Asn Lys Ile Gln Asp
                   245                 250                 255
   Lys Leu Pro Glu Lys Leu Lys Ala Glu Tyr Lys Lys Lys Leu Glu Asp
               260                 265                 270
   Thr Lys Lys Ala Leu Asp Glu Gln Val Lys Ser Ala Ile Thr Glu Phe
           275                 280                 285
   Gln Asn Val Gln Pro Thr Asn Glu Lys Met Thr Asp Leu Gln Asp Thr
       290                 295                 300
   Lys Tyr Val Val Tyr Glu Ser Val Glu Asn Asn Glu Ser Met Met Asp
   305                 310                 315                 320
   Thr Phe Val Lys His Pro Ile Lys Thr Gly Met Leu Asn Gly Lys Lys
                   325                 330                 335
   Tyr Met Val Met Glu Thr Thr Asn Asp Asp Tyr Trp Lys Asp Phe Met
               340                 345                 350
   Val Glu Gly Gln Arg Val Arg Thr Ile Ser Lys Asp Ala Lys Asn Asn
           355                 360                 365
   Thr Arg Thr Ile Ile Phe Pro Tyr Val Glu Gly Lys Thr Leu Tyr Asp
       370                 375                 380
   Ala Ile Val Lys Val His Val Lys Thr Ile Asp Tyr Asp Gly Gln Tyr
   385                 390                 395                 400
   His Val Arg Ile Val Asp Lys Glu Ala Phe Thr Lys Ala Asn Thr Asp
                   405                 410                 415
   Lys Ser Asn Lys Lys Glu Gln Gln Asp Asn Ser Ala Lys Lys Glu Ala
               420                 425                 430
   Thr Pro Ala Thr Pro Ser Lys Pro Thr Pro Ser Pro Val Glu Lys Glu
           435                 440                 445
   Ser Gln Lys Gln Asp Ser Gln Lys Asp Asp Asn Lys Gln Leu Pro Ser
       450                 455                 460
   Val Glu Lys Glu Asn Asp Ala Ser Ser Glu Ser Gly Lys Asp Lys Thr
   465                 470                 475                 480
   Pro Ala Thr Lys Pro Ala Lys Gly Glu Val Glu Ser Ser Ser Thr Thr
                   485                 490                 495
   Pro Thr Lys Val Val Ser Thr Thr Gln Asn Val Ala Lys Pro Thr Thr
               500                 505                 510
   Ala Ser Ser Lys Thr Thr Lys Asp Val Val Gln Thr Ser Ala Ser Ser
           515                 520                 525
   Ser Glu Ala Lys Asp Ser Ala Pro Leu Gln Lys Ala Asn Ile Lys Asn
       530                 535                 540
   Thr Asn Asp Gly His Thr Gln Ser Gln Asn Asn Lys Asn Thr Gln Glu
   545                 550                 555                 560
   Asn Lys Ala Lys Ser
                   565
<210> 12
   <211> 566
   <212> PRT
   <213> Artificial Sequence
<220>
   <223> ORF0657nH
<400> 12
   Ala Glu Glu Thr Gly Gly Thr Asn Thr Glu Ala Gln Pro Lys Thr Glu
    1               5                  10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Ala Val Lys Pro Ala Ala Lys Ala Thr Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Glu Asn Gly Glu Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
   165 170 175
   Lys Asp Tyr Ala Tyr Ile Arg Phe Ser Val Ser Asn Gly Thr Lys Ala
   180 185 190
   Val Lys Ile Val Ser Ser Thr His Phe Asn Asn Lys Glu Glu Lys Tyr
   195 200 205
   Asp Tyr Thr Leu Met Glu Phe Ala Gln Pro Ile Tyr Asn Ser Ala Asp
   210 215 220
   Lys Phe Lys Thr Glu Glu Asp Tyr Lys Ala Glu Lys Leu Leu Ala Pro
   225 230 235 240
   Tyr Lys Lys Ala Lys Thr Leu Glu Arg Gln Val Tyr Glu Leu Asn Lys
   245 250 255
   Ile Gln Asp Lys Leu Pro Glu Lys Leu Lys Ala Glu Tyr Lys Lys Lys
   260 265 270
   Leu Glu Asp Thr Lys Lys Ala Leu Asp Glu Gln Val Lys Ser Ala Ile
   275 280 285
   Thr Glu Phe Gln Asn Val Gln Pro Thr Asn Glu Lys Met Thr Asp Leu
   290 295 300
   Gln Asp Thr Lys Tyr Val Val Tyr Glu Ser Val Glu Asn Asn Glu Ser
   305 310 315 320
   Met Met Asp Thr Phe Val Lys His Pro Ile Lys Thr Gly Met Leu Asn
   325 330 335
   Gly Lys Lys Tyr Met Val Met Glu Thr Thr Asn Asp Asp Tyr Trp Lys
   340 345 350
   Asp Phe Met Val Glu Gly Gln Arg Val Arg Thr Ile Ser Lys Asp Ala
   355 360 365
   Lys Asn Asn Thr Arg Thr Ile Ile Phe Pro Tyr Val Glu Gly Lys Thr
   370 375 380
   Leu Tyr Asp Ala Ile Val Lys Val His Val Lys Thr Ile Asp Tyr Asp
   385 390 395 400
   Gly Gln Tyr His Val Arg Ile Val Asp Lys Glu Ala Phe Thr Lys Ala
   405 410 415
   Asn Thr Asp Lys Ser Asn Lys Lys Glu Gln Gln Asp Asn Ser Ala Lys
   420 425 430
   Lys Glu Ala Thr Pro Ala Thr Pro Ser Lys Pro Thr Pro Ser Pro Val
   435 440 445
   Glu Lys Glu Ser Gln Lys Gln Asp Ser Gln Lys Asp Asp Asn Lys Gln
   450 455 460
   Leu Pro Ser Val Glu Lys Glu Asn Asp Ala Ser Ser Glu Ser Gly Lys
   465 470 475 480
   Asp Lys Thr Pro Ala Thr Lys Pro Thr Lys Gly Glu Val Glu Ser Ser
   485 490 495
   Thr Thr Pro Thr Lys Val Val Ser Thr Thr Gln Asn Val Ala Lys Pro
   500 505 510
   Thr Ala Ser Ser Lys Thr Thr Lys Asp Val Val Gln Thr Ser Ala Gly
   515 520 525
   Ser Ser Glu Ala Lys Asp Ser Ala Pro Leu Gln Lys Ala Asn Ile Lys
   530 535 540
   Asn Thr Asn Asp Gly His Thr Gln Ser Gln Asn Asn Lys Asn Thr Gln
   545 550 555 560
   Glu Asn Lys Ala Lys Ser
   565
<210> 13
   <211> 568
   <212> PRT
   <213> Artificial Sequence
<220>
   <223> ORF0657nH
<400> 13
   Ala Glu Glu Thr Gly Gly Thr Asn Thr Glu Ala Gln Pro Lys Thr Glu
   1               5                   10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Glu Val Lys Pro Ala Ala Lys Ala Thr Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Lys Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385 390 395 400
   Gly Gln Tyr His Val Arg Ile Val Asp Lys Glu Ala Phe Thr Lys Ala
   405 410 415
   Asn Thr Asp Lys Ser Asn Lys Lys Glu Gln Gln Asp Asn Ser Ala Lys
   420 425 430
   Lys Glu Ala Thr Pro Ala Thr Pro Ser Lys Pro Thr Pro Ser Pro Val
   435 440 445
   Glu Lys Glu Ser Gln Lys Gln Asp Ser Gln Lys Asp Asp Asn Lys Gln
   450 455 460
   Leu Pro Ser Val Glu Lys Glu Asn Asp Ala Ser Ser Glu Ser Gly Lys
   465 470 475 480
   Asp Lys Thr Pro Ala Thr Lys Pro Thr Lys Gly Lys Val Glu Ser Ser
   485 490 495
   Ser Thr Thr Pro Thr Lys Val Val Ser Thr Thr Gln Asn Val Ala Lys
   500 505 510
   Pro Thr Thr Ala Ser Ser Lys Thr Thr Lys Asp Val Val Gln Thr Ser
   515 520 525
   Ala Gly Ser Ser Glu Ala Lys Asp Ser Ala Pro Leu Gln Lys Ala Asn
   530 535 540
   Ile Lys Asn Thr Asn Asp Gly His Thr Gln Ser Gln Asn Asn Lys Asn
   545 550 555 560
   Thr Gln Glu Asn Lys Ala Lys Ser
   565
<210> 14
   <211> 568
   <212> PRT
   <213> Artificial Sequence
<220>
   <223> ORF0657nH
<400> 14
   Ala Glu Glu Thr Gly Gly Thr Asn Thr Glu Ala Gln Pro Lys Thr Glu
   1 5 10 15
   Ala Val Ala Ser Pro Thr Thr Thr Ser Glu Lys Ala Pro Glu Thr Lys
   20 25 30
   Pro Val Ala Asn Ala Val Ser Val Ser Asn Lys Glu Val Glu Ala Pro
   35 40 45
   Thr Ser Glu Thr Lys Glu Ala Lys Glu Val Lys Glu Val Lys Ala Pro
   50 55 60
   Lys Glu Thr Lys Glu Val Lys Pro Ala Ala Lys Ala Thr Asn Asn Thr
   65 70 75 80
   Tyr Pro Ile Leu Asn Gln Glu Leu Arg Glu Ala Ile Lys Asn Pro Glu
   85 90 95
   Ile Lys Asp Lys Asp His Ser Ala Pro Asn Ser Arg Pro Ile Asp Phe
   100 105 110
   Glu Met Lys Lys Lys Asp Gly Thr Gln Gln Phe Tyr His Tyr Ala Ser
   115 120 125
   Ser Val Lys Pro Ala Arg Val Ile Phe Thr Asp Ser Lys Pro Glu Ile
   130 135 140
   Glu Leu Gly Leu Gln Ser Gly Gln Phe Trp Arg Lys Phe Glu Val Tyr
   145 150 155 160
   Glu Gly Asp Lys Lys Leu Pro Ile Lys Leu Ala Ser Tyr Asp Thr Val
                   165                 170                 175
   Lys Asp Tyr Ala Tyr Ile Arg Phe Ser Ile Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Ala Lys Gly Glu Val Glu Ser Ser
                   485                 490                 495
   Ser Thr Thr Pro Thr Lys Val Val Ser Thr Thr Gln Asn Val Val Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 15
   <211> 564
   <212> PRT
   <213> Artificial Sequence
<220>
   <223> ORF0657nH
<400> 15
   Ala Glu Glu Thr Gly Gly Thr Asn Thr Glu Ala Gln Pro Lys Thr Glu
   1               5                   10                  15
   Ala Val Ala Ser Pro Thr Thr Thr Thr Glu Lys Ala Pro Glu Ala Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Ala Pro Lys Glu Thr
       50                  55                  60
   Lys Ala Val Lys Pro Ala Ala Lys Ala Asp Asn Asn Thr Tyr Pro Ile
   65                  70                  75                  80
   Leu Asn Gln Glu Leu Arg Glu Ala Ile Lys Asn Pro Ala Ile Lys Asp
                   85                  90                  95
   Lys Asp His Ser Ala Pro Asn Ser Arg Pro Ile Asp Phe Glu Met Lys
               100                 105                 110
   Lys Glu Asn Gly Glu Gln Gln Phe Tyr His Tyr Ala Ser Ser Val Lys
           115                 120                 125
   Pro Ala Arg Val Ile Phe Thr Asp Ser Lys Pro Glu Ile Glu Leu Gly
       130                 135                 140
   Leu Gln Ser Gly Gln Phe Trp Arg Lys Phe Glu Val Tyr Glu Gly Asp
   145                 150                 155                 160
   Lys Lys Leu Pro Ile Lys Leu Val Ser Tyr Asp Thr Val Lys Asp Tyr
                   165                 170                 175
   Ala Tyr Ile Arg Phe Ser Val Ser Asn Gly Thr Lys Ala Val Lys Ile
               180                 185                 190
   Val Ser Ser Thr His Phe Asn Asn Lys Glu Glu Lys Tyr Asp Tyr Thr
           195                 200                 205
   Leu Met Glu Phe Ala Gln Pro Ile Tyr Asn Ser Ala Asp Lys Phe Lys
       210                 215                 220
   Thr Glu Glu Asp Tyr Lys Ala Glu Lys Leu Leu Ala Pro Tyr Lys Lys
   225                 230                 235                 240
   Ala Lys Thr Leu Glu Arg Gln Val Tyr Glu Leu Asn Lys Ile Gln Asp
                   245                 250                 255
   Lys Leu Pro Glu Lys Leu Lys Ala Glu Tyr Lys Lys Lys Leu Glu Asp
               260                 265                 270
   Thr Lys Lys Ala Leu Asp Glu Gln Val Lys Ser Ala Ile Thr Glu Phe
           275                 280                 285
   Gln Asn Val Gln Pro Thr Asn Glu Lys Met Thr Asp Leu Gln Asp Thr
       290                 295                 300
   Lys Tyr Val Val Tyr Glu Ser Val Glu Asn Asn Glu Ser Met Met Asp
   305                 310                 315                 320
   Thr Phe Val Lys His Pro Ile Lys Thr Gly Met Leu Asn Gly Lys Lys
                   325                 330                 335
   Tyr Met Val Met Glu Thr Thr Asn Asp Asp Tyr Trp Lys Asp Phe Met
               340                 345                 350
   Val Glu Gly Gln Arg Val Arg Thr Ile Ser Lys Asp Ala Lys Asn Asn
           355                 360                 365
   Thr Arg Thr Ile Ile Phe Pro Tyr Val Glu Gly Lys Thr Leu Tyr Asp
       370                 375                 380
   Ala Ile Val Lys Val His Val Lys Thr Ile Asp Tyr Asp Gly Gln Tyr
   385                 390                 395                 400
   His Val Arg Ile Val Asp Lys Glu Ala Phe Thr Lys Ala Asn Thr Asp
                   405                 410                 415
   Lys Ser Asn Lys Lys Glu Gln Gln Asp Asn Ser Ala Lys Lys Glu Ala
               420                 425                 430
   Thr Pro Ala Thr Pro Ser Lys Pro Thr Pro Ser Pro Val Glu Lys Glu
           435                 440                 445
   Ser Gln Lys Gln Asp Ser Gln Lys Asp Asp Asn Lys Gln Leu Pro Ser
       450                 455                 460
   Val Glu Lys Glu Asn Asp Ala Ser Ser Glu Ser Gly Lys Asp Lys Thr
   465                 470                 475                 480
   Pro Ala Thr Lys Pro Ala Lys Gly Glu Val Glu Ser Ser Ser Thr Thr
                   485                 490                 495
   Pro Thr Lys Val Val Ser Thr Thr Gln Asn Val Ala Lys Pro Thr Ala
               500                 505                 510
   Ser Ser Lys Thr Thr Lys Asp Val Val Gln Thr Ser Ala Ser Ser Ser
           515                 520                 525
   Glu Ala Lys Asp Ser Ala Pro Leu Gln Lys Ala Asn Ile Lys Asn Thr
       530                 535                 540
   Asn Asp Gly His Thr Gln Ser Gln Asn Asn Lys Asn Thr Gln Glu Asn
   545                 550                 555                 560
   Lys Ala Lys Ser
<210> 16
   <211> 565
   <212> PRT
   <213> Artificial Sequence
<220>
   <223> ORF0657nH
<400> 16
   Ala Glu Glu Thr Gly Gly Thr Asn Thr Glu Ala Gln Pro Lys Thr Glu
    1               5                  10                  15
   Ala Val Ala Ser Pro Thr Thr Thr Thr Glu Lys Ala Pro Glu Ala Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Ala Pro Lys Glu Thr
       50                  55                  60
   Lys Ala Val Lys Pro Ala Ala Lys Ala Asp Asn Asn Thr Tyr Pro Ile
   65                  70                  75                  80
   Leu Asn Gln Glu Leu Arg Glu Ala Ile Lys Asn Pro Ala Ile Lys Asp
                   85                  90                  95
   Lys Asp His Ser Ala Pro Asn Ser Arg Pro Ile Asp Phe Glu Met Lys
               100                 105                 110
   Lys Glu Asn Gly Glu Gln Gln Phe Tyr His Tyr Ala Ser Ser Val Lys
           115                 120                 125
   Pro Ala Arg Val Ile Phe Thr Asp Ser Lys Pro Glu Ile Glu Leu Gly
       130                 135                 140
   Leu Gln Ser Gly Gln Phe Trp Arg Lys Phe Glu Val Tyr Glu Gly Asp
   145                 150                 155                 160
   Lys Lys Leu Pro Ile Lys Leu Val Ser Tyr Asp Thr Val Lys Asp Tyr
                   165                 170                 175
   Ala Tyr Ile Arg Phe Ser Val Ser Asn Gly Thr Lys Ala Val Lys Ile
               180                 185                 190
   Val Ser Ser Thr His Phe Asn Asn Lys Glu Glu Lys Tyr Asp Tyr Thr
           195                 200                 205
   Leu Met Glu Phe Ala Gln Pro Ile Tyr Asn Ser Ala Asp Lys Phe Lys
       210                 215                 220
   Thr Glu Glu Asp Tyr Lys Ala Glu Lys Leu Leu Ala Pro Tyr Lys Lys
   225                 230                 235                 240
   Ala Lys Thr Leu Glu Arg Gln Val Tyr Glu Leu Asn Lys Ile Gln Asp
                   245                 250                 255
   Lys Leu Pro Glu Lys Leu Lys Ala Glu Tyr Lys Lys Lys Leu Glu Asp
               260                 265                 270
   Thr Lys Lys Ala Leu Asp Glu Gln Val Lys Ser Ala Ile Thr Glu Phe
           275                 280                 285
   Gln Asn Val Gln Pro Thr Asn Glu Lys Met Thr Asp Leu Gln Asp Thr
       290                 295                 300
   Lys Tyr Val Val Tyr Glu Ser Val Glu Asn Asn Glu Ser Met Met Asp
   305                 310                 315                 320
   Thr Phe Val Lys His Pro Ile Lys Thr Gly Met Leu Asn Gly Lys Lys
                   325                 330                 335
   Tyr Met Val Met Glu Thr Thr Asn Asp Asp Tyr Trp Lys Asp Phe Met
               340                 345                 350
   Val Glu Gly Gln Arg Val Arg Thr Ile Ser Lys Asp Ala Lys Asn Asn
           355                 360                 365
   Thr Arg Thr Ile Ile Phe Pro Tyr Val Glu Gly Lys Thr Leu Tyr Asp
       370                 375                 380
   Ala Ile Val Arg Val His Val Lys Thr Ile Asp Tyr Asp Gly Gln Tyr
   385                 390                 395                 400
   His Val Arg Ile Val Asp Lys Glu Ala Phe Thr Lys Ala Asn Thr Asp
                   405                 410                 415
   Lys Ser Asn Lys Lys Glu Gln Gln Asp Asn Ser Ala Lys Lys Glu Ala
               420                 425                 430
   Thr Pro Ala Thr Pro Ser Lys Pro Thr Pro Ser Pro Val Glu Lys Glu
           435                 440                 445
   Ser Gln Lys Gln Asp Ser Gln Lys Asp Asp Asn Lys Gln Leu Pro Ser
       450                 455                 460
   Val Glu Lys Glu Asn Asp Ala Ser Ser Glu Ser Gly Lys Asp Lys Thr
   465                 470                 475                 480
   Pro Ala Thr Lys Pro Ala Lys Gly Glu Val Glu Ser Ser Ser Thr Thr
                   485                 490                 495
   Pro Thr Lys Val Val Ser Thr Thr Gln Asn Val Ala Lys Pro Thr Thr
               500                 505                 510
   Ala Ser Ser Lys Thr Thr Lys Asp Val Val Gln Thr Ser Ala Gly Ser
           515                 520                 525
   Ser Glu Ala Lys Asp Ser Ala Pro Leu Gln Lys Ala Asn Ile Lys Asn
       530                 535                 540
   Thr Asn Asp Gly His Thr Gln Ser Gln Asn Asn Lys Asn Thr Gln Glu
   545                 550                 555                 560
   Asn Lys Ala Lys Ser
                   565
<210> 17
   <211> 568
   <212> PRT
   <213> Artificial Sequence
<220>
   <223> ORF0657nH
<400> 17
   Ala Glu Glu Thr Gly Gly Thr Asn Thr Glu Ala Gln Pro Lys Thr Glu
   1               5                   10                  15
   Ala Leu Ala Ser Pro Thr Thr Thr Thr Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Ala Val Lys Pro Ala Ala Lys Ala Asp Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Glu Asn Gly Glu Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Ile Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Ala Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 18
   <211> 565
   <212> PRT
   <213> Artificial Sequence
<220>
   <223> ORF0657nH
<400> 18
   Ala Glu Glu Thr Gly Gly Thr Asn Thr Glu Ala Gln Pro Lys Thr Glu
    1               5                  10                  15
   Ala Val Ala Ser Pro Thr Thr Thr Thr Glu Lys Ala Pro Glu Ala Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Ala Pro Lys Glu Thr
       50                  55                  60
   Lys Ala Val Lys Pro Ala Ala Lys Ala Asp Asn Asn Thr Tyr Pro Ile
   65                  70                  75                  80
   Leu Asn Gln Glu Leu Arg Glu Ala Ile Lys Asn Pro Ala Ile Lys Asp
                   85                  90                  95
   Lys Asp His Ser Ala Pro Asn Ser Arg Pro Ile Asp Phe Glu Met Lys
               100                 105                 110
   Lys Glu Asn Gly Glu Gln Gln Phe Tyr His Tyr Ala Ser Ser Val Lys
           115                 120                 125
   Pro Ala Arg Val Ile Phe Thr Asp Ser Lys Pro Glu Ile Glu Leu Gly
       130                 135                 140
   Leu Gln Ser Gly Gln Phe Trp Arg Lys Phe Glu Val Tyr Glu Gly Asp
   145                 150                 155                 160
   Lys Lys Leu Pro Ile Lys Leu Val Ser Tyr Asp Thr Val Lys Asp Tyr
                   165                 170                 175
   Ala Tyr Ile Arg Phe Ser Val Ser Asn Gly Thr Lys Ala Val Lys Ile
               180                 185                 190
   Val Ser Ser Thr His Phe Asn Asn Lys Glu Glu Lys Tyr Asp Tyr Thr
           195                 200                 205
   Leu Met Glu Phe Ala Gln Pro Ile Tyr Asn Ser Ala Asp Lys Phe Lys
       210                 215                 220
   Thr Glu Glu Asp Tyr Lys Ala Glu Lys Leu Leu Ala Pro Tyr Lys Lys
   225                 230                 235                 240
   Ala Lys Thr Leu Glu Arg Gln Val Tyr Glu Leu Asn Lys Ile Gln Asp
                   245                 250                 255
   Lys Leu Pro Glu Lys Leu Lys Ala Glu Tyr Lys Lys Lys Leu Glu Asp
               260                 265                 270
   Thr Lys Lys Ala Leu Asp Glu Gln Val Lys Ser Ala Ile Thr Glu Phe
           275                 280                 285
   Gln Asn Val Gln Pro Thr Asn Glu Lys Met Thr Asp Leu Gln Asp Thr
       290                 295                 300
   Lys Tyr Val Val Tyr Glu Ser Val Glu Asn Asn Glu Ser Met Met Asp
   305                 310                 315                 320
   Thr Phe Val Lys His Pro Ile Lys Thr Gly Met Leu Asn Gly Lys Lys
                   325                 330                 335
   Tyr Met Val Met Glu Thr Thr Asn Asp Asp Tyr Trp Lys Asp Phe Met
               340                 345                 350
   Val Glu Gly Gln Arg Val Arg Thr Ile Ser Lys Asp Ala Lys Asn Asn
           355                 360                 365
   Thr Arg Thr Ile Ile Phe Pro Tyr Val Glu Gly Lys Thr Leu Tyr Asp
       370                 375                 380
   Ala Ile Val Lys Val His Val Lys Thr Ile Asp Tyr Asp Gly Gln Tyr
   385                 390                 395                 400
   His Val Arg Ile Val Asp Lys Glu Ala Phe Thr Lys Ala Asn Thr Asp
                   405                 410                 415
   Lys Ser Asn Lys Lys Glu Gln Gln Asp Asn Ser Ala Lys Lys Glu Ala
               420                 425                 430
   Thr Pro Ala Thr Pro Ser Lys Pro Thr Pro Ser Pro Val Glu Lys Glu
           435                 440                 445
   Ser Gln Lys Gln Asp Ser Gln Lys Asp Asp Asn Lys Pro Leu Pro Ser
       450                 455                 460
   Val Glu Lys Glu Asn Asp Ala Ser Ser Glu Ser Gly Lys Asp Lys Thr
   465                 470                 475                 480
   Pro Ala Thr Lys Pro Ala Lys Gly Glu Val Glu Ser Ser Ser Thr Thr
                   485                 490                 495
   Pro Thr Lys Val Val Ser Thr Thr Gln Asn Val Ala Lys Pro Thr Thr
               500                 505                 510
   Ala Ser Ser Lys Thr Thr Lys Asp Val Val Gln Thr Ser Ala Ser Ser
           515                 520                 525
   Ser Glu Ala Lys Asp Ser Ala Pro Leu Gln Lys Ala Asn Ile Lys Asn
       530                 535                 540
   Thr Asn Asp Gly His Thr Gln Ser Gln Asn Asn Lys Asn Thr Gln Glu
   545                 550                 555                 560
   Asn Lys Ala Lys Ser
                   565
<210> 19
   <211> 568
   <212> PRT
   <213> Artificial Sequence
<220>
   <223> ORF0657nH
<400> 19
   Ala Glu Glu Thr Gly Gly Thr Asn Thr Glu Ala Gln Pro Lys Thr Glu
    1               5                  10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Ala Val Lys Pro Ala Thr Lys Ala Asp Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Glu Asn Gly Glu Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Arg Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Ala Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 20
   <211> 568
   <212> PRT
   <213> Artificial Sequence
<220>
   <223> ORF0657nH
<400> 20
   Ala Glu Glu Thr Gly Gly Thr Asn Thr Glu Ala Gln Pro Lys Thr Glu
   1               5                   10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Ala Val Lys Pro Ala Thr Lys Ala Asp Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Glu Asn Gly Glu Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Ala Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 21
   <211> 576
   <212> PRT
   <213> Artificial Sequence
<220>
   <223> ORF0657nH
<400> 21
   Ala Glu Glu Thr Gly Val Thr Asn Thr Glu Ala Gln Pro Lys Thr Glu
   1               5                   10                  15
   Ala Val Ala Ser Pro Thr Thr Thr Thr Thr Glu Lys Ala Pro Glu Ala
               20                  25                  30
   Lys Pro Val Ala Lys Pro Val Ala Asn Ala Val Ser Val Ser Asn Lys
           35                  40                  45
   Glu Val Val Ala Pro Thr Thr Glu Thr Lys Glu Ala Lys Glu Val Lys
       50                  55                  60
   Ala Val Lys Glu Val Lys Ala Pro Lys Glu Ala Lys Glu Glu Lys Pro
   65                  70                  75                  80
   Ala Ala Lys Ala Asp Asn Asn Thr Tyr Pro Ile Leu Asn Gln Glu Leu
                   85                  90                  95
   Arg Glu Ala Ile Lys Asn Pro Ala Ile Lys Asp Lys Asp His Ser Ala
               100                 105                 110
   Pro Asn Ser Arg Pro Ile Asp Phe Glu Met Lys Lys Lys Asp Gly Thr
           115                 120                 125
   Gln Gln Phe Tyr His Tyr Ala Gly Ser Val Lys Pro Ala Arg Val Ile
       130                 135                 140
   Phe Thr Asp Ser Lys Pro Glu Ile Glu Leu Gly Leu Gln Ser Gly Gln
   145                 150                 155                 160
   Phe Trp Arg Lys Phe Glu Val Tyr Glu Gly Asp Lys Lys Leu Pro Ile
                   165                 170                 175
   Lys Leu Val Ser Tyr Asp Thr Val Lys Asp Tyr Ala Tyr Ile Arg Phe
               180                 185                 190
   Ser Val Ser Asn Gly Thr Lys Ala Val Lys Ile Val Ser Ser Thr His
           195                 200                 205
   Phe Asn Asn Lys Glu Glu Lys Tyr Asp Tyr Thr Leu Met Glu Phe Ala
       210                 215                 220
   Gln Pro Ile Tyr Asn Ser Ala Asp Lys Phe Lys Thr Glu Glu Asp Tyr
   225                 230                 235                 240
   Lys Ala Glu Lys Leu Leu Ala Pro Tyr Lys Lys Ala Lys Thr Leu Glu
                   245                 250                 255
   Arg Gln Val Tyr Glu Leu Asn Lys Ile Gln Asp Lys Leu Pro Glu Lys
               260                 265                 270
   Leu Lys Ala Glu Tyr Lys Lys Lys Leu Glu Glu Thr Lys Lys Ala Leu
           275                 280                 285
   Asp Glu Gln Val Lys Ser Ala Ile Thr Glu Phe Gln Asn Val Gln Pro
       290                 295                 300
   Thr Asn Glu Lys Met Thr Asp Leu Gln Asp Thr Lys Tyr Val Val Tyr
   305                 310                 315                 320
   Glu Ser Val Glu Asn Asn Glu Ser Met Met Asp Thr Phe Val Lys His
                   325                 330                 335
   Pro Ile Lys Thr Gly Met Leu Asn Gly Lys Lys Tyr Met Val Met Glu
               340                 345                 350
   Thr Thr Asn Asp Asp Tyr Trp Lys Asp Phe Met Val Glu Gly Gln Arg
           355                 360                 365
   Val Arg Thr Ile Ser Lys Asp Ala Lys Asn Asn Thr Arg Thr Ile Ile
       370                 375                 380
   Phe Pro Tyr Val Glu Gly Lys Thr Leu Tyr Asp Ala Ile Val Lys Val
   385 390 395 400
   His Val Lys Thr Ile Asp Tyr Asp Gly Gln Tyr His Val Arg Ile Val
   405 410 415
   Asp Lys Glu Ala Phe Thr Lys Ala Asn Ala Asp Lys Thr Asn Lys Lys
   420 425 430
   Glu Gln Gln Asp Asn Ser Ala Lys Lys Glu Thr Thr Pro Ala Met Pro
   435 440 445
   Ser Lys Pro Thr Thr Pro Pro Val Glu Lys Glu Ser Gln Lys Gln Asp
   450 455 460
   Ser Gln Lys Asp Asp Asn Lys Gln Ser Pro Gly Val Glu Lys Glu Asn
   465 470 475 480
   Asp Ala Ser Ser Glu Ser Gly Lys Asp Lys Met Pro Val Thr Lys Pro
   485 490 495
   Ala Lys Ala Glu Val Glu Ser Ser Ser Thr Thr Pro Thr Lys Val Val
   500 505 510
   Ser Thr Thr Gln Asn Val Ala Lys Pro Thr Thr Ala Ser Ser Glu Thr
   515 520 525
   Thr Lys Asp Val Val Gln Thr Ser Ala Gly Ser Ser Glu Ala Lys Asp
   530 535 540
   Ser Ala Pro Leu Gln Lys Ala Asn Ile Lys Asn Thr Asn Asp Gly His
   545 550 555 560
   Thr Gln Ser Gln Asn Asn Lys Asn Thr Gln Glu Asn Lys Ala Lys Ser
   565 570 575
<210> 22
   <211> 576
   <212> PRT
   <213> Artificial Sequence
<220>
   <223> ORF0657nH
<400> 22
   Ala Glu Glu Thr Gly Val Thr Asn Thr Glu Ala Gln Pro Lys Thr Glu
   1 5 10 15
   Ala Val Ala Ser Pro Thr Thr Thr Thr Thr Glu Lys Ala Pro Glu Ala
   20 25 30
   Lys Pro Val Ala Lys Pro Val Ala Asn Ala Val Ser Val Ser Asn Lys
   35 40 45
   Glu Val Val Ala Pro Thr Thr Glu Thr Lys Glu Ala Lys Glu Val Lys
   50 55 60
   Ala Val Lys Glu Val Lys Ala Pro Lys Glu Ala Lys Glu Glu Lys Pro
   65 70 75 80
   Ala Ala Lys Ala Asp Asn Asn Thr Tyr Pro Ile Leu Asn Gln Glu Leu
   85 90 95
   Arg Glu Ala Ile Lys Asn Pro Ala Ile Lys Asp Lys Asp His Ser Ala
   100 105 110
   Pro Asn Ser Arg Pro Ile Asp Phe Glu Met Lys Lys Lys Asp Gly Thr
   115 120 125
   Gln Gln Phe Tyr His Tyr Ala Ser Ser Val Lys Pro Ala Arg Val Ile
   130 135 140
   Phe Thr Asp Ser Lys Pro Glu Ile Glu Leu Gly Leu Gln Ser Gly Gln
   145 150 155 160
   Phe Trp Arg Lys Phe Glu Val Tyr Glu Gly Asp Lys Lys Leu Pro Ile
                   165                 170                 175
   Lys Leu Val Ser Tyr Asp Thr Val Lys Asp Tyr Ala Tyr Ile Arg Phe
               180                 185                 190
   Ser Val Ser Asn Gly Thr Lys Ala Val Lys Ile Val Ser Ser Thr His
           195                 200                 205
   Phe Asn Asn Lys Glu Glu Lys Tyr Asp Tyr Thr Leu Met Glu Phe Ala
       210                 215                 220
   Gln Pro Ile Tyr Asn Ser Ala Asp Lys Phe Lys Thr Glu Glu Asp Tyr
   225                 230                 235                 240
   Lys Ala Glu Lys Leu Leu Ala Pro Tyr Lys Lys Ala Lys Thr Leu Glu
                   245                 250                 255
   Arg Gln Val Tyr Glu Leu Asn Lys Ile Gln Asp Lys Leu Pro Glu Lys
               260                 265                 270
   Leu Lys Ala Glu Tyr Lys Lys Lys Leu Glu Glu Thr Lys Lys Ala Leu
           275                 280                 285
   Asp Glu Gln Val Lys Ser Ala Ile Thr Glu Phe Gln Asn Val Gln Pro
       290                 295                 300
   Thr Asn Glu Lys Met Thr Asp Leu Gln Asp Thr Lys Tyr Val Val Tyr
   305                 310                 315                 320
   Glu Ser Val Glu Asn Asn Glu Ser Met Met Asp Thr Phe Val Lys His
                   325                 330                 335
   Pro Ile Lys Thr Gly Met Leu Asn Gly Lys Lys Tyr Met Val Met Glu
               340                 345                 350
   Thr Thr Asn Asp Asp Tyr Trp Lys Asp Phe Met Val Glu Gly Gln Arg
           355                 360                 365
   Val Arg Thr Ile Ser Lys Asp Ala Lys Asn Asn Thr Arg Thr Ile Ile
       370                 375                 380
   Phe Pro Tyr Val Glu Gly Lys Thr Leu Tyr Asp Ala Ile Val Lys Val
   385                 390                 395                 400
   His Val Lys Thr Ile Asp Tyr Asp Gly Gln Tyr His Val Arg Ile Val
                   405                 410                 415
   Asp Lys Glu Ala Phe Thr Lys Ala Asn Ala Asp Lys Thr Asn Lys Lys
               420                 425                 430
   Glu Gln Gln Asp Asn Ser Ala Lys Lys Glu Thr Thr Pro Ala Met Pro
           435                 440                 445
   Ser Lys Pro Thr Thr Pro Pro Val Glu Lys Glu Ser Gln Lys Gln Asp
       450                 455                 460
   Ser Gln Lys Asp Asp Asn Lys Gln Ser Pro Ser Val Glu Lys Glu Asn
   465                 470                 475                 480
   Asp Ala Ser Ser Glu Ser Gly Lys Asp Lys Met Pro Val Thr Lys Pro
                   485                 490                 495
   Ala Lys Ala Glu Val Glu Ser Ser Ser Thr Thr Pro Thr Lys Val Val
               500                 505                 510
   Ser Thr Thr Gln Asn Val Ala Lys Pro Thr Thr Ala Ser Ser Glu Thr
           515                 520                 525
   Thr Lys Asp Val Val Gln Thr Ser Ala Gly Ser Ser Glu Ala Lys Asp
       530                 535                 540
   Ser Ala Pro Leu Gln Lys Ala Asn Ile Lys Asn Thr Asn Asp Gly His
   545                 550                 555                 560
   Thr Gln Ser Gln Asn Asn Lys Asn Thr Gln Glu Asn Lys Ala Lys Ser
                   565                 570                 575
<210> 23
   <211> 568
   <212> PRT
   <213> Artificial Sequence
<220>
   <223> ORF0657nH
<400> 23
   Ala Glu Glu Thr Gly Gly Thr Asn Thr Glu Ala Gln Pro Lys Thr Glu
   1               5                   10                  15
   Ala Val Ala Ser Pro Ser Thr Thr Thr Glu Lys Ala Pro Glu Ala Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Glu Val Lys Pro Ala Thr Lys Ala Asp Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Lys Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asn Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Gly
       210                 215                 220
   Lys Phe Lys Thr Glu Glu Asp Tyr Lys Ala Glu Lys Leu Leu Ser Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Glu Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Ala Asp Lys Ser Asn Lys Lys Glu Gln Gln Asp Asn Ser Ala Lys
               420                 425                 430
   Lys Glu Thr Thr Pro Ala Thr Pro Ser Lys Pro Thr Thr Pro Pro Val
           435                 440                 445
   Glu Lys Glu Ser Gln Lys Gln Asp Ser Gln Lys Asp Asp Asn Lys Gln
       450                 455                 460
   Ser Pro Ser Val Glu Lys Glu Asn Asp Ala Ser Ser Glu Ser Gly Lys
   465                 470                 475                 480
   Asp Lys Thr Pro Thr Thr Lys Pro Ala Lys Ala Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Glu Thr Thr Ile Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Glu Asn Asn Lys Asn
   545                 550                 555                 560
   Thr Gln Glu Asn Lys Ala Lys Ser
                   565
<210> 24
   <211> 568
   <212> PRT
   <213> Artificial Sequence
<220>
   <223> ORF0657nH
<400> 24
   Ala Glu Glu Thr Gly Gly Thr Asn Thr Glu Ala Gln Pro Lys Thr Glu
   1               5                   10                  15
   Ala Val Ala Ser Pro Ser Thr Thr Thr Glu Lys Ala Pro Glu Ala Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Glu Val Lys Pro Ala Thr Lys Ala Asp Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Lys Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asn Lys Lys Leu Pro Ile Lys Leu Val Ser Tyr Asp Thr Val
   165 170 175
   Lys Asp Tyr Ala Tyr Ile Arg Phe Ser Val Ser Asn Gly Thr Lys Ala
   180 185 190
   Val Lys Ile Val Ser Ser Thr His Phe Asn Asn Lys Glu Glu Lys Tyr
   195 200 205
   Asp Tyr Thr Leu Met Glu Phe Ala Gln Pro Ile Tyr Asn Ser Ala Asp
   210 215 220
   Lys Phe Lys Thr Glu Glu Asp Tyr Lys Ala Glu Lys Leu Leu Ser Pro
   225 230 235 240
   Tyr Lys Lys Ala Lys Thr Leu Glu Arg Gln Val Tyr Glu Leu Asn Lys
   245 250 255
   Ile Gln Asp Lys Leu Pro Glu Lys Leu Lys Ala Glu Tyr Lys Lys Lys
   260 265 270
   Leu Glu Glu Thr Lys Lys Ala Leu Asp Glu Gln Val Lys Ser Ala Ile
   275 280 285
   Thr Glu Phe Gln Asn Val Gln Pro Thr Asn Glu Lys Met Thr Asp Leu
   290 295 300
   Gln Asp Thr Lys Tyr Val Val Tyr Glu Ser Val Glu Asn Asn Glu Ser
   305 310 315 320
   Met Met Asp Thr Phe Val Lys His Pro Ile Lys Thr Gly Met Leu Asn
   325 330 335
   Gly Lys Lys Tyr Met Val Met Glu Thr Thr Asn Asp Asp Tyr Trp Lys
   340 345 350
   Asp Phe Met Val Glu Gly Gln Arg Val Arg Thr Ile Ser Lys Asp Ala
   355 360 365
   Lys Asn Asn Thr Arg Thr Ile Ile Phe Pro Tyr Val Glu Gly Lys Thr
   370 375 380
   Leu Tyr Asp Ala Ile Val Lys Val His Val Lys Thr Ile Asp Tyr Asp
   385 390 395 400
   Gly Gln Tyr His Val Arg Ile Val Asp Lys Glu Ala Phe Thr Lys Ala
   405 410 415
   Asn Ala Asp Lys Ser Asn Lys Lys Glu Gln Gln Asp Asn Ser Ala Lys
   420 425 430
   Lys Glu Thr Thr Pro Ala Thr Pro Ser Lys Pro Thr Thr Pro Pro Val
   435 440 445
   Glu Lys Glu Ser Gln Lys Gln Asp Ser Gln Lys Asp Asp Asn Lys Gln
   450 455 460
   Ser Pro Ser Val Glu Lys Glu Asn Asp Ala Ser Ser Glu Ser Gly Lys
   465 470 475 480
   Asp Lys Thr Pro Ala Thr Lys Pro Ala Lys Ala Glu Val Glu Ser Ser
   485 490 495
   Ser Thr Thr Pro Thr Lys Val Val Ser Thr Thr Gln Asn Val Ala Lys
   500 505 510
   Pro Thr Thr Ala Ser Ser Glu Thr Thr Ile Asp Val Val Gln Thr Ser
   515 520 525
   Ala Gly Ser Ser Glu Ala Lys Asp Ser Ala Pro Leu Gln Lys Ala Asn
   530 535 540
   Ile Lys Asn Thr Asn Asp Gly His Thr Gln Ser Glu Asn Asn Lys Asn
   545 550 555 560
   Thr Gln Glu Asn Lys Ala Lys Ser
   565
<210> 25
   <211> 568
   <212> PRT
   <213> Artificial Sequence
<220>
   <223> ORF0657nH
<400> 25
   Ala Glu Glu Thr Gly Gly Thr Ile Thr Glu Thr Gln Pro Lys Thr Glu
    1               5                  10                  15
   Ala Val Ala Ser Pro Thr Thr Thr Thr Glu Lys Ala Pro Glu Ala Lys
               20                  25                  30
   Pro Val Ala Asn Ala Val Ser Val Ser Asn Lys Glu Val Ala Ala Pro
           35                  40                  45
   Thr Thr Glu Thr Lys Glu Ala Lys Glu Val Lys Glu Val Lys Ala Pro
       50                  55                  60
   Asn Glu Thr Lys Glu Val Lys Pro Ala Ala Lys Ser Asp Asn Asn Thr
   65                  70                  75                  80
   Tyr Pro Ile Leu Asn Glu Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Lys Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Glu Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Glu Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Ala Asp Lys Ser Asn Lys Lys Glu Gln Gln Asp Asn Ser Ala Lys
               420                 425                 430
   Lys Glu Thr Thr Pro Ala Thr Pro Ser Lys Pro Thr Thr Ala Pro Val
           435                 440                 445
   Glu Lys Glu Ser Gln Lys Gln Asp Ser Gln Lys Asp Asp Asn Lys Gln
       450                 455                 460
   Ser Pro Ser Val Glu Lys Glu Ile Asp Ala Ser Ser Glu Ser Gly Lys
   465                 470                 475                 480
   Asp Lys Thr Pro Ala Thr Lys Pro Ala Lys Gly Glu Val Glu Ser Ser
                   485                 490                 495
   Ser Thr Thr Pro Thr Lys Val Val Ser Ala Thr Gln Asn Val Ala Lys
               500                 505                 510
   Pro Thr Ser Ala Ser Ser Glu Thr Thr Lys Gly Val Val Gln Thr Ser
           515                 520                 525
   Ala Gly Ser Ser Glu Ala Lys Asp Asn Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 26
   <211> 568
   <212> PRT
   <213> Artificial Sequence
<220>
   <223> ORF0657nH
<400> 26
   Ala Glu Glu Thr Gly Gly Thr Ile Thr Glu Thr Gln Pro Lys Thr Glu
   1               5                   10                  15
   Ala Val Ala Ser Pro Thr Thr Thr Thr Glu Lys Ala Pro Glu Ala Lys
               20                  25                  30
   Pro Val Ala Asn Ala Val Ser Val Ser Asn Lys Glu Val Val Ala Pro
           35                  40                  45
   Thr Thr Glu Thr Lys Glu Ala Lys Glu Val Lys Glu Val Lys Ala Pro
       50                  55                  60
   Asn Glu Thr Lys Glu Val Lys Pro Ala Ala Lys Ser Asp Asn Asn Thr
   65                  70                  75                  80
   Tyr Pro Ile Leu Asn Glu Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Lys Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
   165 170 175
   Lys Asp Tyr Ala Tyr Ile Arg Phe Ser Val Ser Asn Gly Thr Lys Ala
   180 185 190
   Val Lys Ile Val Ser Ser Thr His Phe Asn Asn Lys Glu Glu Lys Tyr
   195 200 205
   Asp Tyr Thr Leu Met Glu Phe Ala Gln Pro Ile Tyr Asn Ser Ala Asp
   210 215 220
   Lys Phe Lys Thr Glu Glu Asp Tyr Lys Ala Glu Lys Leu Leu Ala Pro
   225 230 235 240
   Tyr Lys Lys Ala Lys Thr Leu Glu Arg Gln Val Tyr Glu Leu Asn Lys
   245 250 255
   Ile Gln Asp Lys Leu Pro Glu Lys Leu Lys Ala Glu Tyr Lys Lys Lys
   260 265 270
   Leu Glu Glu Thr Lys Lys Ala Leu Asp Glu Gln Val Lys Ser Ala Ile
   275 280 285
   Thr Glu Phe Gln Asn Val Gln Pro Thr Asn Glu Lys Met Thr Asp Leu
   290 295 300
   Gln Asp Thr Lys Tyr Val Val Tyr Glu Ser Val Glu Asn Asn Glu Ser
   305 310 315 320
   Met Met Asp Thr Phe Val Lys His Pro Ile Lys Thr Gly Met Leu Asn
   325 330 335
   Gly Lys Lys Tyr Met Val Met Glu Thr Thr Asn Asp Asp Tyr Trp Lys
   340 345 350
   Asp Phe Met Val Glu Gly Glu Arg Val Arg Thr Ile Ser Lys Asp Ala
   355 360 365
   Lys Asn Asn Thr Arg Thr Ile Ile Phe Pro Tyr Val Glu Gly Lys Thr
   370 375 380
   Leu Tyr Asp Ala Ile Val Lys Val His Val Lys Thr Ile Asp Tyr Asp
   385 390 395 400
   Gly Gln Tyr His Val Arg Ile Val Asp Lys Glu Ala Phe Thr Lys Ala
   405 410 415
   Asn Ala Asp Lys Ser Asn Lys Lys Glu Gln Gln Asp Asn Ser Ala Lys
   420 425 430
   Lys Glu Ala Thr Pro Ala Thr Pro Ser Lys Pro Thr Thr Ala Pro Val
   435 440 445
   Glu Lys Glu Ser Gln Lys Gln Asp Ser Gln Lys Asp Asp Asn Lys Gln
   450 455 460
   Ser Pro Ser Val Glu Lys Glu Ile Asp Ala Ser Ser Glu Ser Gly Lys
   465 470 475 480
   Asp Lys Thr Pro Ala Thr Lys Pro Ala Lys Gly Glu Val Glu Ser Ser
   485 490 495
   Ser Thr Thr Pro Thr Lys Val Val Ser Ala Thr Gln Asn Val Ala Lys
   500 505 510
   Pro Thr Ser Ala Ser Ser Glu Thr Thr Lys Gly Val Val Gln Thr Ser
   515 520 525
   Ala Gly Ser Ser Glu Ala Lys Asp Asn Ala Pro Leu Gln Lys Ala Asn
   530 535 540
   Ile Lys Asn Thr Asn Asp Gly His Thr Gln Ser Gln Asn Asn Lys Asn
   545 550 555 560
   Thr Gln Glu Asn Lys Ala Lys Ser
   565
<210> 27
   <211> 570
   <212> PRT
   <213> Artificial Sequence
<220>
   <223> ORF0657nH
<400> 27
   Met Gly Ala Glu Glu Thr Gly Gly Thr Asn Thr Glu Ala Gln Pro Lys
   1               5                   10                  15
   Thr Glu Ala Val Ala Ser Pro Thr Thr Thr Ser Glu Lys Ala Pro Glu
               20                  25                  30
   Thr Lys Pro Val Ala Asn Ala Val Ser Val Ser Asn Lys Glu Val Glu
           35                  40                  45
   Ala Pro Thr Ser Glu Thr Lys Glu Ala Lys Glu Val Lys Glu Val Lys
       50                  55                  60
   Ala Pro Lys Glu Thr Lys Glu Val Lys Pro Ala Ala Lys Ala Thr Asn
   65                  70                  75                  80
   Asn Thr Tyr Pro Ile Leu Asn Gln Glu Leu Arg Glu Ala Ile Lys Asn
                   85                  90                  95
   Pro Ala Ile Lys Asp Lys Asp His Ser Ala Pro Asn Ser Arg Pro Ile
               100                 105                 110
   Asp Phe Glu Met Lys Lys Lys Asp Gly Thr Gln Gln Phe Tyr His Tyr
           115                 120                 125
   Ala Ser Ser Val Lys Pro Ala Arg Val Ile Phe Thr Asp Ser Lys Pro
       130                 135                 140
   Glu Ile Glu Leu Gly Leu Gln Ser Gly Gln Phe Trp Arg Lys Phe Glu
   145                 150                 155                 160
   Val Tyr Glu Gly Asp Lys Lys Leu Pro Ile Lys Leu Val Ser Tyr Asp
                   165                 170                 175
   Thr Val Lys Asp Tyr Ala Tyr Ile Arg Phe Ser Val Ser Asn Gly Thr
               180                 185                 190
   Lys Ala Val Lys Ile Val Ser Ser Thr His Phe Asn Asn Lys Glu Glu
           195                 200                 205
   Lys Tyr Asp Tyr Thr Leu Met Glu Phe Ala Gln Pro Ile Tyr Asn Ser
       210                 215                 220
   Ala Asp Lys Phe Lys Thr Glu Glu Asp Tyr Lys Ala Glu Lys Leu Leu
   225                 230                 235                 240
   Ala Pro Tyr Lys Lys Ala Lys Thr Leu Glu Arg Gln Val Tyr Glu Leu
                   245                 250                 255
   Asn Lys Ile Gln Asp Lys Leu Pro Glu Lys Leu Lys Ala Glu Tyr Lys
               260                 265                 270
   Lys Lys Leu Glu Asp Thr Lys Lys Ala Leu Asp Glu Gln Val Lys Ser
           275                 280                 285
   Ala Ile Thr Glu Phe Gln Asn Val Gln Pro Thr Asn Glu Lys Met Thr
       290                 295                 300
   Asp Leu Gln Asp Thr Lys Tyr Val Val Tyr Glu Ser Val Glu Asn Asn
   305                 310                 315                 320
   Glu Ser Met Met Asp Thr Phe Val Lys His Pro Ile Lys Thr Gly Met
                   325                 330                 335
   Leu Asn Gly Lys Lys Tyr Met Val Met Glu Thr Thr Asn Asp Asp Tyr
               340                 345                 350
   Trp Lys Asp Phe Met Val Glu Gly Gln Arg Val Arg Thr Ile Ser Lys
           355                 360                 365
   Asp Ala Lys Asn Asn Thr Arg Thr Ile Ile Phe Pro Tyr Val Glu Gly
       370                 375                 380
   Lys Thr Leu Tyr Asp Ala Ile Val Lys Val His Val Lys Thr Ile Asp
   385 390 395 400
   Tyr Asp Gly Gln Tyr His Val Arg Ile Val Asp Lys Glu Ala Phe Thr
   405 410 415
   Lys Ala Asn Thr Asp Lys Ser Asn Lys Lys Glu Gln Gln Asp Asn Ser
   420 425 430
   Ala Lys Lys Glu Ala Thr Pro Ala Thr Pro Ser Lys Pro Thr Pro Ser
   435 440 445
   Pro Val Glu Lys Glu Ser Gln Lys Gln Asp Ser Gln Lys Asp Asp Asn
   450 455 460
   Lys Gln Leu Pro Ser Val Glu Lys Glu Asn Asp Ala Ser Ser Glu Ser
   465 470 475 480
   Gly Lys Gly Val Thr Leu Ala Thr Lys Pro Thr Lys Gly Glu Val Glu
   485 490 495
   Ser Ser Ser Thr Thr Pro Thr Lys Val Val Ser Thr Thr Gln Asn Val
   500 505 510
   Ala Lys Pro Thr Thr Gly Ser Ser Lys Thr Thr Lys Asp Val Val Gln
   515 520 525
   Thr Ser Ala Gly Ser Ser Glu Ala Lys Asp Ser Ala Pro Leu Gln Lys
   530 535 540
   Ala Asn Ile Lys His Thr Asn Asp Gly His Thr Gln Ser Gln Asn Asn
   545 550 555 560
   Lys Asn Thr Gln Glu Asn Lys Ala Lys Ser
   565 570
<210> 28
   <211> 654
   <212> PRT
   <213> Artificial Sequence
<220>
   <223> SEQ ID NO: 2 modified to contain a glycine after the amino terminus methionine and a carboxyl His-Tag
<400> 28
   Met Gly Asn Lys Gln Gln Lys Glu Phe Lys Ser Phe Tyr Ser Ile Arg
   1 5 10 15
   Lys Ser Ser Leu Gly Val Ala Ser Val Ala Ile Ser Thr Leu Leu Leu
   20 25 30
   Leu Met Ser Asn Gly Glu Ala Gln Ala Ala Ala Glu Glu Thr Gly Gly
   35 40 45
   Thr Asn Thr Glu Ala Gln Pro Lys Thr Glu Ala Val Ala Ser Pro Thr
   50 55 60
   Thr Thr Ser Glu Lys Ala Pro Glu Thr Lys Pro Val Ala Asn Ala Val
   65 70 75 80
   Ser Val Ser Asn Lys Glu Val Glu Ala Pro Thr Ser Glu Thr Lys Glu
   85 90 95
   Ala Lys Glu Val Lys Glu Val Lys Ala Pro Lys Glu Thr Lys Glu Val
   100 105 110
   Lys Pro Ala Ala Lys Ala Thr Asn Asn Thr Tyr Pro Ile Leu Asn Gln
   115 120 125
   Glu Leu Arg Glu Ala Ile Lys Asn Pro Ala Ile Lys Asp Lys Asp His
   130 135 140
   Ser Ala Pro Asn Ser Arg Pro Ile Asp Phe Glu Met Lys Lys Lys Asp
   145                 150                 155                 160
   Gly Thr Gln Gln Phe Tyr His Tyr Ala Ser Ser Val Lys Pro Ala Arg
                   165                 170                 175
   Val Ile Phe Thr Asp Ser Lys Pro Glu Ile Glu Leu Gly Leu Gln Ser
               180                 185                 190
   Gly Gln Phe Trp Arg Lys Phe Glu Val Tyr Glu Gly Asp Lys Lys Leu
           195                 200                 205
   Pro Ile Lys Leu Val Ser Tyr Asp Thr Val Lys Asp Tyr Ala Tyr Ile
       210                 215                 220
   Arg Phe Ser Val Ser Asn Gly Thr Lys Ala Val Lys Ile Val Ser Ser
   225                 230                 235                 240
   Thr His Phe Asn Asn Lys Glu Glu Lys Tyr Asp Tyr Thr Leu Met Glu
                   245                 250                 255
   Phe Ala Gln Pro Ile Tyr Asn Ser Ala Asp Lys Phe Lys Thr Glu Glu
               260                 265                 270
   Asp Tyr Lys Ala Glu Lys Leu Leu Ala Pro Tyr Lys Lys Ala Lys Thr
           275                 280                 285
   Leu Glu Arg Gln Val Tyr Glu Leu Asn Lys Ile Gln Asp Lys Leu Pro
       290                 295                 300
   Glu Lys Leu Lys Ala Glu Tyr Lys Lys Lys Leu Glu Asp Thr Lys Lys
   305                 310                 315                 320
   Ala Leu Asp Glu Gln Val Lys Ser Ala Ile Thr Glu Phe Gln Asn Val
                   325                 330                 335
   Gln Pro Thr Asn Glu Lys Met Thr Asp Leu Gln Asp Thr Lys Tyr Val
               340                 345                 350
   Val Tyr Glu Ser Val Glu Asn Asn Glu Ser Met Met Asp Thr Phe Val
           355                 360                 365
   Lys His Pro Ile Lys Thr Gly Met Leu Asn Gly Lys Lys Tyr Met Val
       370                 375                 380
   Met Glu Thr Thr Asn Asp Asp Tyr Trp Lys Asp Phe Met Val Glu Gly
   385                 390                 395                 400
   Gln Arg Val Arg Thr Ile Ser Lys Asp Ala Lys Asn Asn Thr Arg Thr
                   405                 410                 415
   Ile Ile Phe Pro Tyr Val Glu Gly Lys Thr Leu Tyr Asp Ala Ile Val
               420                 425                 430
   Lys Val His Val Lys Thr Ile Asp Tyr Asp Gly Gln Tyr His Val Arg
           435                 440                 445
   Ile Val Asp Lys Glu Ala Phe Thr Lys Ala Asn Thr Asp Lys Ser Asn
       450                 455                 460
   Lys Lys Glu Gln Gln Asp Asn Ser Ala Lys Lys Glu Ala Thr Pro Ala
   465                 470                 475                 480
   Thr Pro Ser Lys Pro Thr Pro Ser Pro Val Glu Lys Glu Ser Gln Lys
                   485                 490                 495
   Gln Asp Ser Gln Lys Asp Asp Asn Lys Gln Leu Pro Ser Val Glu Lys
               500                 505                 510
   Glu Asn Asp Ala Ser Ser Glu Ser Gly Lys Asp Lys Thr Pro Ala Thr
           515                 520                 525
   Lys Pro Thr Lys Gly Glu Val Glu Ser Ser Ser Thr Thr Pro Thr Lys
       530                 535                 540
   Val Val Ser Thr Thr Gln Asn Val Ala Lys Pro Thr Thr Ala Ser Ser
   545                 550                 555                 560
   Lys Thr Thr Lys Asp Val Val Gln Thr Ser Ala Gly Ser Ser Glu Ala
                   565                 570                 575
   Lys Asp Ser Ala Pro Leu Gln Lys Ala Asn Ile Lys Asn Thr Asn Asp
               580                 585                 590
   Gly His Thr Gln Ser Gln Asn Asn Lys Asn Thr Gln Glu Asn Lys Ala
           595                 600                 605
   Lys Ser Leu Pro Gln Thr Gly Glu Glu Ser Asn Lys Asp Met Thr Leu
       610                 615                 620
   Pro Leu Met Ala Leu Leu Ala Leu Ser Ser Ile Val Ala Phe Val Leu
   625                 630                 635                 640
   Pro Arg Lys Arg Lys Asn Leu Glu His His His His His His
                   645                 650
<210> 29
   <211> 1962
   <212> DNA
   <213> Artificial Sequence
<220>
   <223> Full length ORF0657n + Carboxyl His-Tag
<400> 29
   atgaacaaac agcaaaaaga atttaaatca ttttattcaa ttagaaagtc atcactaggc 60
   gttgcatctg tagcaattag tacactttta ttattaatgt caaatggcga agcacaagca 120
   gcagctgaag aaacaggtgg tacaaataca gaagcacaac caaaaactga agcagttgca 180
   agtccaacaa caacatctga aaaagctcca gaaactaaac cagtagctaa tgctgtctca 240
   gtatctaata aagaagttga ggcccctact tctgaaacaa aagaagctaa agaagttaaa 300
   gaagttaaag cccctaagga aacaaaagaa gttaaaccag cagcaaaagc cactaacaat 360
   acatatccta ttttgaatca ggaacttaga gaagcgatta aaaaccctgc aataaaagac 420
   aaagatcata gcgcaccaaa ctctcgtcca attgattttg aaatgaaaaa gaaagatgga 480
   actcaacagt tttatcatta tgcaagttct gttaaacctg ctagagttat tttcactgat 540
   tcaaaaccag aaattgaatt aggattacaa tcaggtcaat tttggagaaa atttgaagtt 600
   tatgaaggtg acaaaaagtt gccaattaaa ttagtatcat acgatactgt taaagattat 660
   gcttacattc gcttctctgt atcaaacgga acaaaagctg ttaaaattgt tagttcaaca 720
   cacttcaata acaaagaaga aaaatacgat tacacattaa tggaattcgc acaaccaatt 780
   tataacagtg cagataaatt caaaactgaa gaagattata aagctgaaaa attattagcg 840
   ccatataaaa aagcgaaaac actagaaaga caagtttatg aattaaataa aattcaagat 900
   aaacttcctg aaaaattaaa ggctgagtac aagaagaaat tagaggatac aaagaaagct 960
   ttagatgagc aagtgaaatc agctattact gaattccaaa atgtacaacc aacaaatgaa 1020
   aaaatgactg atttacaaga tacaaaatat gttgtttatg aaagtgttga gaataacgaa 1080
   tctatgatgg atacttttgt taaacaccct attaaaacag gtatgcttaa cggcaaaaaa 1140
   tatatggtca tggaaactac taatgacgat tactggaaag atttcatggt tgaaggtcaa 1200
   cgtgttagaa ctataagcaa agatgctaaa aataatacta gaacaattat tttcccatat 1260
   gttgaaggta aaactctata tgatgctatc gttaaagttc acgtaaaaac gattgattat 1320
   gatggacaat accatgtcag aatcgttgat aaagaagcat ttacaaaagc caataccgat 1380
   aaatctaaca aaaaagaaca acaagataac tcagctaaga aggaagctac tccagctacg 1440
   cctagcaaac caacaccatc acctgttgaa aaagaatcac aaaaacaaga cagccaaaaa 1500
   gatgacaata aacaattacc aagtgttgaa aaagaaaatg acgcatctag tgagtcaggt 1560
   aaagacaaaa cgcctgctac aaaaccaact aaaggtgaag tagaatcaag tagtacaact 1620
   ccaactaagg tagtatctac gactcaaaat gttgcaaaac caacaactgc ttcatcaaaa 1680
   acaacaaaag atgttgttca aacttcagca ggttctagcg aagcaaaaga tagtgctcca 1740
   ttacaaaaag caaacattaa aaacacaaat gatggacaca ctcaaagcca aaacaataaa 1800
   aatacacaag aaaataaagc aaaatcatta ccacaaactg gtgaagaatc aaataaagat 1860
   atgacattac cattaatggc attattagct ttaagtagca tcgttgcatt cgtattacct 1920
   agaaaacgta aaaacctcga gcaccaccac caccaccact ga                   1962
<210> 30
   <211> 1737
   <212> DNA
   <213> Artificial Sequence
<220>
   <223> ORF0657nH + Carboxyl His-Tag
<400> 30
   atgaacgctg aagaaacagg tggtacaaat acagaagcac aaccaaaaac tgaagcagtt 60
   gcaagtccaa caacaacatc tgaaaaagct ccagaaacta aaccagtagc taatgctgtc 120
   tcagtatcta ataaagaagt tgaggcccct acttctgaaa caaaagaagc taaagaagtt 180
   aaagaagtta aagcccctaa ggaaacaaaa gaagttaaac cagcagcaaa agccactaac 240
   aatacatatc ctattttgaa tcaggaactt agagaagcga ttaaaaaccc tgcaataaaa 300
   gacaaagatc atagcgcacc aaactctcgt ccaattgatt ttgaaatgaa aaagaaagat 360
   ggaactcaac agttttatca ttatgcaagt tctgttaaac ctgctagagt tattttcact 420
   gattcaaaac cagaaattga attaggatta caatcaggtc aattttggag aaaatttgaa 480
   gtttatgaag gtgacaaaaa gttgccaatt aaattagtat catacgatac tgttaaagat 540
   tatgcttaca ttcgcttctc tgtatcaaac ggaacaaaag ctgttaaaat tgttagttca 600
   acacacttca ataacaaaga agaaaaatac gattacacat taatggaatt cgcacaacca 660
   atttataaca gtgcagataa attcaaaact gaagaagatt ataaagctga aaaattatta 720
   gcgccatata aaaaagcgaa aacactagaa agacaagttt atgaattaaa taaaattcaa 780
   gataaacttc ctgaaaaatt aaaggctgag tacaagaaga aattagagga tacaaagaaa 840
   gctttagatg agcaagtgaa atcagctatt actgaattcc aaaatgtaca accaacaaat 900
   gaaaaaatga ctgatttaca agatacaaaa tatgttgttt atgaaagtgt tgagaataac 960
   gaatctatga tggatacttt tgttaaacac cctattaaaa caggtatgct taacggcaaa 1020
   aaatatatgg tcatggaaac tactaatgac gattactgga aagatttcat ggttgaaggt 1080
   caacgtgtta gaactataag caaagatgct aaaaataata ctagaacaat tattttccca 1140
   tatgttgaag gtaaaactct atatgatgct atcgttaaag ttcacgtaaa aacgattgat 1200
   tatgatggac aataccatgt cagaatcgtt gataaagaag catttacaaa agccaatacc 1260
   gataaatcta acaaaaaaga acaacaagat aactcagcta agaaggaagc tactccagct 1320
   acgcctagca aaccaacacc atcacctgtt gaaaaagaat cacaaaaaca agacagccaa 1380
   aaagatgaca ataaacaatt accaagtgtt gaaaaagaaa atgacgcatc tagtgagtca 1440
   ggtaaagaca aaacgcctgc tacaaaacca actaaaggtg aagtagaatc aagtagtaca 1500
   actccaacta aggtagtatc tacgactcaa aatgttgcaa aaccaacaac tgcttcatca 1560
   aaaacaacaa aagatgttgt tcaaacttca gcaggttcta gcgaagcaaa agatagtgct 1620
   ccattacaaa aagcaaacat taaaaacaca aatgatggac acactcaaag ccaaaacaat 1680
   aaaaatacac aagaaaataa agcaaaatca ctcgagcacc accaccacca ccactga    1737
<210> 31
   <211> 1941
   <212> DNA
   <213> Artificial Sequence
<220>
   <223> Encodes SEQ ID NO: 28 without a carboxyl His-Tag and is codon optimized for yeast expression
<400> 31
   atgggtaaca agcaacaaaa ggaattcaag tctttctact ccattagaaa gtcttccttg 60
   ggtgttgctt ctgtcgctat ctccaccttg ttgttgttga tgtctaacgg tgaagctcaa 120
   gctgctgctg aagaaactgg tggtaccaac actgaagctc aaccaaagac cgaagctgtc 180
   gcttccccaa ccactacctc tgaaaaggct ccagaaacta agccagttgc taacgctgtc 240
   tccgtttcta acaaggaagt cgaagctcca acctccgaaa ctaaggaagc taaggaagtt 300
   aaggaagtca aggctccaaa ggaaactaag gaagtcaagc cagctgctaa ggctaccaac 360
   aacacttacc caattttgaa ccaagaattg agagaagcta ttaagaaccc agctatcaag 420
   gacaaggacc actccgctcc aaactctaga ccaatcgact tcgaaatgaa gaagaaggac 480
   ggtacccaac aattctacca ctacgcgtcc tctgtcaagc cagctagagt tattttcacc 540
   gactctaagc cagaaatcga attgggtttg caatccggtc aattctggag aaagttcgaa 600
   gtctacgaag gtgacaagaa gttgccaatt aagttggttt cctacgacac cgtcaaggac 660
   tacgcttaca tcagattctc cgtttctaac ggtactaagg ctgtcaagat tgtctcttcc 720
   acccacttca acaacaagga agaaaagtac gactacactt tgatggaatt cgctcaacca 780
   atttacaact ctgctgacaa gttcaagacc gaagaagact acaaggctga aaagttgttg 840
   gctccataca agaaggctaa gactttggaa agacaagttt acgaattgaa caagatccaa 900
   gacaagttgc cagaaaagtt gaaggctgaa tacaagaaga agttggaaga caccaagaag 960
   gctttggacg aacaagtcaa gtccgctatc accgaattcc aaaacgttca accaactaac 1020
   gaaaagatga ctgacttgca agacactaag tacgtcgtct acgaatccgt cgaaaacaac 1080
   gaatccatga tggacacctt cgttaagcac ccaattaaga ctggtatgtt gaacggtaag 1140
   aagtacatgg tcatggaaac cactaacgac gactactgga aggacttcat ggttgaaggt 1200
   caaagagtca gaaccatctc caaggacgct aagaacaaca ctagaaccat tatcttccca 1260
   tacgttgaag gtaagacttt gtacgacgct atcgtcaagg ttcacgtcaa gactattgac 1320
   tacgacggtc aataccacgt tagaattgtt gacaaggaag ctttcaccaa ggctaacacc 1380
   gacaagtcca acaagaagga acaacaagac aactctgcta agaaggaagc taccccagct 1440
   accccatcta agccaacccc atctccagtt gaaaaggaat ctcaaaagca agactcccaa 1500
   aaggacgaca acaagcaatt gccatccgtc gaaaaggaaa acgacgcgtc ttctgaatcc 1560
   ggtaaggaca agactccagc taccaagcca actaagggtg aagttgaatc ttcctctact 1620
   actccaacca aggttgtctc cactacccaa aacgtcgcta agccaactac cgcttcttcc 1680
   aagactacca aggacgttgt ccaaacttct gctggttcct ctgaagctaa ggactctgct 1740
   ccattgcaaa aggctaacat caagaacacc aacgacggtc acacccaatc ccaaaacaac 1800
   aagaacactc aagaaaacaa ggctaagtct ttgccacaaa ccggtgaaga atccaacaag 1860
   gacatgacct tgccattgat ggctttgttg gctttgtctt ccatcgttgc tttcgtcttg 1920
   ccaagaaaga gaaagaacta a                                           1941
<210> 32
   <211> 1710
   <212> DNA
   <213> Artificial Sequence
<220>
   <223> Encodes SEQ ID NO: 3 and is codon optimized for yeast expression
<400> 32
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aaggacaaga ctccagctac caagccaact aagggtgaag ttgaatcttc ctctactact 1500
   ccaaccaagg ttgtctccac tacccaaaac gtcgctaagc caactaccgc ttcttccaag 1560
   actaccaagg acgttgtcca aacttctgct ggttcctctg aagctaagga ctctgctcca 1620
   ttgcaaaagg ctaacatcaa gaacaccaac gacggtcaca cccaatccca aaacaacaag 1680
   aacactcaag aaaacaaggc taagtcttaa                                  1710
<210> 33
   <211> 1341
<212> DNA
   <213> Artificial Sequence
<220>
   <223> Encodes SEQ ID NO: 1 and is codon optimized for yeast expression
<400> 33
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccata a                                           1341
<210> 34
   <211> 1710
   <212> DNA
   <213> Artificial Sequence
<220>
   <223> Encodes SEQ ID NO: 7 containing an amino terminus methionine and is codon optimized for yeast expression
<400> 34
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagggtgtca ctttggctac caagccaact aagggtgaag ttgaatcttc ctctactact 1500
   ccaaccaagg ttgtctccac tacccaaaac gtcgctaagc caactaccgg ttcttccaag 1560
   actaccaagg acgttgtcca aacttctgct ggttcctctg aagctaagga ctctgctcca 1620
   ttgcaaaagg ctaacatcaa gcacaccaac gacggtcaca cccaatccca aaacaacaag 1680
   aacactcaag aaaacaaggc taagtcttaa                                 1710
<210>
   <211> 1710
   <212> DNA
   <213> Artificial Sequence
<220>
   <223> Encodes SEQ ID NO: 7 containing an amino terminus methionine and is codon optimized for yeast expression
<400> 35
<210> 36
   <211> 1710
   <212> DNA
   <213> Artificial Sequence
<220>
   <223> Encodes SEQ ID NO: 7 containing an amino terminus methionine and is codon optimized for yeast expression
<400> 36
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagggtgtta ctttggctac caagccaact aagggtgaag ttgaatcttc ctctactact 1500
   ccaaccaagg ttgtctccac tacccaaaac gtcgctaagc caactaccgg ttcttccaag 1560
   actaccaagg acgttgtcca aacttctgct ggttcctctg aagctaagga ctctgctcca 1620
   ttgcaaaagg ctaacatcaa gcacaccaac gacggtcaca cccaatccca aaacaacaag 1680
   aacactcaag aaaacaaggc taagtcttaa 1710
<210> 37
   <211> 1710
   <212> DNA
   <213> Artificial Sequence
<220>
   <223> Encodes SEQ ID NO: 7 containing an amino terminus methionine and is codon optimized for yeast expression
<400> 37
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagggcgtta ctttggctac caagccaact aagggtgaag ttgaatcttc ctctactact 1500
   ccaaccaagg ttgtctccac tacccaaaac gtcgctaagc caactaccgg ttcttccaag 1560
   actaccaagg acgttgtcca aacttctgct ggttcctctg aagctaagga ctctgctcca 1620
   ttgcaaaagg ctaacatcaa gcacaccaac gacggtcaca cccaatccca aaacaacaag 1680
   aacactcaag aaaacaaggc taagtcttaa 1710
<210> 38
   <211> 1710
   <212> DNA
   <213> Artificial Sequence
<220>
   <223> Encodes SEQ ID NO: 7 containing an amino terminus methionine and is codon optimized for yeast expression
<400> 38
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagggtgtca ctttagctac caagccaact aagggtgaag ttgaatcttc ctctactact 1500
   ccaaccaagg ttgtctccac tacccaaaac gtcgctaagc caactaccgg ttcttccaag 1560
   actaccaagg acgttgtcca aacttctgct ggttcctctg aagctaagga ctctgctcca 1620
   ttgcaaaagg ctaacatcaa gcacaccaac gacggtcaca cccaatccca aaacaacaag 1680
   aacactcaag aaaacaaggc taagtcttaa                                  1710
<210> 39
   <211> 1710
   <212> DNA
   <213> Artificial Sequence
<220>
   <223> Encodes SEQ ID NO: 7 containing an amino terminus methionine and is codon optimized for yeast expression
<400> 39
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagggtgtca ctttggctac caagccaact aagggtgaag ttgaatcttc ctctactact 1500
   ccaaccaagg ttgtctccac tacccaaaac gtcgctaagc caactaccgg ctcttccaag 1560
   actaccaagg acgttgtcca aacttctgct ggttcctctg aagctaagga ctctgctcca 1620
   ttgcaaaagg ctaacatcaa gcacaccaac gacggtcaca cccaatccca aaacaacaag 1680
   aacactcaag aaaacaaggc taagtcttaa                                 1710
<210> 40
   <211> 1710
   <212> DNA
   <213> Artificial Sequence
<220>
   <223> Encodes SEQ ID NO: 7 containing an amino terminus methionine and is codon optimized for yeast expression
<400> 40
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagggtgtta ctttagctac caagccaact aagggtgaag ttgaatcttc ctctactact 1500
   ccaaccaagg ttgtctccac tacccaaaac gtcgctaagc caactaccgg ttcttccaag 1560
   actaccaagg acgttgtcca aacttctgct ggttcctctg aagctaagga ctctgctcca 1620
   ttgcaaaagg ctaacatcaa gcacaccaac gacggtcaca cccaatccca aaacaacaag 1680
   aacactcaag aaaacaaggc taagtcttaa 1710
<210> 41
   <211> 1710
   <212> DNA
   <213> Artificial Sequence
<220>
   <223> Encodes SEQ ID NO: 7 containing an amino terminus methionine and is codon optimized for yeast expression
<400> 41
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagggtgtta ctttggctac caagccaact aagggtgaag ttgaatcttc ctctactact 1500
   ccaaccaagg ttgtctccac tacccaaaac gtcgctaagc caactaccgg ctcttccaag 1560
   actaccaagg acgttgtcca aacttctgct ggttcctctg aagctaagga ctctgctcca 1620
   ttgcaaaagg ctaacatcaa gcacaccaac gacggtcaca cccaatccca aaacaacaag 1680
   aacactcaag aaaacaaggc taagtcttaa 1710
<210> 42
   <211> 481
   <212> PRT
   <213> Artificial Sequence
<220>
   <223> ORF0657nI+
<400> 42
   Met Ala Glu Glu Thr Gly Gly Thr Asn Thr Glu Ala Gln Pro Lys Thr
   1 5 10 15
   Glu Ala Val Ala Ser Pro Thr Thr Thr Ser Glu Lys Ala Pro Glu Thr
   20 25 30
   Lys Pro Val Ala Asn Ala Val Ser Val Ser Asn Lys Glu Val Glu Ala
   35 40 45
   Pro Thr Ser Glu Thr Lys Glu Ala Lys Glu Val Lys Glu Val Lys Ala
   50 55 60
   Pro Lys Glu Thr Lys Glu Val Lys Pro Ala Ala Lys Ala Thr Asn Asn
   65 70 75 80
   Thr Tyr Pro Ile Leu Asn Gln Glu Leu Arg Glu Ala Ile Lys Asn Pro
   85 90 95
   Ala Ile Lys Asp Lys Asp His Ser Ala Pro Asn Ser Arg Pro Ile Asp
   100 105 110
   Phe Glu Met Lys Lys Lys Asp Gly Thr Gln Gln Phe Tyr His Tyr Ala
   115 120 125
   Ser Ser Val Lys Pro Ala Arg Val Ile Phe Thr Asp Ser Lys Pro Glu
   130 135 140
   Ile Glu Leu Gly Leu Gln Ser Gly Gln Phe Trp Arg Lys Phe Glu Val
   145 150 155 160
   Tyr Glu Gly Asp Lys Lys Leu Pro Ile Lys Leu Val Ser Tyr Asp Thr
   165 170 175
   Val Lys Asp Tyr Ala Tyr Ile Arg Phe Ser Val Ser Asn Gly Thr Lys
   180 185 190
   Ala Val Lys Ile Val Ser Ser Thr His Phe Asn Asn Lys Glu Glu Lys
   195 200 205
   Tyr Asp Tyr Thr Leu Met Glu Phe Ala Gln Pro Ile Tyr Asn Ser Ala
   210 215 220
   Asp Lys Phe Lys Thr Glu Glu Asp Tyr Lys Ala Glu Lys Leu Leu Ala
   225 230 235 240
   Pro Tyr Lys Lys Ala Lys Thr Leu Glu Arg Gln Val Tyr Glu Leu Asn
   245 250 255
   Lys Ile Gln Asp Lys Leu Pro Glu Lys Leu Lys Ala Glu Tyr Lys Lys
   260 265 270
   Lys Leu Glu Asp Thr Lys Lys Ala Leu Asp Glu Gln Val Lys Ser Ala
   275 280 285
   Ile Thr Glu Phe Gln Asn Val Gln Pro Thr Asn Glu Lys Met Thr Asp
   290 295 300
   Leu Gln Asp Thr Lys Tyr Val Val Tyr Glu Ser Val Glu Asn Asn Glu
   305 310 315 320
   Ser Met Met Asp Thr Phe Val Lys His Pro Ile Lys Thr Gly Met Leu
   325 330 335
   Asn Gly Lys Lys Tyr Met Val Met Glu Thr Thr Asn Asp Asp Tyr Trp
   340 345 350
   Lys Asp Phe Met Val Glu Gly Gln Arg Val Arg Thr Ile Ser Lys Asp
   355 360 365
   Ala Lys Asn Asn Thr Arg Thr Ile Ile Phe Pro Tyr Val Glu Gly Lys
   370 375 380
   Thr Leu Tyr Asp Ala Ile Val Lys Val His Val Lys Thr Ile Asp Tyr
   385 390 395 400
   Asp Gly Gln Tyr His Val Arg Ile Val Asp Lys Glu Ala Phe Thr Lys
   405 410 415
   Ala Asn Thr Asp Lys Ser Asn Lys Lys Glu Gln Gln Asp Asn Ser Ala
   420 425 430
   Lys Lys Glu Ala Thr Pro Ala Thr Pro Ser Lys Pro Thr Pro Ser Pro
   435 440 445
   Val Glu Lys Glu Ser Gln Lys Gln Asp Ser Gln Lys Asp Asp Asn Lys
       450                 455                 460
   Gln Leu Pro Ser Val Glu Lys Glu Asn Asp Ala Ser Ser Glu Ser Gly
   465                 470                 475                 480
   Lys
<210> 43
   <211> 1452
   <212> DNA
   <213> Artificial Sequence
<220>
   <223> Encodes SEQ ID NO: 42 and is codon optimized for yeast expression
<400> 43
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagtaaggat cc                                                     1452
<210> 44
   <211> 605
   <212> PRT
   <213> ORF0657nG
<400> 44
   Met Ala Glu Glu Thr Gly Gly Thr Asn Thr Glu Ala Gln Pro Lys Thr
   1               5                   10                  15
   Glu Ala Val Ala Ser Pro Thr Thr Thr Ser Glu Lys Ala Pro Glu Thr
               20                  25                  30
   Lys Pro Val Ala Asn Ala Val Ser Val Ser Asn Lys Glu Val Glu Ala
           35                  40                  45
   Pro Thr Ser Glu Thr Lys Glu Ala Lys Glu Val Lys Glu Val Lys Ala
   50 55 60
   Pro Lys Glu Thr Lys Glu Val Lys Pro Ala Ala Lys Ala Thr Asn Asn
   65 70 75 80
   Thr Tyr Pro Ile Leu Asn Gln Glu Leu Arg Glu Ala Ile Lys Asn Pro
   85 90 95
   Ala Ile Lys Asp Lys Asp His Ser Ala Pro Asn Ser Arg Pro Ile Asp
   100 105 110
   Phe Glu Met Lys Lys Lys Asp Gly Thr Gln Gln Phe Tyr His Tyr Ala
   115 120 125
   Ser Ser Val Lys Pro Ala Arg Val Ile Phe Thr Asp Ser Lys Pro Glu
   130 135 140
   Ile Glu Leu Gly Leu Gln Ser Gly Gln Phe Trp Arg Lys Phe Glu Val
   145 150 155 160
   Tyr Glu Gly Asp Lys Lys Leu Pro Ile Lys Leu Val Ser Tyr Asp Thr
   165 170 175
   Val Lys Asp Tyr Ala Tyr Ile Arg Phe Ser Val Ser Asn Gly Thr Lys
   180 185 190
   Ala Val Lys Ile Val Ser Ser Thr His Phe Asn Asn Lys Glu Glu Lys
   195 200 205
   Tyr Asp Tyr Thr Leu Met Glu Phe Ala Gln Pro Ile Tyr Asn Ser Ala
   210 215 220
   Asp Lys Phe Lys Thr Glu Glu Asp Tyr Lys Ala Glu Lys Leu Leu Ala
   225 230 235 240
   Pro Tyr Lys Lys Ala Lys Thr Leu Glu Arg Gln Val Tyr Glu Leu Asn
   245 250 255
   Lys Ile Gln Asp Lys Leu Pro Glu Lys Leu Lys Ala Glu Tyr Lys Lys
   260 265 270
   Lys Leu Glu Asp Thr Lys Lys Ala Leu Asp Glu Gln Val Lys Ser Ala
   275 280 285
   Ile Thr Glu Phe Gln Asn Val Gln Pro Thr Asn Glu Lys Met Thr Asp
   290 295 300
   Leu Gln Asp Thr Lys Tyr Val Val Tyr Glu Ser Val Glu Asn Asn Glu
   305 310 315 320
   Ser Met Met Asp Thr Phe Val Lys His Pro Ile Lys Thr Gly Met Leu
   325 330 335
   Asn Gly Lys Lys Tyr Met Val Met Glu Thr Thr Asn Asp Asp Tyr Trp
   340 345 350
   Lys Asp Phe Met Val Glu Gly Gln Arg Val Arg Thr Ile Ser Lys Asp
   355 360 365
   Ala Lys Asn Asn Thr Arg Thr Ile Ile Phe Pro Tyr Val Glu Gly Lys
   370 375 380
   Thr Leu Tyr Asp Ala Ile Val Lys Val His Val Lys Thr Ile Asp Tyr
   385 390 395 400
   Asp Gly Gln Tyr His Val Arg Ile Val Asp Lys Glu Ala Phe Thr Lys
   405 410 415
   Ala Asn Thr Asp Lys Ser Asn Lys Lys Glu Gln Gln Asp Asn Ser Ala
   420 425 430
   Lys Lys Glu Ala Thr Pro Ala Thr Pro Ser Lys Pro Thr Pro Ser Pro
   435 440 445
   Val Glu Lys Glu Ser Gln Lys Gln Asp Ser Gln Lys Asp Asp Asn Lys
   450 455 460
   Gln Leu Pro Ser Val Glu Lys Glu Asn Asp Ala Ser Ser Glu Ser Gly
   465 470 475 480
   Lys Asp Lys Thr Pro Ala Thr Lys Pro Thr Lys Gly Glu Val Glu Ser
                   485                 490                 495
   Ser Ser Thr Thr Pro Thr Lys Val Val Ser Thr Thr Gln Asn Val Ala
               500                 505                 510
   Lys Pro Thr Thr Ala Ser Ser Lys Thr Thr Lys Asp Val Val Gln Thr
           515                 520                 525
   Ser Ala Gly Ser Ser Glu Ala Lys Asp Ser Ala Pro Leu Gln Lys Ala
       530                 535                 540
   Asn Ile Lys Asn Thr Asn Asp Gly His Thr Gln Ser Gln Asn Asn Lys
   545                 550                 555                 560
   Asn Thr Gln Glu Asn Lys Ala Lys Ser Leu Pro Gln Thr Gly Glu Glu
                   565                 570                 575
   Ser Asn Lys Asp Met Thr Leu Pro Leu Met Ala Leu Leu Ala Leu Ser
               580                 585                 590
   Ser Ile Val Ala Phe Val Leu Pro Arg Lys Arg Lys Asn
           595                 600                 605
<210> 45
   <211> 1818
   <212> DNA
   <213> Artificial Sequence
<220>
   <223> Encodes SEQ ID NO: 44 containing an amino terminus methionine and is codon optimized for yeast expression
<400> 45
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggaa gtcaagccag ctgctaaggc taccaacaac 240
   acttacccaa ttttgaacca agaattgaga gaagctatta agaacccagc tatcaaggac 300
   aaggaccact ccgctccaaa ctctagacca atcgacttcg aaatgaagaa gaaggacggt 360
   acccaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aaggacaaga ctccagctac caagccaact aagggtgaag ttgaatcttc ctctactact 1500
   ccaaccaagg ttgtctccac tacccaaaac gtcgctaagc caactaccgc ttcttccaag 1560
   actaccaagg acgttgtcca aacttctgct ggttcctctg aagctaagga ctctgctcca 1620
   ttgcaaaagg ctaacatcaa gaacaccaac gacggtcaca cccaatccca aaacaacaag 1680
   aacactcaag aaaacaaggc taagtctttg ccacaaaccg gtgaagaatc caacaaggac 1740
   atgaccttgc cattgatggc tttgttggct ttgtcttcca tcgttgcttt cgtcttgcca 1800
   agaaagagaa agaactaa                                               1818
<210> 46
   <211> 1710
   <212> DNA
   <213> Artificial Sequence
<220>
   <223> Encodes SEQ ID NO: 17 containing an amino terminus methionine and is codon optimized for yeast expression
<400> 46
   atggctgaag aaactggtgg taccaacact gaagctcaac caaagaccga agctttggct 60
   tccccaacca ctaccactga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggct gtcaagccag ctgctaaggc tgacaacaac 240
   acttacccaa ttttgaacca agaattgaga gaagctatta agaacccagc tatcaaggac 300
   aaggaccact ccgctccaaa ctctagacca atcgacttcg aaatgaagaa ggaaaacggt 360
   gaacaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aaggacaaga ctccagctac caagccagct aagggtgaag ttgaatcttc ctctactact 1500
   ccaaccaagg ttgtctccac tacccaaaac gtcgctaagc caactaccgc ttcttccaag 1560
   actaccaagg acgttgtcca aacttctgct ggttcctctg aagctaagga ctctgctcca 1620
   ttgcaaaagg ctaacatcaa gaacaccaac gacggtcaca cccaatccca aaacaacaag 1680
   aacactcaag aaaacaaggc taagtcttaa                                  1710
<210> 47
   <211> 1446
   <212> DNA
   <213> Artificial Sequence
<220>
   <223> Encodes the SEQ ID NO: 17 I+ region, is codon optimized for yeast expression, and encodes a methionine initiation codon
<400> 47
   atggctgaag aaactggtgg taccaacact gaagctcaac caaagaccga agctttggct 60
   tccccaacca ctaccactga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggct gtcaagccag ctgctaaggc tgacaacaac 240
   acttacccaa ttttgaacca agaattgaga gaagctatta agaacccagc tatcaaggac 300
   aaggaccact ccgctccaaa ctctagacca atcgacttcg aaatgaagaa ggaaaacggt 360
   gaacaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagtaa                                                            1446
<210> 48
   <211> 1341
   <212> DNA
   <213> Artificial Sequence
<220>
   <223> Encodes the SEQ ID NO: 17 I region, is codon optimized for yeast expression, and encodes a methionine initiation codon
<400> 48
   atggctgaag aaactggtgg taccaacact gaagctcaac caaagaccga agctttggct 60
   tccccaacca ctaccactga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggct gtcaagccag ctgctaaggc tgacaacaac 240
   acttacccaa ttttgaacca agaattgaga gaagctatta agaacccagc tatcaaggac 300
   aaggaccact ccgctccaaa ctctagacca atcgacttcg aaatgaagaa ggaaaacggt 360
   gaacaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccata a 1341
<210> 49
   <211> 1938
   <212> DNA
   <213> Artificial Sequence
<220>
   <223> Encodes for full length ORF0657n containing SEQ ID NO: 17 modified to contain a glycine afer the amino terminus methionine and is codon optimized for yeast expression
<400> 49
   atgggtaaca agcaacaaaa ggaattcaag tctttctact ccattagaaa gtcttccttg 60
   ggtgttgctt ctgtcgctat ctccaccttg ttgttgttga tgtctaacgg tgaagctcaa 120
   gctgctgaag aaactggtgg taccaacact gaagctcaac caaagaccga agctttggct 180
   tccccaacca ctaccactga aaaggctcca gaaactaagc cagttgctaa cgctgtctcc 240
   gtttctaaca aggaagtcga agctccaacc tccgaaacta aggaagctaa ggaagttaag 300
   gaagtcaagg ctccaaagga aactaaggct gtcaagccag ctgctaaggc tgacaacaac 360
   acttacccaa ttttgaacca agaattgaga gaagctatta agaacccagc tatcaaggac 420
   aaggaccact ccgctccaaa ctctagacca atcgacttcg aaatgaagaa ggaaaacggt 480
   gaacaacaat tctaccacta cgcgtcctct gtcaagccag ctagagttat tttcaccgac 540
   tctaagccag aaatcgaatt gggtttgcaa tccggtcaat tctggagaaa gttcgaagtc 600
   tacgaaggtg acaagaagtt gccaattaag ttggtttcct acgacaccgt caaggactac 660
   gcttacatca gattctccgt ttctaacggt actaaggctg tcaagattgt ctcttccacc 720
   cacttcaaca acaaggaaga aaagtacgac tacactttga tggaattcgc tcaaccaatt 780
   tacaactctg ctgacaagtt caagaccgaa gaagactaca aggctgaaaa gttgttggct 840
   ccatacaaga aggctaagac tttggaaaga caagtttacg aattgaacaa gatccaagac 900
   aagttgccag aaaagttgaa ggctgaatac aagaagaagt tggaagacac caagaaggct 960
   ttggacgaac aagtcaagtc cgctatcacc gaattccaaa acgttcaacc aactaacgaa 1020
   aagatgactg acttgcaaga cactaagtac gtcgtctacg aatccgtcga aaacaacgaa 1080
   tccatgatgg acaccttcgt taagcaccca attaagactg gtatgttgaa cggtaagaag 1140
   tacatggtca tggaaaccac taacgacgac tactggaagg acttcatggt tgaaggtcaa 1200
   agagtcagaa ccatctccaa ggacgctaag aacaacacta gaaccattat cttcccatac 1260
   gttgaaggta agactttgta cgacgctatc gtcaaggttc acgtcaagac tattgactac 1320
   gacggtcaat accacgttag aattgttgac aaggaagctt tcaccaaggc taacaccgac 1380
   aagtccaaca agaaggaaca acaagacaac tctgctaaga aggaagctac cccagctacc 1440
   ccatctaagc caaccccatc tccagttgaa aaggaatctc aaaagcaaga ctcccaaaag 1500
   gacgacaaca agcaattgcc atccgtcgaa aaggaaaacg acgcgtcttc tgaatccggt 1560
   aaggacaaga ctccagctac caagccagct aagggtgaag ttgaatcttc ctctactact 1620
   ccaaccaagg ttgtctccac tacccaaaac gtcgctaagc caactaccgc ttcttccaag 1680
   actaccaagg acgttgtcca aacttctgct ggttcctctg aagctaagga ctctgctcca 1740
   ttgcaaaagg ctaacatcaa gaacaccaac gacggtcaca cccaatccca aaacaacaag 1800
   aacactcaag aaaacaaggc taagtctttg ccacaaaccg gtgaagaatc caacaaggac 1860
   atgaccttgc cattgatggc tttgttggct ttgtcttcca tcgttgcttt cgtcttgcca 1920
   agaaagagaa agaactaa 1938
<210> 50
   <211> 1710
   <212> DNA
   <213> Artificial Sequence
<220>
   <223> Encodes SEQ ID NO: 20, is codon optimized for yeast expression, and encodes a methionine initiation codon
<400> 50
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggct gtcaagccag ctactaaggc tgacaacaac 240
   acttacccaa ttttgaacca agaattgaga gaagctatta agaacccagc tatcaaggac 300
   aaggaccact ccgctccaaa ctctagacca atcgacttcg aaatgaagaa ggaaaacggt 360
   gaacaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aaggacaaga ctccagctac caagccagct aagggtgaag ttgaatcttc ctctactact 1500
   ccaaccaagg ttgtctccac tacccaaaac gtcgctaagc caactaccgc ttcttccaag 1560
   actaccaagg acgttgtcca aacttctgct ggttcctctg aagctaagga ctctgctcca 1620
   ttgcaaaagg ctaacatcaa gaacaccaac gacggtcaca cccaatccca aaacaacaag 1680
   aacactcaag aaaacaaggc taagtcttaa                                 1710
<210> 51
   <211> 1446
   <212> DNA
   <213> Artificial Sequence
<220>
   <223> Encodes SEQ ID NO: 20 I+ region, is codon optimized for yeast expression, and encodes a methionine initiation codon
<400> 51
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggct gtcaagccag ctactaaggc tgacaacaac 240
   acttacccaa ttttgaacca agaattgaga gaagctatta agaacccagc tatcaaggac 300
   aaggaccact ccgctccaaa ctctagacca atcgacttcg aaatgaagaa ggaaaacggt 360
   gaacaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccatc tccagttgaa aaggaatctc aaaagcaaga ctcccaaaag 1380
   gacgacaaca agcaattgcc atccgtcgaa aaggaaaacg acgcgtcttc tgaatccggt 1440
   aagtaa                                                            1446
<210> 52
   <211> 1341
   <212> DNA
   <213> Artificial Sequence
<220>
   <223> Encodes the SEQ ID NO: 20 I region, is codon optimized for yeast expression, and encoders a methionine initiation codon
<400> 52
   atggctgaag aaactggtgg taccaacact gaagctcaac caaagaccga agctgtcgct 60
   tccccaacca ctacctctga aaaggctcca gaaactaagc cagttgctaa cgctgtctcc 120
   gtttctaaca aggaagtcga agctccaacc tccgaaacta aggaagctaa ggaagttaag 180
   gaagtcaagg ctccaaagga aactaaggct gtcaagccag ctactaaggc tgacaacaac 240
   acttacccaa ttttgaacca agaattgaga gaagctatta agaacccagc tatcaaggac 300
   aaggaccact ccgctccaaa ctctagacca atcgacttcg aaatgaagaa ggaaaacggt 360
   gaacaacaat tctaccacta cgcgtcctct gtcaagccag ctagagttat tttcaccgac 420
   tctaagccag aaatcgaatt gggtttgcaa tccggtcaat tctggagaaa gttcgaagtc 480
   tacgaaggtg acaagaagtt gccaattaag ttggtttcct acgacaccgt caaggactac 540
   gcttacatca gattctccgt ttctaacggt actaaggctg tcaagattgt ctcttccacc 600
   cacttcaaca acaaggaaga aaagtacgac tacactttga tggaattcgc tcaaccaatt 660
   tacaactctg ctgacaagtt caagaccgaa gaagactaca aggctgaaaa gttgttggct 720
   ccatacaaga aggctaagac tttggaaaga caagtttacg aattgaacaa gatccaagac 780
   aagttgccag aaaagttgaa ggctgaatac aagaagaagt tggaagacac caagaaggct 840
   ttggacgaac aagtcaagtc cgctatcacc gaattccaaa acgttcaacc aactaacgaa 900
   aagatgactg acttgcaaga cactaagtac gtcgtctacg aatccgtcga aaacaacgaa 960
   tccatgatgg acaccttcgt taagcaccca attaagactg gtatgttgaa cggtaagaag 1020
   tacatggtca tggaaaccac taacgacgac tactggaagg acttcatggt tgaaggtcaa 1080
   agagtcagaa ccatctccaa ggacgctaag aacaacacta gaaccattat cttcccatac 1140
   gttgaaggta agactttgta cgacgctatc gtcaaggttc acgtcaagac tattgactac 1200
   gacggtcaat accacgttag aattgttgac aaggaagctt tcaccaaggc taacaccgac 1260
   aagtccaaca agaaggaaca acaagacaac tctgctaaga aggaagctac cccagctacc 1320
   ccatctaagc caaccccata a 1341
<210> 53
   <211> 1938
   <212> DNA
   <213> Artificial Sequence
<220>
   <223> Encodes for full length ORF0657n containing SEQ ID NO: 20 modified to contain a glycine after the amino terminus methionine and is codon optimized for yeast expression
<400> 53
   atgggtaaca agcaacaaaa ggaattcaag tctttctact ccattagaaa gtcttccttg 60
   ggtgttgctt ctgtcgctat ctccaccttg ttgttgttga tgtctaacgg tgaagctcaa 120
   gctgctgaag aaactggtgg taccaacact gaagctcaac caaagaccga agctgtcgct 180
   tccccaacca ctacctctga aaaggctcca gaaactaagc cagttgctaa cgctgtctcc 240
   gtttctaaca aggaagtcga agctccaacc tccgaaacta aggaagctaa ggaagttaag 300
   gaagtcaagg ctccaaagga aactaaggct gtcaagccag ctactaaggc tgacaacaac 360
   acttacccaa ttttgaacca agaattgaga gaagctatta agaacccagc tatcaaggac 420
   aaggaccact ccgctccaaa ctctagacca atcgacttcg aaatgaagaa ggaaaacggt 480
   gaacaacaat tctaccacta cgcgtcctct gtcaagccag ctagagttat tttcaccgac 540
   tctaagccag aaatcgaatt gggtttgcaa tccggtcaat tctggagaaa gttcgaagtc 600
   tacgaaggtg acaagaagtt gccaattaag ttggtttcct acgacaccgt caaggactac 660
   gcttacatca gattctccgt ttctaacggt actaaggctg tcaagattgt ctcttccacc 720
   cacttcaaca acaaggaaga aaagtacgac tacactttga tggaattcgc tcaaccaatt 780
   tacaactctg ctgacaagtt caagaccgaa gaagactaca aggctgaaaa gttgttggct 840
   ccatacaaga aggctaagac tttggaaaga caagtttacg aattgaacaa gatccaagac 900
   aagttgccag aaaagttgaa ggctgaatac aagaagaagt tggaagacac caagaaggct 960
   ttggacgaac aagtcaagtc cgctatcacc gaattccaaa acgttcaacc aactaacgaa 1020
   aagatgactg acttgcaaga cactaagtac gtcgtctacg aatccgtcga aaacaacgaa 1080
   tccatgatgg acaccttcgt taagcaccca attaagactg gtatgttgaa cggtaagaag 1140
   tacatggtca tggaaaccac taacgacgac tactggaagg acttcatggt tgaaggtcaa 1200
   agagtcagaa ccatctccaa ggacgctaag aacaacacta gaaccattat cttcccatac 1260
   gttgaaggta agactttgta cgacgctatc gtcaaggttc acgtcaagac tattgactac 1320
   gacggtcaat accacgttag aattgttgac aaggaagctt tcaccaaggc taacaccgac 1380
   aagtccaaca agaaggaaca acaagacaac tctgctaaga aggaagctac cccagctacc 1440
   ccatctaagc caaccccatc tccagttgaa aaggaatctc aaaagcaaga ctcccaaaag 1500
   gacgacaaca agcaattgcc atccgtcgaa aaggaaaacg acgcgtcttc tgaatccggt 1560
   aaggacaaga ctccagctac caagccagct aagggtgaag ttgaatcttc ctctactact 1620
   ccaaccaagg ttgtctccac tacccaaaac gtcgctaagc caactaccgc ttcttccaag 1680
   actaccaagg acgttgtcca aacttctgct ggttcctctg aagctaagga ctctgctcca 1740
   ttgcaaaagg ctaacatcaa gaacaccaac gacggtcaca cccaatccca aaacaacaag 1800
   aacactcaag aaaacaaggc taagtctttg ccacaaaccg gtgaagaatc caacaaggac 1860
   atgaccttgc cattgatggc tttgttggct ttgtcttcca tcgttgcttt cgtcttgcca 1920
   agaaagagaa agaactaa 1938
<210> 54
   <211> 565
   <212> PRT
   <213> Artificial Sequence
<220>
   <223> ORF0657nH
<400> 54
   Ala Glu Glu Thr Gly Gly Thr Asn Thr Glu Ala Gln Pro Lys Thr Glu
    1               5                  10                  15
   Ala Val Ala Ser Pro Thr Thr Thr Thr Glu Lys Ala Pro Glu Ala Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Ala Pro Lys Glu Thr
       50                  55                  60
   Lys Ala Val Lys Pro Ala Ala Lys Ala Asp Asn Asn Thr Tyr Pro Ile
   65                  70                  75                  80
   Leu Asn Gln Glu Leu Arg Glu Ala Ile Lys Asn Pro Ala Ile Lys Asp
                   85                  90                  95
   Lys Asp His Ser Ala Pro Asn Ser Arg Pro Ile Asp Phe Glu Met Lys
               100                 105                 110
   Lys Glu Asn Gly Glu Gln Gln Phe Tyr His Tyr Ala Ser Ser Val Lys
           115                 120                 125
   Pro Ala Arg Val Ile Phe Thr Asp Ser Lys Pro Glu Ile Glu Leu Gly
       130                 135                 140
   Leu Gln Ser Gly Gln Phe Trp Arg Lys Phe Glu Val Tyr Glu Gly Asp
   145                 150                 155                 160
   Lys Lys Leu Pro Ile Lys Leu Val Ser Tyr Asp Thr Val Lys Asp Tyr
                   165                 170                 175
   Ala Tyr Ile Arg Phe Ser Val Ser Asn Gly Thr Lys Ala Val Lys Ile
               180                 185                 190
   Val Ser Ser Thr His Phe Asn Asn Lys Glu Glu Lys Tyr Asp Tyr Thr
           195                 200                 205
   Leu Met Glu Phe Ala Gln Pro Ile Tyr Asn Ser Ala Asp Lys Phe Lys
       210                 215                 220
   Thr Glu Glu Asp Tyr Lys Ala Glu Lys Leu Leu Ala Pro Tyr Lys Lys
   225                 230                 235                 240
   Ala Lys Thr Leu Glu Arg Gln Val Tyr Glu Leu Asn Lys Ile Gln Asp
                   245                 250                 255
   Lys Leu Pro Glu Lys Leu Lys Ala Glu Tyr Lys Lys Lys Leu Glu Asp
               260                 265                 270
   Thr Lys Lys Ala Leu Asp Glu Gln Val Lys Ser Ala Ile Thr Glu Phe
           275                 280                 285
   Gln Asn Val Gln Pro Thr Asn Glu Lys Met Thr Asp Leu Gln Asp Thr
       290                 295                 300
   Lys Tyr Ala Val Tyr Glu Ser Val Glu Asn Asn Glu Ser Met Met Asp
   305                 310                 315                 320
   Thr Phe Val Lys His Pro Ile Lys Thr Gly Met Leu Asn Gly Lys Lys
                   325                 330                 335
   Tyr Met Val Met Glu Thr Thr Asn Asp Asp Tyr Trp Lys Asp Phe Met
               340                 345                 350
   Val Glu Gly Gln Arg Val Arg Thr Ile Ser Lys Asp Ala Lys Asn Asn
           355                 360                 365
   Thr Arg Thr Ile Ile Phe Pro Tyr Val Glu Gly Lys Thr Leu Tyr Asp
       370                 375                 380
   Ala Ile Val Lys Val His Val Lys Thr Ile Asp Tyr Asp Gly Gln Tyr
   385                 390                 395                 400
   His Val Arg Ile Val Asp Lys Glu Ala Phe Thr Lys Ala Asn Thr Asp
                   405                 410                 415
   Lys Ser Asn Lys Lys Glu Gln Gln Asp Asn Ser Ala Lys Lys Glu Ala
   420 425 430
   Thr Pro Ala Thr Pro Ser Lys Pro Thr Pro Ser Pro Val Glu Lys Glu
   435 440 445
   Ser Gln Lys Gln Asp Ser Gln Lys Asp Asp Asn Lys Gln Leu Pro Ser
   450 455 460
   Val Glu Lys Glu Asn Asp Ala Ser Ser Glu Ser Gly Lys Asp Lys Thr
   465 470 475 480
   Pro Ala Thr Lys Pro Ala Lys Gly Glu Val Glu Ser Ser Ser Thr Thr
   485 490 495
   Pro Thr Lys Val Val Ser Thr Thr Gln Asn Val Ala Lys Pro Thr Thr
   500 505 510
   Ala Ser Ser Lys Thr Thr Lys Asp Val Val Gln Thr Ser Ala Ser Ser
   515 520 525
   Ser Glu Ala Lys Asp Ser Ala Pro Leu Gln Lys Ala Asn Ile Lys Asn
   530 535 540
   Thr Asn Asp Gly His Thr Gln Ser Gln Asn Asn Lys Asn Thr Gln Glu
   545 550 555 560
   Asn Lys Ala Lys Ser
   565
<210> 55
   <211> 568
   <212> PRT
   <213> Artificial Sequence
<220>
   <223> ORF0657nH
<400> 55
   Ala Glu Glu Thr Gly Gly Thr Asn Thr Glu Ala Gln Pro Lys Thr Glu
   1 5 10 15
   Ala Val Ala Ser Pro Thr Thr Thr Ser Glu Lys Ala Pro Glu Thr Lys
   20 25 30
   Pro Val Ala Asn Ala Val Ser Val Ser Asn Lys Glu Val Glu Ala Pro
   35 40 45
   Thr Ser Glu Thr Lys Glu Ala Lys Glu Val Lys Glu Val Lys Ala Pro
   50 55 60
   Lys Glu Thr Lys Glu Val Lys Pro Ala Ala Lys Ala Thr Asn Asn Thr
   65 70 75 80
   Tyr Pro Ile Leu Asn Gln Gly Leu Arg Glu Ala Ile Lys Asn Pro Ala
   85 90 95
   Ile Lys Asp Lys Asp His Ser Ala Pro Asn Ser Arg Pro Ile Asp Phe
   100 105 110
   Glu Met Lys Lys Lys Asp Gly Thr Gln Gln Phe Tyr His Tyr Ala Ser
   115 120 125
   Ser Val Lys Pro Ala Arg Val Ile Phe Thr Asp Ser Lys Pro Glu Ile
   130 135 140
   Glu Leu Gly Leu Gln Ser Gly Gln Phe Trp Arg Lys Phe Glu Val Tyr
   145 150 155 160
   Glu Gly Asp Lys Lys Leu Pro Ile Lys Leu Val Ser Tyr Asp Thr Val
   165 170 175
   Lys Asp Tyr Ala Tyr Ile Arg Phe Ser Val Ser Asn Gly Thr Lys Ala
   180 185 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Pro Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Thr Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Ile Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 56
   <2.11> 568
   <212> PRT
   <213> Artificial Sequence
<220>
   <223> ORF0657nH
<400> 56
<210> 57
   <211> 568
   <212> PRT
   <213> Artificial Sequence
<220>
   <223> ORF0657nH
   <221> SITE
   <222> 247
   <223> Unknown
<400> 57
   Ala Glu Glu Thr Gly Gly Thr Asn Thr Glu Ala Gln Pro Lys Thr Glu
    1               5                  10                  15
   Ala Val Ala Ser Pro Thr Thr Thr Ser Glu Lys Ala Pro Glu Thr Lys
               20                  25                  30
   Pro Val Ala Asn Ala Val Pro Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Glu Val Lys Pro Ala Ala Lys Ala Thr Asn Asn Thr
   65                  70                  75                  80
   Tyr Pro Ile Leu Asn Gln Glu Leu Arg Glu Ala Thr Lys Asn Pro Glu
                   85                  90                  95
   Ile Lys Asp Lys Asp His Ser Ala Pro Asn Ser Arg Pro Thr Asp Phe
               100                 105                 110
   Glu Met Lys Lys Asn Asp Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Ile Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Xaa Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Ala Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Leu Leu Lys Thr His Asp Gly His Thr Gln Ser Gln Asn Ile Lys Asn
   545                 550                 555                 560
   Thr Lys Lys Asp Lys Ala Lys Ser
                   565
<210> 58
   <211> 568
   <212> PRT
   <213> Artificial Sequence
<220>
   <223> ORF0657nH
<400> 58
   Ala Glu Glu Thr Gly Gly Thr Asn Thr Glu Ala Gln Pro Lys Thr Glu
   1               5                   10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Glu Val Lys Pro Ala Ala Lys Ala Thr Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Lys Lys Glu Asn Gly Thr Gln Gln Phe Tyr His Tyr Ala Ser
           115                 120                 125
   Ser Val Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Tyr
           195                 200                 205
   Asp Tyr Thr Leu Met Glu Phe Ala Gln Pro Ile Tyr Asn Ser Ala Asp
       210                 215                 220
   Lys Phe Lys Thr Glu Glu Asp Tyr Lys Ala Glu Lys Leu Leu Ala Pro
   225                 230                 235                 240
   Tyr Lys Lys Ala Lys Thr Leu Glu Arg Gln Val Tyr Glu Leu Asn Lys
                   245                 250                 255
   Ile Gln Asp Lys Leu Pro Glu Lys Leu Lys Ala Glu Tyr Lys Lys Lys
               260                 265                 270
   Leu Glu Asp Thr Lys Lys Ala Leu Asp Glu Gln Val Lys Ser Ala Ile
           275                 280                 285
   Thr Glu Phe Gln Asn Val Gln Pro Thr Asn Glu Lys Met Thr Asp Leu
       290                 295                 300
   Gln Asp Thr Lys Tyr Val Val Tyr Glu Ser Val Glu Asn Asn Glu Ser
   305                 310                 315                 320
   Met Met Asp Thr Phe Val Lys His Pro Ile Lys Thr Gly Met Leu Asn
                   325                 330                 335
   Gly Lys Lys Tyr Met Val Met Glu Thr Thr Asn Asp Asp Tyr Trp Lys
               340                 345                 350
   Asp Phe Met Val Glu Gly Gln Arg Val Arg Thr Ile Ser Lys Asp Ala
           355                 360                 365
   Lys Asn Asn Thr Arg Thr Ile Ile Phe Pro Tyr Val Glu Gly Lys Thr
       370                 375                 380
   Leu Tyr Asp Ala Ile Val Lys Val His Val Lys Thr Ile Asp Tyr Asp
   385                 390                 395                 400
   Gly Gln Tyr His Val Arg Ile Val Asp Lys Glu Ala Phe Thr Lys Ala
                   405                 410                 415
   Asn Thr Asp Lys Ser Asn Lys Lys Glu Gln Gln Asp Asn Ser Ala Lys
               420                 425                 430
   Lys Glu Ala Thr Pro Ala Thr Pro Ser Lys Pro Thr Pro Ser Pro Val
           435                 440                 445
   Glu Lys Glu Ser Gln Lys Gln Asp Ser Gln Lys Asp Asp Asn Lys Gln
       450                 455                 460
   Leu Pro Ser Val Glu Lys Glu Asn Asp Ala Ser Ser Glu Ser Gly Lys
   465                 470                 475                 480
   Asp Lys Thr Pro Ala Thr Lys Pro Thr Lys Gly Glu Val Glu Ser Ser
                   485                 490                 495
   Ser Thr Thr Pro Thr Lys Val Val Ser Thr Thr Gln Asn Val Ala Lys
               500                 505                 510
   Pro Thr Thr Ala Ser Ser Lys Thr Thr Lys Asp Val Val Gln Thr Ser
           515                 520                 525
   Ala Gly Ser Ser Glu Ala Lys Asp Ser Ala Pro Leu Gln Lys Ala Asn
       530                 535                 540
   Ile Lys Asn Thr Asn Asp Gly His Thr Gln Ser Gln Asn Asn Lys Asn
   545                 550                 555                 560
   Thr Gln Glu Asn Lys Ala Lys Ser
                   565
<210> 59
   <211> 567
   <212> PRT
   <213> Artificial Sequence
<220>
   <223> ORF0657nH
<400> 59
   Ala Glu Glu Thr Gly Gly Thr Asn Thr Glu Ala Gln Pro Lys Thr Glu
   1               5                   10                  15
   Ala Val Ala Ser Pro Thr Thr Thr Ser Glu Lys Ala Pro Glu Thr Lys
               20                  25                  30
   Pro Val Ala Asn Ala Val Ser Val Ser Asn Lys Glu Val Glu Ala Pro
           35                  40                  45
   Thr Ser Glu Thr Lys Glu Ala Lys Glu Val Lys Glu Val Lys Ala Pro
       50                  55                  60
   Lys Glu Thr Lys Ala Val Lys Pro Ala Ala Lys Ala Thr Asn Asn Thr
   65                  70                  75                  80
   Tyr Pro Ile Leu Asn Gln Glu Leu Arg Glu Ala Ile Lys Asn Pro Ala
                   85                  90                  95
   Ile Lys Asp Lys Asp His Ser Ala Pro Asn Ser Arg Pro Ile Asp Phe
               100                 105                 110
   Glu Met Asn Lys Lys Asn Gly Glu Gln Gln Phe Tyr His Tyr Ala Ser
           115                 120                 125
   Ser Ala Lys Pro Ala Arg Val Ile Phe Thr Asp Ser Lys Pro Glu Ile
       130                 135                 140
   Glu Leu Gly Leu Gln Ser Gly Gln Phe Trp Arg Lys Phe Glu Val Tyr
   145                 150                 155                 160
   Glu Gly Asp Lys Lys Leu Pro Ile Lys Leu Val Ser Tyr Asp Thr Val
                   165                 170                 175
   Lys Asp Tyr Ala Tyr Ile Arg Phe Ser Val Ser Asn Gly Thr Lys Ala
               180                 185                 190
   Val Lys Ile Val Ser Ser Thr His Phe Asn Asn Lys Glu Glu Lys Asp
           195                 200                 205
   Tyr Thr Leu Met Glu Phe Ala Gln Pro Ile Tyr Asn Ser Ala Asp Lys
       210                 215                 220
   Phe Lys Thr Glu Glu Asp Tyr Lys Ala Glu Lys Leu Leu Ala Pro Tyr
   225                 230                 235                 240
   Lys Lys Ala Lys Thr Leu Glu Arg Gln Val Tyr Glu Leu Asn Lys Ile
                   245                 250                 255
   Gln Asp Lys Leu Pro Glu Lys Leu Lys Ala Glu Tyr Lys Lys Lys Leu
               260                 265                 270
   Glu Asp Thr Lys Lys Ala Leu Asp Glu Gln Val Lys Ser Ala Ile Thr
           275                 280                 285
   Glu Phe Gln Asn Val Gln Pro Thr Asn Glu Lys Met Thr Asp Leu Gln
       290                 295                 300
   Asp Thr Lys Tyr Val Val Tyr Glu Ser Val Glu Asn Asn Glu Ser Met
   305                 310                 315                 320
   Met Asp Thr Phe Val Lys His Pro Ile Lys Thr Gly Met Leu Asn Gly
                   325                 330                 335
   Lys Lys Tyr Met Val Met Glu Thr Thr Asn Asp Asp Tyr Trp Lys Asp
               340                 345                 350
   Phe Met Val Glu Gly Gln Arg Val Arg Thr Ile Ser Lys Asp Ala Lys
           355                 360                 365
   Asn Asn Thr Arg Thr Ile Ile Phe Pro Tyr Val Glu Gly Lys Thr Leu
       370                 375                 380
   Tyr Asp Ala Ile Val Lys Val His Val Lys Thr Ile Asp Tyr Asp Gly
   385                 390                 395                 400
   Gln Tyr His Val Arg Ile Val Asp Lys Glu Ala Phe Thr Lys Ala Asn
                   405                 410                 415
   Thr Asp Lys Ser Asn Lys Lys Glu Gln Gln Asp Asn Ser Ala Lys Lys
               420                 425                 430
   Glu Ala Thr Pro Ala Thr Pro Ser Lys Pro Thr Pro Ser Pro Val Glu
           435                 440                 445
   Lys Glu Ser Gln Lys Gln Asp Ser Gln Lys Asp Asp Asn Lys Gln Leu
       450                 455                 460
   Pro Ser Val Glu Lys Glu Asn Asp Ala Ser Ser Glu Ser Gly Lys Asp
   465                 470                 475                 480
   Lys Thr Pro Ala Thr Lys Pro Thr Lys Gly Glu Val Glu Ser Ser Ser
                   485                 490                 495
   Thr Thr Pro Thr Lys Val Val Ser Thr Thr Gln Asn Val Ala Lys Pro
               500                 505                 510
   Thr Thr Ala Ser Ser Lys Thr Thr Lys Asp Val Val Gln Thr Ser Ala
           515                 520                 525
   Gly Ser Ser Glu Ala Lys Asp Ser Ala Pro Leu Gln Lys Ala Asn Ile
       530                 535                 540
   Lys Asn Thr Asn Asp Gly His Thr Gln Ser Gln Asn Asn Lys Asn Thr
   545                 550                 555                 560
   Gln Glu Asn Lys Ala Lys Ser
                   565
<210> 60
   <211> 576
   <212> PRT
   <213> Artificial Sequence
<220>
   <223> ORF0657nH
<400> 60
   Ala Glu Glu Thr Gly Val Thr Asn Thr Glu Ala Gln Pro Lys Thr Glu
    1               5                  10                  15
   Ala Val Ala Ser Pro Thr Thr Thr Thr Thr Glu Lys Ala Pro Glu Ala
               20                  25                  30
   Lys Pro Val Ala Lys Pro Val Ala Asn Ala Val Ser Val Ser Asn Lys
           35                  40                  45
   Glu Val Val Ala Pro Thr Thr Glu Thr Lys Glu Ala Lys Glu Val Lys
       50                  55                  60
   Ala Val Lys Glu Val Lys Ala Pro Lys Glu Ala Lys Glu Glu Lys Pro
   65                  70                  75                  80
   Ala Ala Lys Ala Asp Asn Asn Thr Tyr Pro Ile Leu Asn Gln Glu Leu
                   85                  90                  95
   Arg Glu Ala Ile Lys Asn Pro Ala Ile Lys Asp Lys Asp His Ser Ala
               100                 105                 110
   Pro Asn Ser Arg Pro Ile Asp Phe Glu Met Lys Lys Lys Asp Gly Thr
           115                 120                 125
   Gln Gln Phe Tyr His Tyr Ala Ser Ser Val Lys Pro Ala Arg Val Ile
       130                 135                 140
   Phe Thr Asp Ser Lys Pro Glu Ile Glu Leu Gly Leu Gln Ser Gly Gln
   145                 150                 155                 160
   Phe Trp Arg Lys Phe Glu Val Tyr Glu Gly Asp Lys Lys Leu Pro Ile
                   165                 170                 175
   Lys Leu Val Ser Tyr Asp Thr Val Lys Asp Tyr Ala Tyr Ile Arg Phe
               180                 185                 190
   Ser Val Ser Asn Gly Thr Lys Ala Val Lys Ile Val Ser Ser Thr His
           195                 200                 205
   Phe Asn Asn Lys Glu Glu Lys Tyr Asp Tyr Thr Leu Met Glu Phe Ala
       210                 215                 220
   Gln Pro Ile Tyr Asn Ser Ala Asp Lys Phe Lys Thr Glu Glu Asp Tyr
   225                 230                 235                 240
   Lys Ala Glu Lys Leu Leu Ala Pro Tyr Lys Lys Ala Lys Thr Leu Glu
                   245                 250                 255
   Arg Gln Val Tyr Glu Leu Asn Lys Ile Gln Asp Lys Leu Pro Glu Lys
               260                 265                 270
   Leu Lys Ala Glu Tyr Lys Lys Lys Leu Glu Glu Thr Lys Lys Ala Leu
           275                 280                 285
   Asp Glu Gln Val Lys Ser Ala Ile Thr Glu Phe Gln Asn Val Gln Pro
       290                 295                 300
   Thr Asn Glu Lys Met Thr Asp Leu Gln Asp Thr Lys Tyr Val Ala Tyr
   305                 310                 315                 320
   Glu Ser Val Glu Asn Asn Glu Ser Met Met Asp Thr Phe Val Lys His
                   325                 330                 335
   Pro Ile Lys Thr Gly Met Leu Asn Gly Lys Lys Tyr Met Val Met Glu
               340                 345                 350
   Thr Thr Asn Asp Asp Tyr Trp Lys Asp Phe Met Val Glu Gly Gln Arg
           355                 360                 365
   Val Arg Thr Ile Ser Lys Asp Ala Lys Asn Asn Thr Arg Thr Ile Ile
       370                 375                 380
   Phe Pro Tyr Val Glu Gly Lys Thr Leu Tyr Asp Ala Ile Val Lys Val
   385 390 395 400
   His Val Lys Thr Ile Asp Tyr Asp Gly Gln Tyr His Val Arg Ile Val
   405 410 415
   Asp Lys Glu Ala Phe Thr Lys Ala Asn Ala Asp Lys Thr Asn Lys Lys
   420 425 430
   Glu Gln Gln Asp Asn Ser Ala Lys Lys Glu Thr Thr Pro Ala Met Pro
   435 440 445
   Ser Lys Pro Thr Thr Pro Pro Val Glu Lys Glu Ser Gln Lys Gln Asp
   450 455 460
   Ser Gln Lys Asp Asp Asn Lys Gln Ser Pro Ser Val Glu Lys Glu Asn
   465 470 475 480
   Asp Ala Ser Ser Glu Ser Gly Lys Asp Lys Met Pro Val Thr Lys Pro
   485 490 495
   Ala Lys Ala Glu Val Glu Ser Ser Ser Thr Thr Pro Thr Lys Val Val
   500 505 510
   Ser Thr Thr Gln Asn Val Ala Lys Pro Thr Thr Ala Ser Ser Glu Thr
   515 520 525
   Thr Lys Asp Val Val Gln Thr Ser Ala Gly Ser Ser Glu Ala Lys Asp
   530 535 540
   Ser Ala Pro Leu Gln Lys Ala Asn Ile Lys Asn Thr Asn Asp Gly His
   545 550 555 560
   Thr Gln Ser Gln Asn Asn Lys Asn Thr Gln Glu Asn Lys Ala Lys Ser
   565 570 575
<210> 61
   <211> 572
   <212> PRT
   <213> Artificial Sequence
<220>
   <223> ORF0657nH
<400> 61
   Ala Glu Glu Thr Gly Val Thr Asn Thr Glu Ala Gln Pro Lys Thr Glu
   1 5 10 15
   Ala Val Ala Ser Pro Thr Thr Thr Thr Thr Glu Lys Ala Pro Glu Ala
   20 25 30
   Lys Pro Val Ala Asn Ala Val Ser Val Ser Asn Lys Glu Val Val Ala
   35 40 45
   Pro Thr Thr Glu Thr Lys Glu Ala Lys Glu Val Lys Ala Val Lys Glu
   50 55 60
   Val Lys Ala Pro Lys Glu Ala Lys Glu Glu Lys Pro Ala Ala Lys Ala
   65 70 75 80
   Asp Asn Asn Thr Tyr Pro Ile Leu Asn Gln Glu Leu Arg Glu Ala Ile
   85 90 95
   Lys Asn Pro Ala Ile Lys Asp Lys Asp His Ser Ala Pro Asn Ser Arg
   100 105 110
   Pro Ile Asp Phe Glu Met Lys Lys Lys Asp Gly Thr Gln Gln Phe Tyr
   115 120 125
   His Tyr Ala Ser Ser Val Lys Pro Ala Arg Val Ile Phe Thr Asp Ser
   130 135 140
   Lys Pro Glu Ile Glu Leu Gly Leu Gln Ser Gly Gln Phe Trp Arg Lys
   145 150 155 160
   Phe Glu Val Tyr Glu Gly Asp Lys Lys Leu Pro Ile Lys Leu Val Ser
   165 170 175
   Tyr Asp Thr Val Lys Asp Tyr Ala Tyr Ile Arg Phe Ser Val Ser Asn
   180 185 190
   Gly Thr Lys Ala Val Lys Ile Val Ser Ser Thr His Phe Asn Asn Lys
   195 200 205
   Glu Glu Lys Tyr Asp Tyr Thr Leu Met Glu Phe Ala Gln Pro Ile Tyr
   210 215 220
   Asn Ser Ala Asp Lys Phe Lys Thr Glu Glu Asp Tyr Lys Ala Glu Lys
   225 230 235 240
   Leu Leu Ala Pro Tyr Lys Lys Ala Lys Thr Leu Glu Arg Gln Val Tyr
   245 250 255
   Glu Leu Asn Lys Ile Gln Asp Lys Leu Pro Glu Lys Leu Lys Ala Glu
   260 265 270
   Tyr Lys Lys Lys Leu Glu Glu Thr Lys Lys Ala Leu Asp Glu Gln Val
   275 280 285
   Lys Ser Ala Ile Thr Glu Phe Gln Asn Val Gln Pro Thr Asn Glu Lys
   290 295 300
   Met Thr Asp Leu Gln Asp Thr Lys Tyr Val Val Tyr Glu Ser Val Glu
   305 310 315 320
   Asn Asn Glu Ser Met Met Asp Thr Phe Val Lys His Pro Ile Lys Thr
   325 330 335
   Gly Met Leu Asn Gly Lys Lys Tyr Met Val Met Glu Thr Thr Asn Asp
   340 345 350
   Asp Tyr Trp Lys Asp Phe Met Val Glu Gly Gln Arg Val Arg Thr Ile
   355 360 365
   Ser Lys Asp Ala Lys Asn Asn Thr Arg Thr Ile Ile Phe Pro Cys Val
   370 375 380
   Glu Gly Lys Thr Leu Tyr Asp Ala Ile Val Lys Val His Val Lys Thr
   385 390 395 400
   Ile Asp Tyr Asp Gly Gln Tyr His Val Arg Ile Val Asp Lys Glu Ala
   405 410 415
   Phe Thr Lys Ala Asn Ala Asp Lys Thr Asn Lys Lys Glu Gln Gln Asp
   420 425 430
   Asn Ser Ala Lys Lys Glu Thr Thr Pro Ala Met Pro Ser Lys Pro Thr
   435 440 445
   Thr Pro Pro Val Glu Lys Glu Ser Gln Lys Gln Asp Ser Gln Lys Asp
   450 455 460
   Asp Asn Lys Gln Ser Pro Ser Val Glu Lys Glu Asn Asp Ala Ser Ser
   465 470 475 480
   Glu Ser Gly Lys Asp Lys Met Pro Val Thr Lys Pro Ala Lys Ala Glu
   485 490 495
   Val Glu Ser Ser Ser Thr Thr Pro Thr Lys Val Val Ser Thr Thr Gln
   500 505 510
   Asn Val Ala Lys Pro Thr Thr Ala Ser Ser Glu Thr Thr Lys Asp Val
   515 520 525
   Val Gln Thr Ser Ala Gly Ser Ser Glu Ala Lys Asp Ser Ala Pro Leu
   530 535 540
   Gln Lys Ala Asn Ile Lys Asn Thr Asn Asp Gly His Thr Gln Ser Gln
   545 550 555 560
   Asn Asn Lys Asn Thr Gln Glu Asn Lys Ala Lys Ser
   565 570
<210> 62
   <211> 572
   <212> PRT
   <213> Artificial Sequence
<220>
   <223> ORF0657nH
<400> 62
   Ala Glu Glu Thr Gly Val Thr Asn Thr Glu Ala Gln Pro Lys Thr Glu
   1               5                   10                  15
   Ala Val Ala Ser Pro Thr Thr Thr Thr Thr Glu Lys Ala Pro Glu Ala
               20                  25                  30
   Lys Pro Val Ala Asn Ala Val Ser Val Ser Asn Lys Glu Val Val Ala
           35                  40                  45
   Pro Thr Thr Glu Thr Lys Glu Ala Lys Glu Val Lys Ala Val Lys Glu
       50                  55                  60
   Val Lys Ala Pro Lys Glu Ala Lys Glu Glu Lys Pro Ala Ala Lys Ala
   65                  70                  75                  80
   Asp Asn Asn Thr Tyr Pro Ile Leu Asn Gln Glu Leu Arg Glu Ala Ile
                   85                  90                  95
   Lys Asn Pro Ala Ile Lys Asp Lys Asp His Ser Ala Pro Asn Ser Arg
               100                 105                 110
   Pro Ile Asp Phe Glu Met Lys Lys Lys Asp Gly Thr Gln Gln Phe Tyr
           115                 120                 125
   His Tyr Ala Ser Ser Val Lys Pro Ala Arg Val Ile Phe Thr Asp Ser
       130                 135                 140
   Lys Pro Glu Ile Glu Leu Gly Leu Gln Ser Gly Gln Phe Trp Arg Lys
   145                 150                 155                 160
   Phe Glu Val Tyr Glu Gly Asp Lys Lys Leu Pro Ile Lys Leu Val Ser
                   165                 170                 175
   Tyr Asp Thr Val Lys Asp Tyr Ala Tyr Ile Arg Phe Ser Val Ser Asn
               180                 185                 190
   Gly Thr Lys Ala Val Lys Ile Val Ser Ser Thr His Phe Asn Asn Lys
           195                 200                 205
   Glu Glu Lys Tyr Asp Tyr Thr Leu Met Glu Phe Ala Gln Pro Ile Tyr
       210                 215                 220
   Asn Ser Ala Asp Lys Phe Lys Thr Glu Glu Asp Tyr Lys Ala Glu Lys
   225                 230                 235                 240
   Leu Leu Ala Pro Tyr Lys Lys Ala Lys Thr Leu Glu Arg Gln Val Tyr
                   245                 250                 255
   Glu Leu Asn Lys Ile Gln Asp Lys Leu Pro Glu Lys Leu Lys Ala Glu
               260                 265                 270
   Tyr Lys Lys Lys Leu Glu Glu Thr Lys Lys Ala Leu Asp Glu Gln Val
           275                 280                 285
   Lys Ser Ala Ile Thr Glu Phe Gln Asn Val Gln Pro Thr Asn Glu Lys
       290                 295                 300
   Met Thr Asp Leu Gln Asp Thr Lys Tyr Val Val Tyr Glu Ser Val Glu
   305                 310                 315                 320
   Asn Asn Glu Ser Met Met Asp Thr Phe Val Lys His Pro Ile Lys Thr
                   325                 330                 335
   Gly Met Leu Asn Gly Lys Lys Tyr Met Val Met Glu Thr Thr Asn Asp
               340                 345                 350
   Asp Tyr Trp Lys Asp Phe Met Val Glu Gly Gln Arg Val Arg Thr Ile
           355                 360                 365
   Ser Lys Asp Ala Lys Asn Asn Thr Arg Thr Ile Ile Phe Pro Tyr Val
       370                 375                 380
   Glu Gly Lys Thr Leu Tyr Asp Ala Ile Val Lys Val His Val Lys Thr
   385 390 395 400
   Ile Asp Tyr Asp Gly Gln Tyr His Val Arg Ile Val Asp Lys Glu Ala
   405 410 415
   Phe Thr Lys Ala Asn Ala Asp Lys Thr Asn Lys Lys Glu Gln Gln Asp
   420 425 430
   Asn Ser Ala Lys Lys Glu Thr Thr Pro Ala Met Pro Ser Lys Pro Thr
   435 440 445
   Thr Pro Pro Val Glu Lys Glu Ser Gln Lys Gln Asp Ser Gln Lys Asp
   450 455 460
   Asp Asn Lys Gln Ser Pro Ser Val Glu Lys Glu Asn Asp Ala Ser Ser
   465 470 475 480
   Glu Ser Gly Lys Asp Lys Met Pro Val Thr Lys Pro Ala Lys Ala Glu
   485 490 495
   Val Glu Ser Ser Ser Thr Thr Pro Thr Lys Val Val Ser Thr Thr Gln
   500 505 510
   Asn Val Ala Lys Pro Thr Thr Ala Ser Ser Glu Thr Thr Lys Asp Val
   515 520 525
   Val Gln Thr Ser Ala Gly Ser Ser Glu Ala Lys Asp Ser Ala Pro Leu
   530 535 540
   Gln Lys Ala Asn Ile Lys Asn Thr Asn Asp Gly His Thr Gln Ser Gln
   545 550 555 560
   Asn Asn Lys Asn Thr Gln Glu Asn Lys Ala Lys Ser
   565 570
<210> 63
   <211> 566
   <212> PRT
   <213> Artificial Sequence
<220>
   <223> ORF0657nH
<400> 63
   Ala Glu Glu Thr Gly Gly Thr Asn Thr Glu Ala Gln Pro Lys Thr Glu
   1 5 10 15
   Ala Val Ala Ser Pro Thr Thr Thr Thr Thr Glu Lys Ala Pro Glu Ala
   20 25 30
   Lys Pro Val Ala Asn Ala Val Ser Val Ser Asn Lys Glu Val Val Ala
   35 40 45
   Pro Thr Thr Glu Thr Lys Glu Ala Lys Glu Val Lys Ala Pro Lys Glu
   50 55 60
   Thr Lys Glu Val Lys Pro Ala Ala Lys Ala Asp Asn Asn Thr Tyr Pro
   65 70 75 80
   Ile Leu Asn Lys Glu Leu Arg Glu Ala Ile Lys Asn Pro Ala Ile Lys
   85 90 95
   Asp Lys Asp His Ser Ala Pro Asn Ser Arg Pro Ile Asp Phe Glu Met
   100 105 110
   Lys Lys Glu Asn Gly Glu Gln Gln Phe Tyr His Tyr Ala Ser Ser Val
   115 120 125
   Lys Pro Ala Arg Val Ile Phe Thr Asp Ser Lys Pro Glu Ile Glu Leu
   130 135 140
   Gly Leu Gln Ser Gly Gln Phe Trp Arg Lys Phe Glu Val Tyr Glu Gly
   145 150 155 160
   Asp Lys Lys Leu Pro Ile Lys Leu Val Ser Tyr Asp Thr Val Lys Asp
   165 170 175
   Tyr Ala Tyr Ile Arg Phe Ser Val Ser Asn Gly Thr Lys Ala Val Lys
   180 185 190
   Ile Val Ser Ser Thr His Phe Asn Asn Lys Glu Glu Lys Tyr Asp Tyr
   195 200 205
   Thr Leu Met Glu Phe Ala Gln Pro Ile Tyr Asn Ser Ala Asp Lys Phe
   210 215 220
   Lys Thr Glu Glu Asp Tyr Lys Ala Glu Lys Leu Leu Ala Pro Tyr Lys
   225 230 235 240
   Lys Ala Lys Thr Leu Glu Arg Gln Val Tyr Glu Leu Asn Lys Ile Gln
   245 250 255
   Asp Lys Leu Pro Glu Lys Leu Lys Ala Glu Tyr Lys Lys Lys Leu Glu
   260 265 270
   Glu Thr Lys Lys Ala Leu Asp Glu Gln Val Lys Ser Ala Ile Thr Glu
   275 280 285
   Phe Gln Asn Val Gln Pro Thr Asn Glu Lys Met Thr Asp Leu Gln Asp
   290 295 300
   Thr Lys Tyr Val Val Tyr Glu Ser Val Glu Asn Asn Glu Ser Met Met
   305 310 315 320
   Asp Thr Phe Val Lys His Pro Ile Lys Thr Gly Met Leu Asn Gly Lys
   325 330 335
   Lys Tyr Met Val Met Glu Thr Thr Asn Asp Asp Tyr Trp Lys Asp Phe
   340 345 350
   Met Val Glu Gly Gln Arg Val Arg Thr Ile Ser Lys Asp Ala Lys Asn
   355 360 365
   Asn Thr Arg Thr Ile Ile Phe Pro Tyr Ile Glu Gly Lys Thr Leu Tyr
   370 375 380
   Asp Ala Ile Val Lys Val His Val Lys Thr Ile Asp Tyr Asp Gly Gln
   385 390 395 400
   Tyr His Val Arg Ile Val Asp Lys Glu Ala Phe Thr Lys Ala Asn Ala
   405 410 415
   Asp Lys Ser Asn Lys Lys Glu Gln Gln Asp Asn Ser Ala Lys Lys Glu
   420 425 430
   Thr Thr Pro Ala Thr Pro Ser Lys Pro Thr Thr Pro Pro Val Glu Lys
   435 440 445
   Glu Ser Gln Lys Gln Asp Ser Gln Lys Asp Asp Asn Thr Gln Ser Pro
   450 455 460
   Ser Val Glu Lys Glu Asn Asp Ala Ser Ser Glu Ser Gly Lys Asp Lys
   465 470 475 480
   Thr Pro Ala Thr Lys Pro Ala Lys Gly Glu Val Glu Ser Ser Ser Thr
   485 490 495
   Thr Pro Thr Lys Val Val Ser Thr Thr Gln Asn Val Ala Lys Pro Thr
   500 505 510
   Thr Ala Ser Ser Glu Thr Thr Lys Asp Val Val Gln Thr Ser Ala Gly
   515 520 525
   Pro Ser Glu Ala Lys Asp Ser Ala Pro Leu Gln Lys Ala Asn Ile Lys
   530 535 540
   Asn Thr Asn Asp Gly His Thr Gln Ser Gln Asn Asn Lys Asn Thr Gln
   545 550 555 560
   Glu Asp Lys Ala Lys Ser
   565
<210> 64
   <211> 8
   <212> PRT
   <213> Artificial Sequence
<220>
   <223> His-Tag
<400> 64
   Leu Glu His His His His His His
   1 5
<210> 65
   <211> 16
   <212> DNA
   <213> Artificial Sequence
<220>
   <223> Primer
<400> 65
   ctggccgtcg ttttac 16
<210> 66
   <211> 17
   <212> DNA
   <213> Artificial Sequence
<220>
   <223> Primer
<400> 66
   caggaaacag ctatgac 17
<210> 67
   <211> 39
   <212> DNA
   <213> Artificial Sequence
<220>
   <223> Primer
<400> 67
   aaccggtttt ccatggggaa caaacagcaa aaagaattt 39
<210> 68
   <211> 38
   <212> DNA
   <213> Artificial Sequence
<220>
   <223> Primer
<400> 68
   accggtttct cgaggttttt acgttttcta ggtaatac 38
<210> 69
   <211> 109
   <212> DNA
   <213> Artificial Sequence
<220>
   <223> ORF0657n oligomer
<400> 69
   atgggtaaca agcaacaaaa ggaattcaag tctttctact ccattagaaa gtcttccttg 60
   ggtgttgctt ctgtcgctat ctccaccttg ttgttgttga tgtctaacg            109
<210> 70
   <211> 110
   <212> DNA
   <213> Artificial Sequence
<220>
   <223> ORF0657n oligomer
<400> 70
   gttggggaag cgacagcttc ggtctttggt tgagcttcag tgttggtacc accagtttct 60
   tcagcagcag cttgagcttc accgttagac atcaacaaca acaaggtgga           110
<210> 71
   <211> 110
   <212> DNA
   <213> Artificial Sequence
<220>
   <223> ORF0657n oligomer
<400> 71
   agaccgaagc tgtcgcttcc ccaaccacta cctctgaaaa ggctccagaa actaagccag 60
   ttgctaacgc tgtctccgtt tctaacaagg aagtcgaagc tccaacctcc           110
<210> 72
   <211> 109
   <212> DNA
   <213> Artificial Sequence
<220>
   <223> ORF0657n oligomer
<400> 72
   tggtagcctt agcagctggc ttgacttcct tagtttcctt tggagccttg acttccttaa 60
   cttccttagc ttccttagtt tcggaggttg gagcttcgac ttccttgtt            109
<210> 73
   <211> 108
   <212> DNA
   <213> Artificial Sequence
<220>
   <223> ORF0657n oligomer
<400> 73
   aagtcaagcc agctgctaag gctaccaaca acacttaccc aattttgaac caagaattga 60
   gagaagctat taagaaccca gctatcaagg acaaggacca ctccgctc 108
<210> 74
   <211> 109
   <212> DNA
   <213> Artificial Sequence
<220>
   <223> ORF0657n oligomer
<400> 74
   tggcttgaca gaggacgcgt agtggtagaa ttgttgggta ccgtccttct tcttcatttc 60
   gaagtcgatt ggtctagagt ttggagcgga gtggtccttg tccttgata 109
<210> 75
   <211> 102
   <212> DNA
   <213> Artificial Sequence
<220>
   <223> ORF0657n oligomer
<400> 75
   accactacgc gtcctctgtc aagccagcta gagttatttt caccgactct aagccagaaa 60
   tcgaattggg tttgcaatcc ggtcaattct ggagaaagtt cg 102
<210> 76
   <211> 104
   <212> DNA
   <213> Artificial Sequence
<220>
   <223> ORF0657n oligomer
<400> 76
   ctgatgtaag cgtagtcctt gacggtgtcg taggaaacca acttaattgg caacttcttg 60
   tcaccttcgt agacttcgaa ctttctccag aattgaccgg attg 104
<210> 77
   <211> 109
   <212> DNA
   <213> Artificial Sequence
<220>
   <223> ORF0657n oligomer
<400> 77
   caccgtcaag gactacgctt acatcagatt ctccgtttct aacggtacta aggctgtcaa 60
   gattgtctct tccacccact tcaacaacaa ggaagaaaag tacgactac             109
<210> 78
   <211> 109
   <212> DNA
   <213> Artificial Sequence
<220>
   <223> ORF0657n oligomer
<400> 78
   aacttttcag ccttgtagtc ttcttcggtc ttgaacttgt cagcagagtt gtaaattggt 60
   tgagcgaatt ccatcaaagt gtagtcgtac ttttcttcct tgttgttga             109
<210> 79
   <211> 106
   <212> DNA
   <213> Artificial Sequence
<220>
   <223> ORF0657n oligomer
<400> 799
<210> 80
   <211> 109
   <212> DNA
   <213> Artificial Sequence
<220>
   <223> ORF0657n oligomer
<400> 80
   tcggtgatag cggacttgac ttgttcgtcc aaagccttct tggtgtcttc caacttcttc 60
   ttgtattcag ccttcaactt ttctggcaac ttgtcttgga tcttgttca             109
<210> 81
   <211> 109
   <212> DNA
   <213> Artificial Sequence
<220>
   <223> ORF0657n oligomer
<400> 81
   cgaacaagtc aagtccgcta tcaccgaatt ccaaaacgtt caaccaacta acgaaaagat 60
   gactgacttg caagacacta agtacgtcgt ctacgaatcc gtcgaaaac 109
<210> 82
   <211> 109
   <212> DNA
   <213> Artificial Sequence
<220>
   <223> ORF0657n oligomer
<400> 82
   tttccatgac catgtacttc ttaccgttca acataccagt cttaattggg tgcttaacga 60
   aggtgtccat catggattcg ttgttttcga cggattcgta gacgacgta 109
<210> 83
   <211> 109
   <212> DNA
   <213> Artificial Sequence
<220>
   <223> ORF0657n oligomer
<400> 83
   gaacggtaag aagtacatgg tcatggaaac cactaacgac gactactgga aggacttcat 60
   ggttgaaggt caaagagtca gaaccatctc caaggacgct aagaacaac 109
<210> 84
   <211> 101
   <212> DNA
   <213> Artificial Sequence
<220>
   <223> ORF0657n oligomer
<400> 84
   gtcttgacgt gaaccttgac gatagcgtcg tacaaagtct taccttcaac gtatgggaag 60
   ataatggttc tagtgttgtt cttagcgtcc ttggagatgg t 101
<210> 85
   <211> 106
   <212> DNA
   <213> Artificial Sequence
<220>
   <223> ORF0657n oligomer
<400> 85
   cgctatcgtc aaggttcacg tcaagactat tgactacgac ggtcaatacc acgttagaat 60
   tgttgacaag gaagctttca ccaaggctaa caccgacaag tccaac 106
<210> 86
   <211> 96
   <212> DNA
   <213> Artificial Sequence
<220>
   <223> ORF0657n oligomer
<400> 86
   tggggttggc ttagatgggg tagctggggt agcttccttc ttagcagagt tgtcttgttg 60
   ttccttcttg ttggacttgt cggtgttagc cttggt 96
<210> 87
   <211> 85
   <212> DNA
   <213> Artificial Sequence
<220>
   <223> ORF0657n oligomer
<400> 87
   cagctacccc atctaagcca accccatctc cagttgaaaa ggaatctcaa aagcaagact 60
   cccaaaagga cgacaacaag caatt 85
<210> 88
   <211> 100
   <212> DNA
   <213> Artificial Sequence
<220>
   <223> ORF0657n oligomer
<400> 88
   gttggcttgg tagctggagt cttgtcctta ccggattcag aagacgcgtc gttttccttt 60
   tcgacggatg gcaattgctt gttgtcgtcc ttttgggagt 100
<210> 89
   <211> 101
<212> DNA
   <213> Artificial Sequence
<220>
   <223> ORF0657n oligomer
<400> 89
   ggacaagact ccagctacca agccaactaa gggtgaagtt gaatcttcct ctactactcc 60
   aaccaaggtt gtctccacta cccaaaacgt cgctaagcca a 101
<210> 90
   <211> 101
   <212> DNA
   <213> Artificial Sequence
<220>
   <223> ORF0657n oligomer
<400> 90
   agcagagtcc ttagcttcag aggaaccagc agaagtttgg acaacgtcct tggtagtctt 60
   ggaagaagcg gtagttggct tagcgacgtt ttgggtagtg g 101
<210> 91
   <211> 91
   <212> DNA
   <213> Artificial Sequence
<220>
   <223> ORF0657n oligomer
<400> 91
   ggttcctctg aagctaagga ctctgctcca ttgcaaaagg ctaacatcaa gaacaccaac 60
   gacggtcaca cccaatccca aaacaacaag a 91
<210> 92
   <211> 98
   <212> DNA
   <213> Artificial Sequence
<220>
   <223> ORF0657n oligomer
<400> 92
   gtgaagaatc caacaaggac atgaccttgc cattgatggc tttgttggct ttgtcttcca 60
   tcgttgcttt cgtcttgcca agaaagagaa agaactaa 98
<210> 93
   <211> 98
   <212> DNA
   <213> Artificial Sequence
<220>
   <223> ORF0657n oligomer
<400> 93
   gtgaagaatc caacaaggac atgaccttgc cattgatggc tttgttggct ttgtcttcca 60
   tcgttgcttt cgtcttgcca agaaagagaa agaactaa 98
<210> 94
   <211> 31
   <212> DNA
   <213> Artificial Sequence
<220>
   <223> Primer
<400> 94
   cttaaagctt atgtcacttt ctcttgtatc g 31
<210> 95
   <211> 30
   <212> DNA
   <213> Artificial Sequence
<220>
   <223> Primer
<400> 95
   tgataagctt gctcaatggt tctcttcctc 30
<210> 96
   <211> 53
   <212> DNA
   <213> Artificial Sequence
<220>
   <223> Primer
<400> 96
   aaccggtttg gatcccacaa aacaaaatgg gtaacaagca acaaaaggaa ttc 53
<210> 97 .
   <211> 42
   <212> DNA
   <213> Artificial Sequence
<220>
   <223> Primer
<400> 97
   aaccggtttg gatccttagt tctttctctt tcttggcaag ac 42
<210> 98
   <211> 24
   <212> DNA
   <213> Artificial Sequence
<220>
   <223> Primer
<400> 98
   gctgaagaaa ctggtggtac caac 24
<210> 99
   <211> 42
   <212> DNA
   <213> Artificial Sequence
<220>
   <223> Primer
<400> 99
   gtcacggatc cttaagactt agccttgttt tcttgagtgt tc 42
<210> 100
   <211> 41
   <212> DNA
   <213> Artificial Sequence
<220>
   <223> Primer
<400> 100
   ggggggatcc cacaaaacaa aatggctgaa gaaactggtg g 41
<210> 101
   <211> 39
   <212> DNA
   <213> Artificial Sequence
<220>
   <223> Primer
<400> 101
   ggggggggat ccttaagact tagccttgtt ttcttgagt 39
<210> 102
   <211> 41
   <212> DNA
   <213> Artificial Sequence
<220>
   <223> Primer
<400> 102
   ggggggatcc cacaaaacaa aatggctgaa gaaactggtg g 41
<210> 103
   <211> 32
   <212> DNA
   <213> Artificial Sequence
<220>
   <223> Primer
<400> 103
   gggggggatc cttagttctt tctctttctt gg 32
<210> 104
   <211> 39
   <212> DNA
   <213> Artificial Sequence
<220>
   <223> Primer
<400> 104
   ctccggatcc cacaaaacaa aatggctgaa gaaactggt 39
<210> 105
   <211> 38
   <212> DNA
   <213> Artificial Sequence
<220>
   <223> Primer
<400> 105
   gctgccggga tccttatggg gttggcttag atggggta 38
<210> 106
   <211> 644
   <212> PRT
   <213> S. aureus
<400> 106
   Met Asn Lys Gln Gln Lys Glu Phe Lys Ser Phe Tyr Ser Ile Arg Lys
    1               5                  10                  15
   Ser Ser Leu Gly Val Ala Ser Val Ala Ile Ser Thr Leu Leu Leu Leu
               20                  25                  30
   Met Ser Asn Gly Glu Ala Gln Ala Ala Glu Glu Thr Gly Gly Thr Asn
           35                  40                  45
   Thr Glu Ala Gln Pro Lys Thr Glu Ala Leu Ala Ser Pro Thr Thr Thr
       50                  55                  60
   Thr Glu Lys Ala Pro Glu Thr Lys Pro Val Ala Asn Ala Val Ser Val
   65                  70                  75                  80
   Ser Asn Lys Glu Val Glu Ala Pro Thr Ser Glu Thr Lys Glu Ala Lys
                   85                  90                  95
   Glu Val Lys Glu Val Lys Ala Pro Lys Glu Thr Lys Ala Val Lys Pro
               100                 105                 110
   Ala Ala Lys Ala Asp Asn Asn Thr Tyr Pro Ile Leu Asn Gln Glu Leu
           115                 120                 125
   Arg Glu Ala Ile Lys Asn Pro Ala Ile Lys Asp Lys Asp His Ser Ala
       130                 135                 140
   Pro Asn Ser Arg Pro Ile Asp Phe Glu Met Lys Lys Glu Asn Gly Glu
   145                 150                 155                 160
   Gln Gln Phe Tyr His Tyr Ala Ser Ser Val Lys Pro Ala Arg Val Ile
                   165                 170                 175
   Phe Thr Asp Ser Lys Pro Glu Ile Glu Leu Gly Leu Gln Ser Gly Gln
               180                 185                 190
   Phe Trp Arg Lys Phe Glu Val Tyr Glu Gly Asp Lys Lys Leu Pro Ile
           195                 200                 205
   Lys Leu Val Ser Tyr Asp Thr Val Lys Asp Tyr Ala Tyr Ile Arg Phe
       210                 215                 220
   Ser Val Ser Asn Gly Thr Lys Ala Val Lys Ile Val Ser Ser Thr His
   225                 230                 235                 240
   Phe Asn Asn Lys Glu Glu Lys Tyr Asp Tyr Thr Leu Met Glu Phe Ala
                   245                 250                 255
   Gln Pro Ile Tyr Asn Ser Ala Asp Lys Phe Lys Thr Glu Glu Asp Tyr
               260                 265                 270
   Lys Ala Glu Lys Leu Leu Ala Pro Tyr Lys Lys Ala Lys Thr Leu Glu
           275                 280                 285
   Arg Gln Val Tyr Glu Leu Asn Lys Ile Gln Asp Lys Leu Pro Glu Lys
       290                 295                 300
   Leu Lys Ala Glu Tyr Lys Lys Lys Leu Glu Asp Thr Lys Lys Ala Leu
   305                 310                 315                 320
   Asp Glu Gln Val Lys Ser Ala Ile Thr Glu Phe Gln Asn Val Gln Pro
                   325                 330                 335
   Thr Asn Glu Lys Met Thr Asp Leu Gln Asp Thr Lys Tyr Val Val Tyr
               340                 345                 350
   Glu Ser Val Glu Asn Asn Glu Ser Met Met Asp Thr Phe Val Lys His
           355                 360                 365
   Pro Ile Lys Thr Gly Met Leu Asn Gly Lys Lys Tyr Met Val Met Glu
       370                 375                 380
   Thr Thr Asn Asp Asp Tyr Trp Lys Asp Phe Met Val Glu Gly Gln Arg
   385                 390                 395                 400
   Val Arg Thr Ile Ser Lys Asp Ala Ile Asn Asn Thr Arg Thr Ile Ile
                   405                 410                 415
   Phe Pro Tyr Val Glu Gly Lys Thr Leu Tyr Asp Ala Ile Val Lys Val
               420                 425                 430
   His Val Lys Thr Ile Asp Tyr Asp Gly Gln Tyr His Val Arg Ile Val
   435 440 445
   Asp Lys Glu Ala Phe Thr Lys Ala Asn Thr Asp Lys Ser Asn Lys Lys
   450 455 460
   Glu Gln Gln Asp Asn Ser Ala Lys Lys Glu Ala Thr Pro Ala Thr Pro
   465 470 475 480
   Ser Lys Pro Thr Pro Ser Pro Val Glu Lys Glu Ser Gln Lys Gln Asp
   485 490 495
   Ser Gln Lys Asp Asp Asn Lys Gln Leu Pro Ser Val Glu Lys Glu Asn
   500 505 510
   Asp Ala Ser Ser Glu Ser Gly Lys Asp Lys Thr Pro Ala Thr Lys Pro
   515 520 525
   Ala Lys Gly Glu Val Glu Ser Ser Ser Thr Thr Pro Thr Lys Val Val
   530 535 540
   Ser Thr Thr Gln Asn Val Ala Lys Pro Thr Thr Ala Ser Ser Lys Thr
   545 550 555 560
   Thr Lys Asp Val Val Gln Thr Ser Ala Gly Ser Ser Glu Ala Lys Asp
   565 570 575
   Ser Ala Pro Leu Gln Lys Ala Asn Ile Lys Asn Thr Asn Asp Gly His
   580 585 590
   Thr Gln Ser Gln Asn Asn Lys Asn Thr Gln Glu Asn Lys Ala Lys Ser
   595 600 605
   Leu Pro Gln Thr Gly Glu Glu Ser Asn Lys Asp Met Thr Leu Pro Leu
   610 615 620
   Met Ala Leu Leu Ala Leu Ser Ser Ile Val Ala Phe Val Leu Pro Arg
   625 630 635 640
   Lys Arg Lys Asn
<210> 107
   <211> 644
   <212> PRT
   <213> S. aureus
<400> 107
   Met Asn Lys Gln Gln Lys Glu Phe Lys Ser Phe Tyr Ser Ile Arg Lys
   1 5 10 15
   Ser Ser Leu Gly Val Ala Ser Val Ala Ile Ser Thr Leu Leu Leu Leu
   20 25 30
   Met Ser Asn Gly Glu Ala Gln Ala Ala Glu Glu Thr Gly Gly Thr Asn
   35 40 45
   Thr Glu Ala Gln Pro Lys Thr Glu Ala Val Ala Ser Pro Thr Thr Thr
   50 55 60
   Ser Glu Lys Ala Pro Glu Thr Lys Pro Val Ala Asn Ala Val Ser Val
   65 70 75 80
   Ser Asn Lys Glu Val Glu Ala Pro Thr Ser Glu Thr Lys Glu Ala Lys
   85 90 95
   Glu Val Lys Glu Val Lys Ala Pro Lys Glu Thr Lys Ala Val Lys Pro
   100 105 110
   Ala Thr Lys Ala Asp Asn Asn Thr Tyr Pro Ile Leu Asn Gln Glu Leu
   115 120 125
   Arg Glu Ala Ile Lys Asn Pro Ala Ile Lys Asp Lys Asp His Ser Ala
   130 135 140
   Pro Asn Ser Arg Pro Ile Asp Phe Glu Met Lys Lys Glu Asn Gly Glu
   145 150 155 160
   Gln Gln Phe Tyr His Tyr Ala Ser Ser Val Lys Pro Ala Arg Val Ile
                   165                 170                 175
   Phe Thr Asp Ser Lys Pro Glu Ile Glu Leu Gly Leu Gln Ser Gly Gln
               180                 185                 190
   Phe Trp Arg Lys Phe Glu Val Tyr Glu Gly Asp Lys Lys Leu Pro Ile
           195                 200                 205
   Lys Leu Val Ser Tyr Asp Thr Val Lys Asp Tyr Ala Tyr Ile Arg Phe
       210                 215                 220
   Ser Val Ser Asn Gly Thr Lys Ala Val Lys Ile Val Ser Ser Thr His
   225                 230                 235                 240
   Phe Asn Asn Lys Glu Glu Lys Tyr Asp Tyr Thr Leu Met Glu Phe Ala
                   245                 250                 255
   Gln Pro Ile Tyr Asn Ser Ala Asp Lys Phe Lys Thr Glu Glu Asp Tyr
               260                 265                 270
   Lys Ala Glu Lys Leu Leu Ala Pro Tyr Lys Lys Ala Lys Thr Leu Glu
           275                 280                 285
   Arg Gln Val Tyr Glu Leu Asn Lys Ile Gln Asp Lys Leu Pro Glu Lys
       290                 295                 300
   Leu Lys Ala Glu Tyr Lys Lys Lys Leu Glu Asp Thr Lys Lys Ala Leu
   305                 310                 315                 320
   Asp Glu Gln Val Lys Ser Ala Ile Thr Glu Phe Gln Asn Val Gln Pro
                   325                 330                 335
   Thr Asn Glu Lys Met Thr Asp Leu Gln Asp Thr Lys Tyr Val Val Tyr
               340                 345                 350
   Glu Ser Val Glu Asn Asn Glu Ser Met Met Asp Thr Phe Val Lys His
           355                 360                 365
   Pro Ile Lys Thr Gly Met Leu Asn Gly Lys Lys Tyr Met Val Met Glu
       370                 375                 380
   Thr Thr Asn Asp Asp Tyr Trp Lys Asp Phe Met Val Glu Gly Gln Arg
   385                 390                 395                 400
   Val Arg Thr Ile Ser Lys Asp Ala Lys Asn Asn Thr Arg Thr Ile Ile
                   405                 410                 415
   Phe Pro Tyr Val Glu Gly Lys Thr Leu Tyr Asp Ala Ile Val Lys Val
               420                 425                 430
   His Val Lys Thr Ile Asp Tyr Asp Gly Gln Tyr His Val Arg Ile Val
           435                 440                 445
   Asp Lys Glu Ala Phe Thr Lys Ala Asn Thr Asp Lys Ser Asn Lys Lys
       450                 455                 460
   Glu Gln Gln Asp Asn Ser Ala Lys Lys Glu Ala Thr Pro Ala Thr Pro
   465                 470                 475                 480
   Ser Lys Pro Thr Pro Ser Pro Val Glu Lys Glu Ser Gln Lys Gln Asp
                   485                 490                 495
   Ser Gln Lys Asp Asp Asn Lys Gln Leu Pro Ser Val Glu Lys Glu Asn
               500                 505                 510
   Asp Ala Ser Ser Glu Ser Gly Lys Asp Lys Thr Pro Ala Thr Lys Pro
           515                 520                 525
   Ala Lys Gly Glu Val Glu Ser Ser Ser Thr Thr Pro Thr Lys Val Val
       530                 535                 540
   Ser Thr Thr Gln Asn Val Ala Lys Pro Thr Thr Ala Ser Ser Lys Thr
   545                 550                 555                 560
   Thr Lys Asp Val Val Gln Thr Ser Ala Gly Ser Ser Glu Ala Lys Asp
                   565                 570                 575
   Ser Ala Pro Leu Gln Lys Ala Asn Ile Lys Asn Thr Asn Asp Gly His
               580                 585                 590
   Thr Gln Ser Gln Asn Asn Lys Asn Thr Gln Glu Asn Lys Ala Lys Ser
           595                 600                 605
   Leu Pro Gln Thr Gly Glu Glu Ser Asn Lys Asp Met Thr Leu Pro Leu
       610                 615                 620
   Met Ala Leu Leu Ala Leu Ser Ser Ile Val Ala Phe Val Leu Pro Arg
   625                 630                 635                 640
   Lys Arg Lys Asn

## Description

### CROSS-REFERENCE TO RELATED APPLICATIONS

The present application claims the benefit of U.S. Provisional Application No. 60/489,840, filed July 24, 2003 and U.S. Provisional Application No. 60/520,115, filed November 14, 2003.

### BACKGROUND OF THE INVENTION

The references cited throughout the present application are not admitted to be prior art to the claimed invention.

*Staphylococcus aureus* is a pathogen responsible for a wide range of diseases and conditions. Examples of diseases and conditions caused by *S. aureus* include bacteremia, infective endocarditis, folliculitis, furuncle, carbuncle, impetigo, bullous impetigo, cellulitis, botryomyosis, toxic shock syndrome, scalded skin syndrome, central nervous system infections, infective and inflammatory eye disease, osteomyletitis and other infections of joints and bones, and respiratory tract infections. *(*The Staphylococci in Human Disease, Crossley and Archer (eds.), Churchill Livingstone Inc. 1997.)

Immunological based strategies can be employed to try to control *S. aureus* infections and the spread of *S. aureus.* Immunological based strategies include passive and active immunization. Passive immunization employs immunoglobulins targeting *S. aureus.* Active immunization induces immune responses against *S. aureus.*

Potential *S. aureus* vaccines target *S. aureus* polysaccharides and polypeptides. Targeting can be achieved using suitable *S. aureus* polysaccharides or polypeptides as vaccine components. Examples of potential polysaccharides vaccine components include *S. aureus* type 5 and type 8 capsular polysaccharides. *(*Shinefield et al., N. Eng. J. Med. 346:491-496, 2002.) Examples of polypeptides that may be employed as possible vaccine components include collagen adhesin, fibrinogen binding proteins, and clumping factor. (Mamo et al., FEMS Immunology and Medical Microbiology 10:47-54, 1994, Nilsson et al., J. Clin. Invest. 101:2640-2649, 1998, Josefsson et al., The Journal of Infectious Diseases 184:1572-1580, 2001.)

Information concerning *S. aureus* polypeptide sequences has been obtained from sequencing the *S. aureus* genome. (Kuroda et al., Lancet 357:1225-1240, 2001, Baba et al., Lancet 359:1819-1827, 2000, Kunsch *et al.,* European Patent Publication EP 0 786 519, published July 30, 1997.) To some extent bioinformatics has been employed in efforts to characterize polypeptide sequences obtained from genome sequencing. (Kunsch *et al.,* European Patent Publication EP 0 786 519, published July 30, 1997.)

Techniques such as those involving display technology and sera from infected patients can be used as part of an effort to try to identify genes coding for potential antigens. (Foster *et al.,* International Publication Number WO 01/98499, published December 27,2001, Meinke *et al.,* International Publication Number WO 02/059148, published August 1, 2002.)

### SUMMARY OF THE INVENTION

The present invention features polypeptides comprising an amino acid sequence structurally related to SEQ ID NO: 1, uses of such polypeptides, and expression systems for producing such polypeptides. SEQ ID NO: 1 is a truncated derivative of a full length *S. aureus* polypeptide. The full-length polypeptide is referred to herein as full-length "ORF0657n". Polypeptides containing the amino acid sequence of SEQ ID NO: 1 were found to produce a protective immune response against *S*. *aureus.*

Reference to "protective" immunity or immune response indicates a detectable level of protection against *S. aureus* infection. The level of protection can be assessed using animal models such as those described herein.

Thus the present invention provides a polypeptide immunogen comprising an amino acid sequence at least 90% identical to SEQ ID NO: 1, wherein the polypeptide does not contain a carboxyl terminus provided by amino acids 609-645 of SEQ ID NO: 2 and the polypeptide provides protective immunity against *S*. *aureus.* SEQ ID NO: 2 provides a full length ORF0657n polypeptide, wherein amino acids 609-645 provide the carboxyl terminus domain starting at the LPXTG motif (as referred to herein as the "cell wall sorting signal").

Reference to "immunogen" indicates the ability to provide protective immunity.

Reference to comprising an amino acid sequence at least 90% identical to SEQ ID NO: 1 indicates that a SEQ ID NO: 1 related region is present and additional polypeptide regions may be present. If additional polypeptide regions are present, then the polypeptide does not have a carboxyl LPXTG motif as provided by amino acids 609-645 of SEQ ID NO: 2.

Another aspect of the present invention describes an immunogen comprising an amino acid sequence that provides protective immunity against *S*. *aureus.* The immunogen comprises an amino acid sequence at least 90% identical to SEQ ID NO: 1 and one or more additional regions or moieties covalently joined at the carboxyl terminus or amino terminus, said additional regions or moieties do not provide a carboxyl terminus containing amino acids 609-645 of SEQ ID NO:2, wherein each region or moiety is independently selected from a region or moiety having at least one of the following properties: enhances the immune response, facilitates purification, or facilitates polypeptide stability.

Reference to "additional region or moiety" indicates a region or moiety different from a ORF0657n related polypeptide which would be produced in a biological host, such as a prokaryotic or eukaryotic host. The additional region or moiety can be, for example, an additional polypeptide region or a non-peptide-region.

Another aspect of the present invention describes a composition able to induce protective immunity against *S. aureus* in a patient. The composition comprises a pharmaceutically acceptable carrier and an immunologically effective amount of an immunogen as described above providing protective immunity against *S*. *aureus*. An immunologically effective amount is an amount sufficient to provide protective immunity against *S*. *aureus* infection. The amount should be sufficient to significantly prevent the likelihood or severity of a *S*. *aureus* infection.

Another aspect of the present invention describes a nucleic acid comprising a recombinant gene encoding a polypeptide as described above that provides protective immunity against *S. aureus*. A recombinant gene contains recombinant nucleic acid encoding a polypeptide along with regulatory elements for proper transcription and processing (which may include translational and post translational elements). The recombinant gene can exist independent of a host genome or can be part of a host genome.

A recombinant nucleic acid is nucleic acid that by virtue of its sequence and/or form does not occur in nature. Examples of recombinant nucleic acid include purified nucleic acid, two or more nucleic acid regions combined together that provides a different nucleic acid than found in nature, and the absence of one or more nucleic acid regions (*e.g.*, upstream or downstream regions) that are naturally associated with each other.

Another aspect of the present invention describes a recombinant cell. The cell comprises a recombinant gene encoding a polypeptide as described above that provides protective immunity against *S. aureus.*

Another aspect of the present invention describes a method of making a polypeptide as described above that provides protective immunity against *S. aureus.* The method involves growing a recombinant cell containing recombinant nucleic acid encoding the polypeptide and purifying the polypeptide.

Another aspect of the present invention describes a polypeptide as described above that provides protective immunity against *S. aureus* made by a process comprising the steps of growing a recombinant cell containing recombinant nucleic acid encoding the polypeptide in a host and purifying the polypeptide. Different host cells can be employed. In an embodiment of the present invention the host cell is a yeast cell.

Another aspect of the present invention describes an immunogen as described above for inducing a protective immune response in a patient against *S*. *aureus*. The immunogen is administered to the patient in an immunologically effective amount that provides protective immunity againt *S. aureus.*

Another aspect of the present invention describes an immunogen as described above for inducing an anamnestic response in a patient. The method comprises the step of administering to the patient an effective amount of an immunogen to produce the anamnestic response.

Another aspect of the present invention describes nucleic acid encoding an ORF0657n related polypeptide that is optimized for expression in yeast. One or more codons are optimized for yeast expression.

Another aspect of the present invention describes a method of making a polypeptide as described above that provides protective immunity against *S. aureus* in a recombinant yeast cell. The method comprises the steps of:
(a) growing a recombinant yeast cell under conditions wherein the polypeptide is expressed, wherein the recombinant yeast cell comprises a recombinant gene encoding the polypeptide and the polypeptide is a full-length ORF0657n related polypeptide that provides protective immunity against *S*. *aureus* infection, or a fragment thereof comprising an amino acid sequence at least 90% identical to SEQ ID NO: 1; and
(b) purifying the polypeptide.

Unless particular terms are mutually exclusive, reference to "or" indicates either or both possibillities. Occasionally phrases such as "and/or" are used to highlight either or both possibilities.

Reference to open-ended terms such as "comprises" allows for additional elements or steps. Occasionally phrases such as "one or more" are used with or without open-ended terms to highlight the possibility of additional elements or steps.

Unless explicitly stated reference to terms such as "a" or "an" is not limited to one, For example, "a cell" does not exclude "cells". Occasionally phrases such as one of more are used to highlight the presence of a plurality.

Other features and advantages of the present invention are apparent from the additional descriptions provided herein including the different examples. The provided examples illustrate different components and methodology useful in practicing the present invention. The examples do not limit the claimed invention. Based on the present disclosure the skilled artisan can identify and employ other components and methodology useful for practicing the present invention.

### BRIEF DESCRIPTION OF THE DRAWINGS

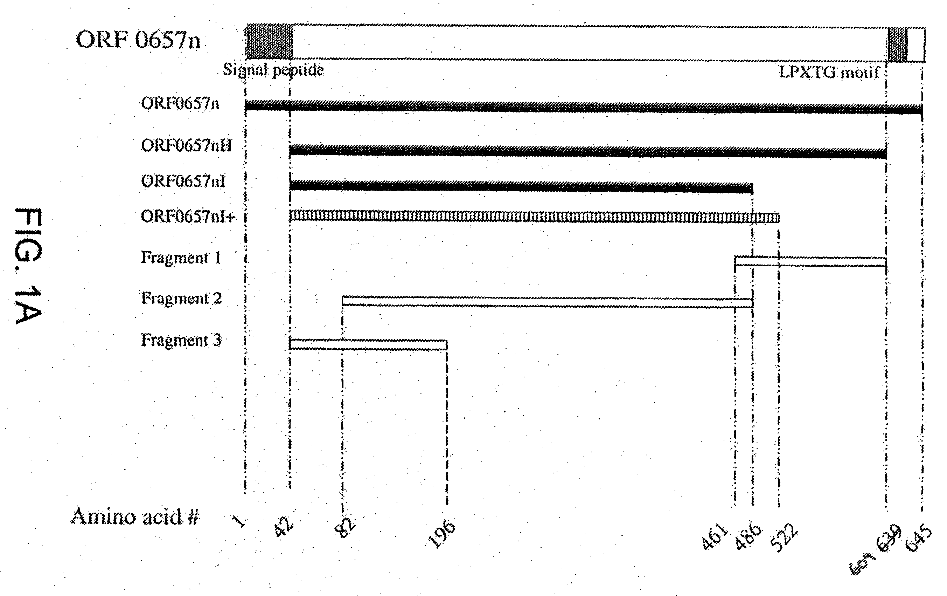

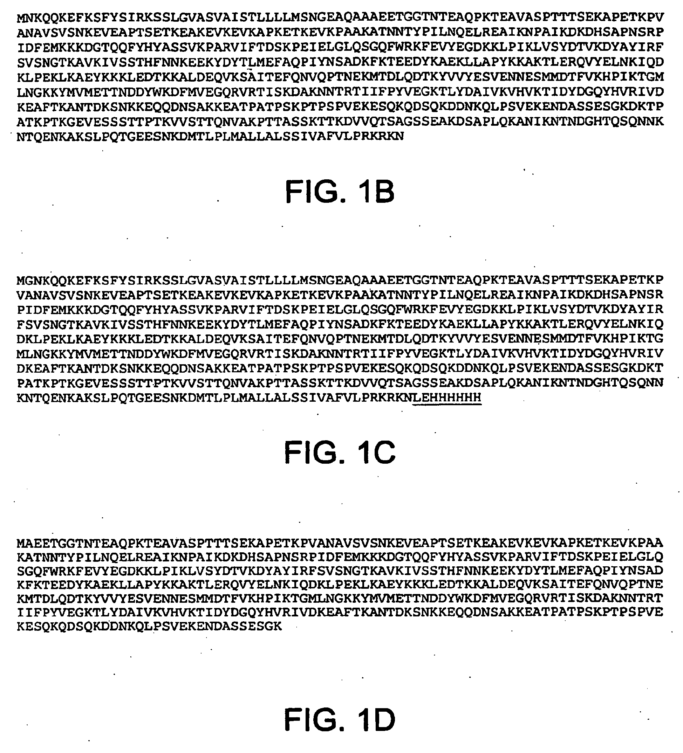

Figures 1A, 1B, 1C, and 1D provide a schematic illustrating ORF0657n related polypeptide regions screened for protection in animals, and some different ORF0657n sequences. Figure 1A illustrates a schematic of polypeptides that were tested and found protective (shown by filled in rectangles), polypeptides tested and found not to be protective (shown by open rectangles), and a polypeptide not tested (hatched box). Figure 1B provides a full-length sequence used as a reference for Figure 1A (SEQ ID NO: 2). Figure 1C illustrates SEQ ID NO: 28. SEQ ID NO: 28 contains a carboxyl "His-Tag" (LEHHHHHH; SEQ ID NO: 64). SEQ ID NO: 28 containing a carboxyl His-Tag is also referred to herein as "His-Tag ORF0657n". Figure 1D illustrates an ORF0657nI⁺ sequence.

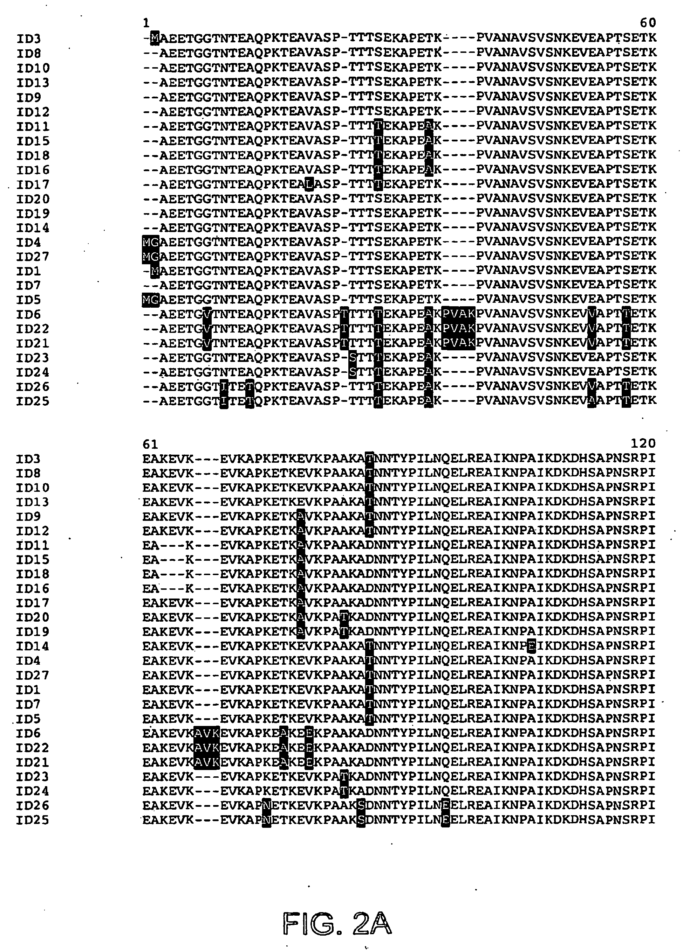

Figures 2A-2E provides a sequence comparison of different ORF0657n related sequences across the ORF0657nH region. SEQ ID NOs: are indicated in the Figure by "ID".

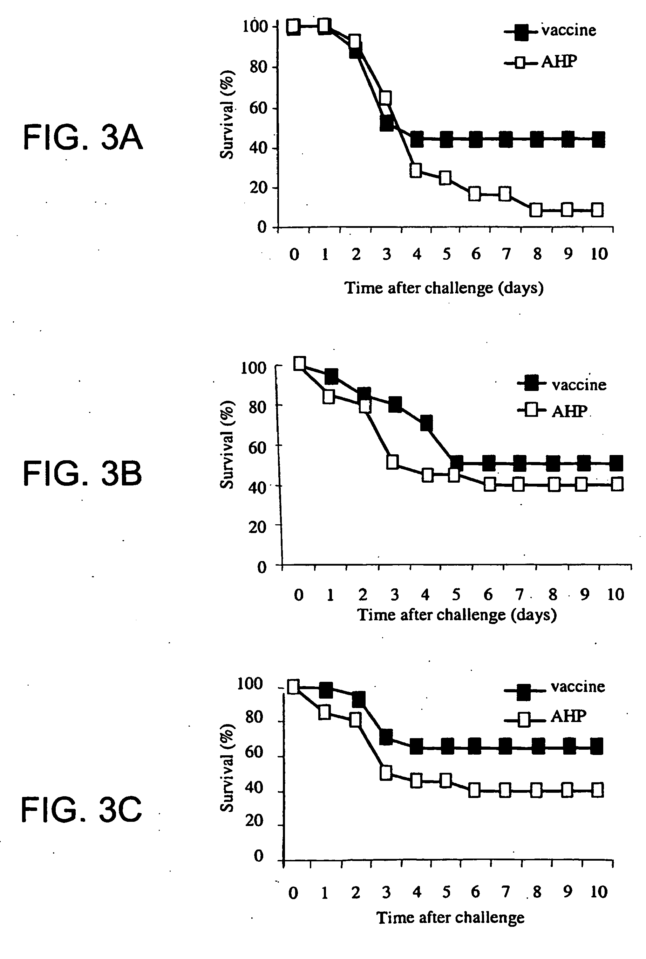

Figures 3A, 3B, and 3C illustrates the ability of ORF0657n related polypeptides providing the full-length sequence, the OFR0657nH region and the ORF0657nI region to provide protective immunity against *S. aureus* Becker. The polypeptides were used with an aluminum hydroxyphosphate adjuvant (AHP). Figure 3A illustrates results with SEQ ID NO: 28. Figure 3B illustrates results with SEQ ID NO: 4 containing a carboxyl His-Tag. Figure 3C illustrates results with SEQ ID NO: 5 containing a carboxyl His-Tag. Reference to a "carboxyl His-Tag" indicates the His-Tag group LEHHHHHH (SEQ ID NO: 64) is present at the carboxyl terminus.

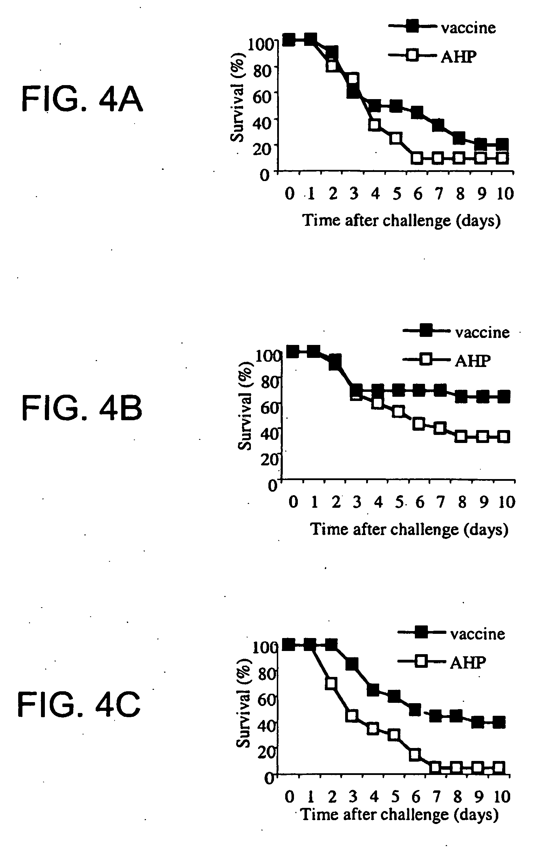

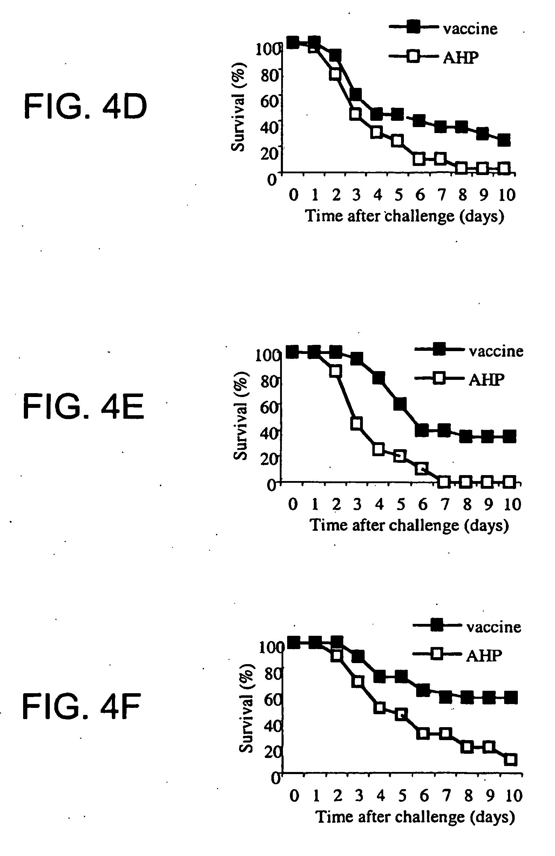

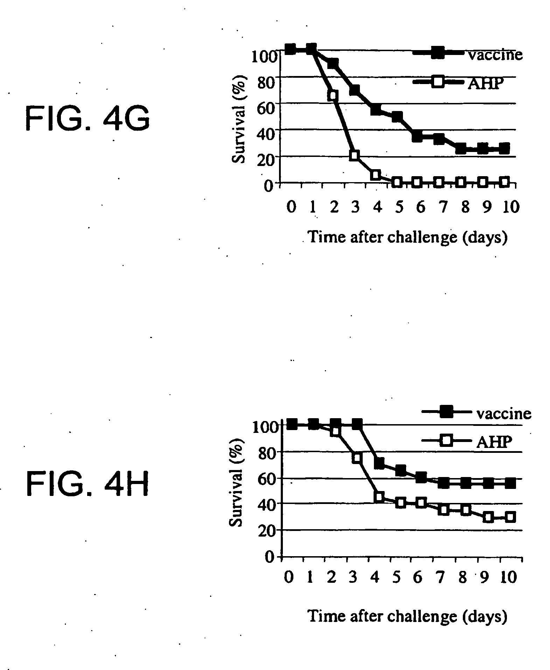

Figures 4A-4H illustrates the ability of ORF0657n related polypeptides to provide protective immunity against different *S. aureus* challenges. The polypeptide of SEQ ID NO: 28 was used as immunogen Figure 4A shows results using challenge strain CL-10 (2.2 X 10⁸ CFU/ml). Figure 4B shows results using challenge strain CL-10 (2.1 X 10⁸ CFU/ml). Figure 4C shows results using challenge strain CL-13 (2.9 X 10⁸ CFU/ml). Figure 4D shows results using challenge strain CL-13 (2.8 X 10⁸ CFU/ml). Figure 4E shows results using challenge strain CL-30 (3.1 X 10⁸ CFU/ml). Figure 4F shows results using challenge strain CL-30 (3.0 X 10⁸ CFU/ml). Figure 4G shows results using challenge strain CL-18 (1.0 X 10⁸ CFU/ml. Figure 4H shows results using challenge strain CL-21 (1.6 X 10⁸ CFU/ml).

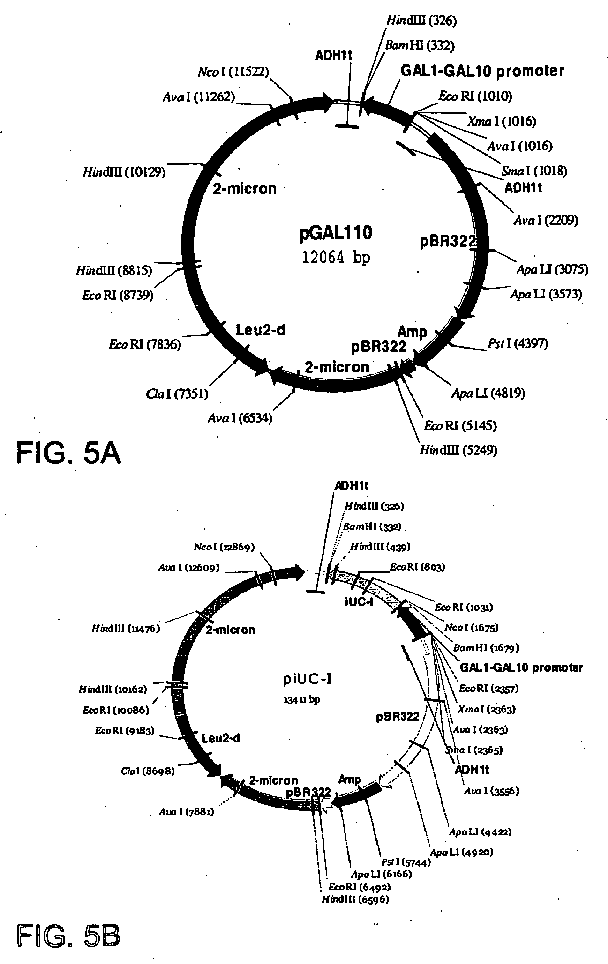

Figures 5A and 5B illustrate plasmid maps of *S. cerevisiae* expression plasmids. Figure 5A provides a plasmid map of vector pGAL110. Figure 5B provides a plasmid map for piUC-I showing a yeast codon-optimized sequence cloned under control of the *GAL1* promoter of pGAL110.

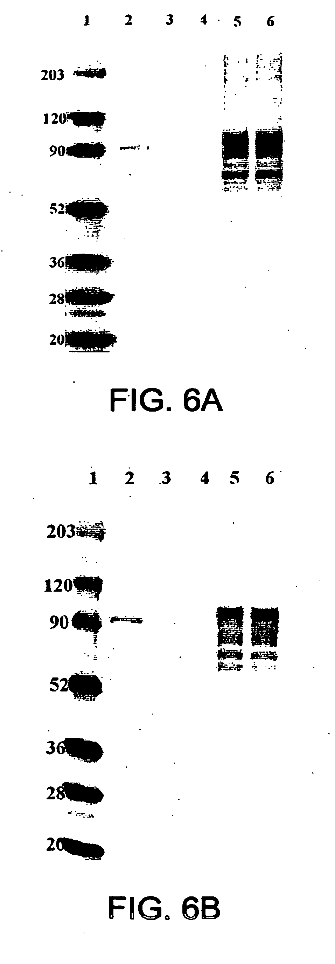

Figures 6A and 6B show Western blots depicting intracellular expression of a full-length ORF0657n having amino acids 1-646 of SEQ ID NO: 28 (SEQ ID NO: 28 without the carboxyl His-Tag). Lane 1, molecular size standards; lane 2, purified *E*. *coli* produced recombinant full-length ORF0657n region (SEQ ID NO: 28), 100 ng; lanes 3-6 contain 20 µg of yeast cell lysate; lanes 3 and 4, cell lysates from duplicate fermentations of transformants of 1260 (Figure 6A) and 1309 (Figure 6B) containing only vector pGAL110; lanes 5 and 6, cell lysates from duplicate fermentations of transformants of 1260 (Figure 6A) and 1309 (Figure 6B) containing pRUnkC-pGAL110 which expresses full-length ORF0657n region (SEQ ID NO: 28 without the carboxyl His-Tag).

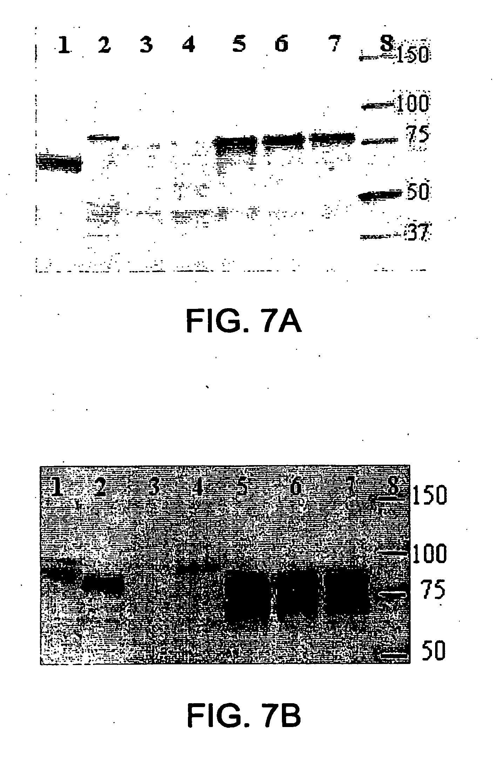

Figures 7A and 7B show a Coomassie stain of an SDS-PAGE gel and a Western blot, respectively, depicting intracellular expression in *S*. *cerevisiae* from nucleic acid encoding SEQ ID NO: 3. Lane 1, Panel A, BSA, 1.25 µg; Panel B, purified *E. coli* recombinant full length ORF0657n (SEQ ID NO: 28), 100 ng; lane 2, cell-lysate from producer of ORF0657nH (SEQ ID NO: 4 with a carboxyl His-Tag) in *E. coli;* Panel A, 1.25 µg, Panel B, 0.5 µg. Lanes 3-7, Panels A and B, contain 1.25 and 0.5 µg of yeast cell-lysate, respectively: lane 3, transformant containing only vector pGAL110; lane 4, transformant containing full-length ORF0657n (SEQ ID NO: 28 without the carboxyl His-Tag); lanes 5, 6, and 7, transformant 1-1 that contains piUC-S(-) which expresses mature ORF0657nH region (SEQ ID NO: 3); lane 7 contains a cell-lysate (of transformant 1-1) that was frozen and subsequently thawed. Lane 8 contains molecular size standards.

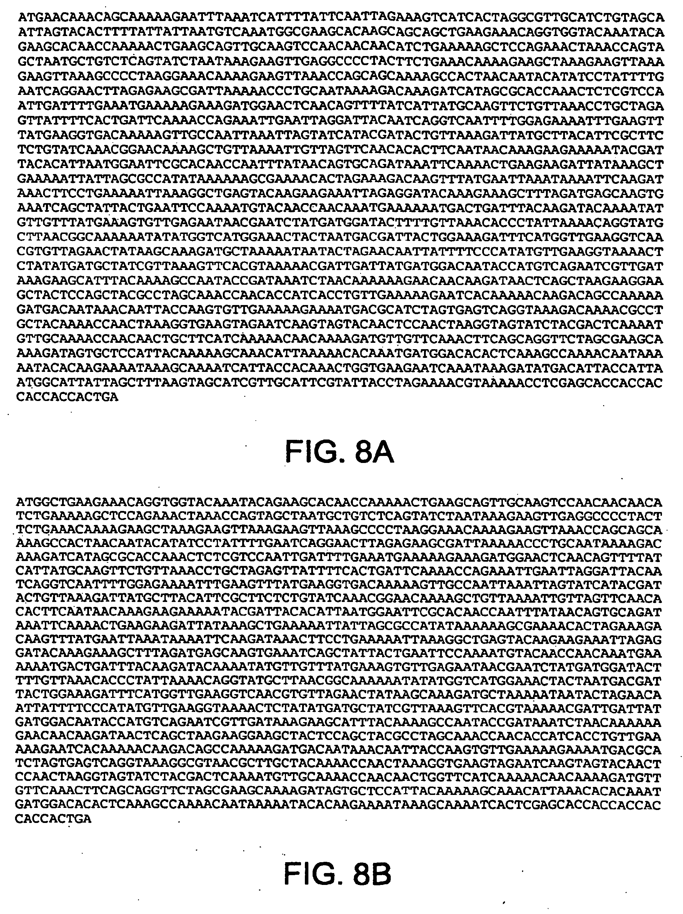

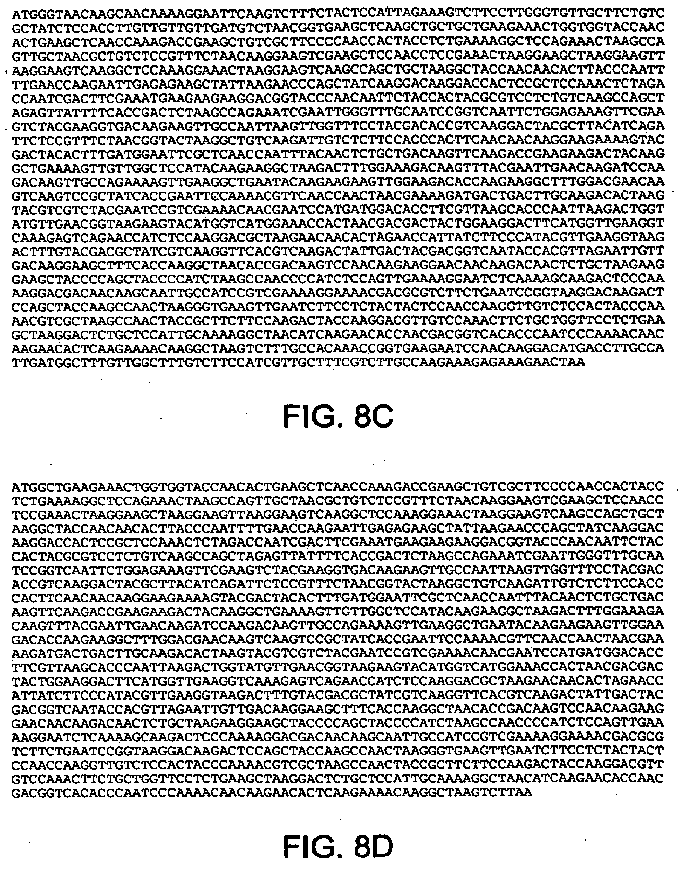

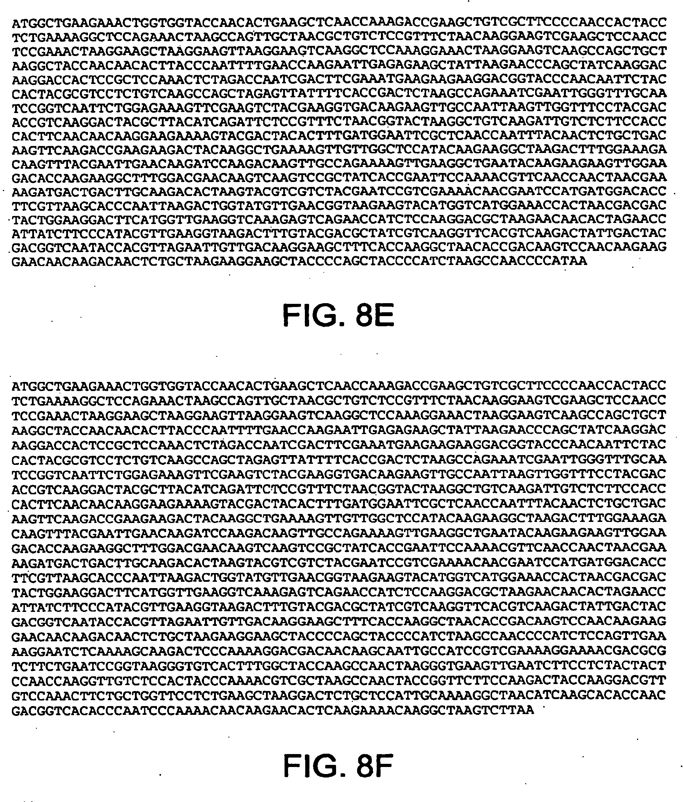

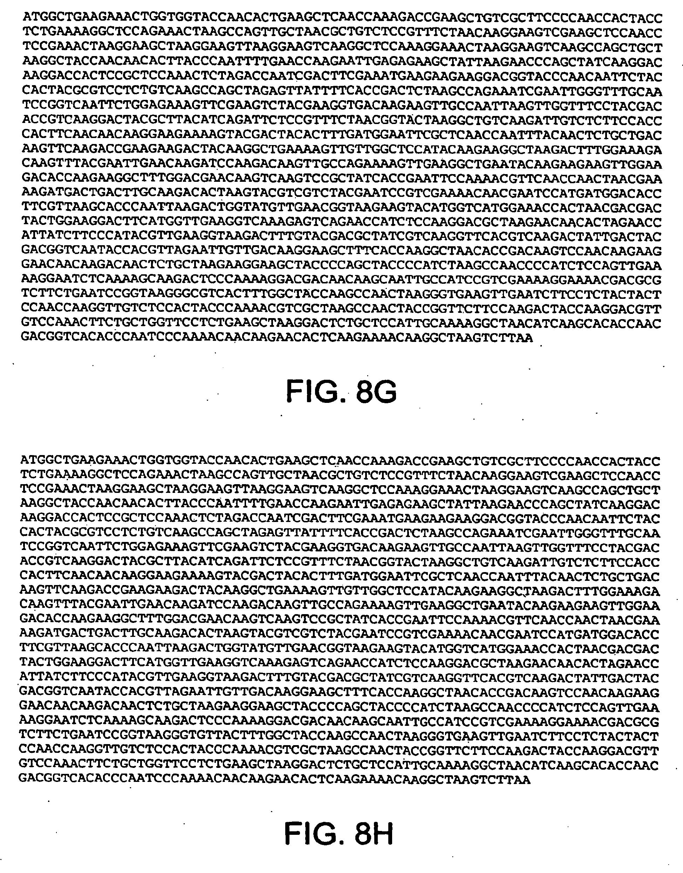

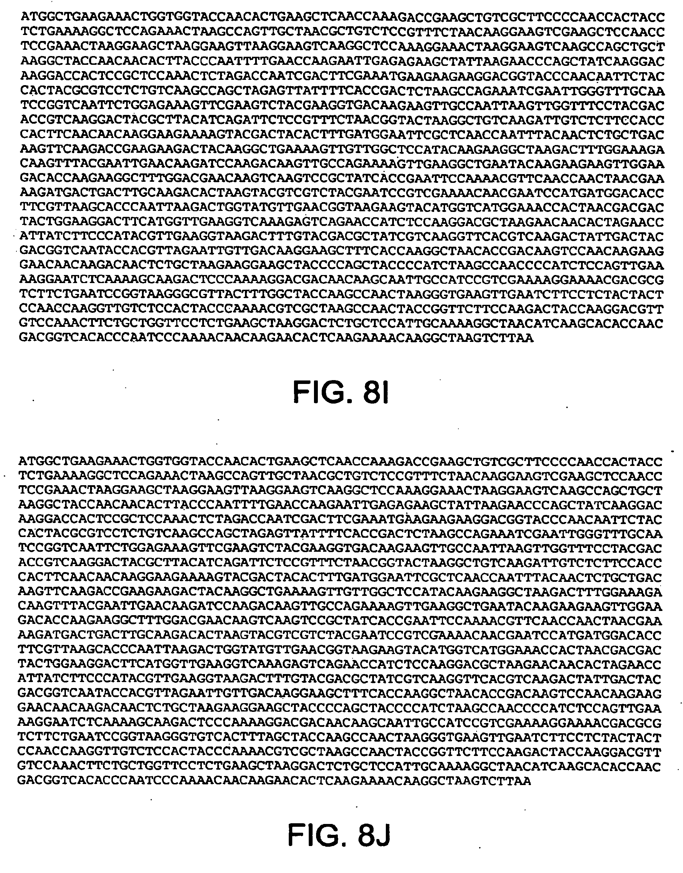

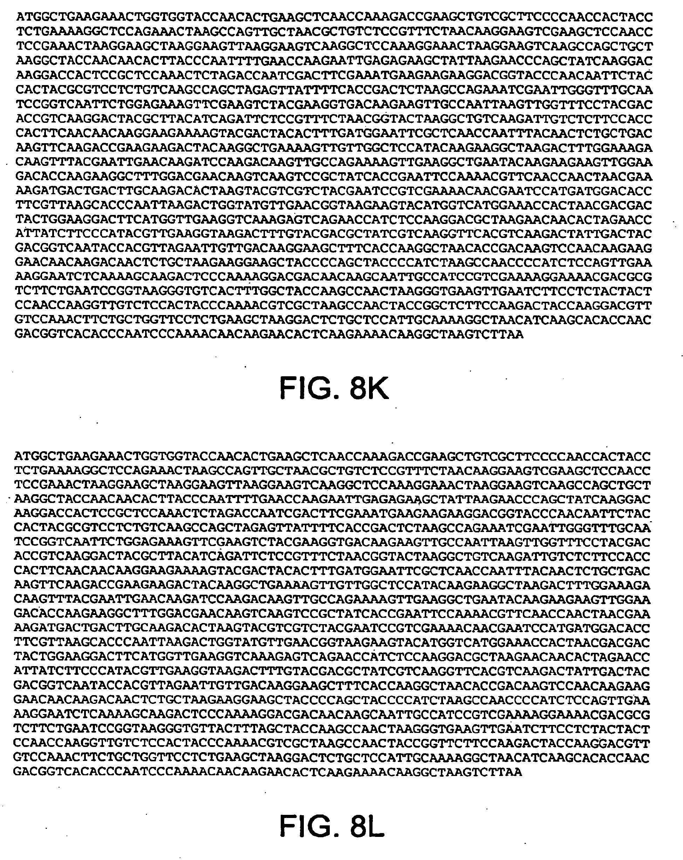

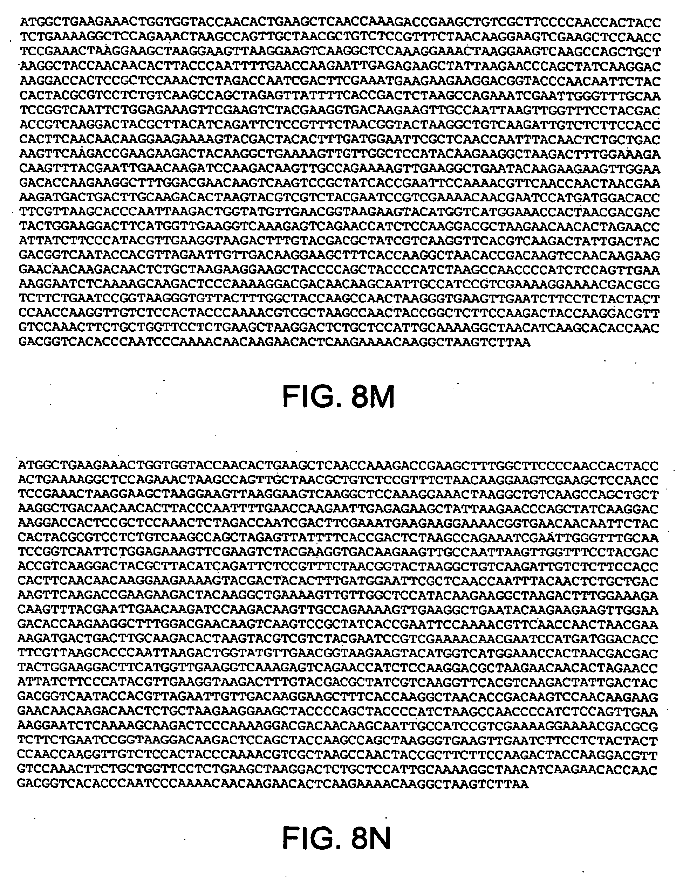

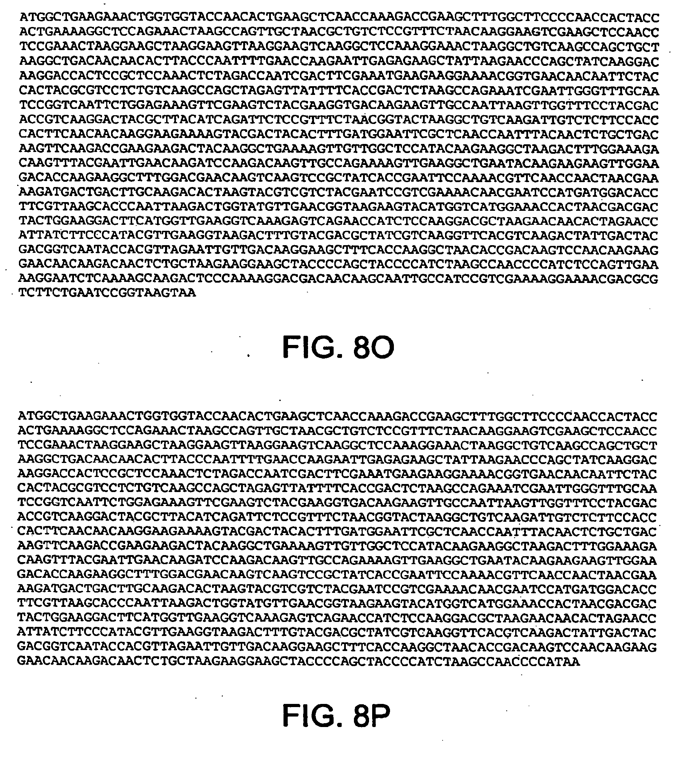

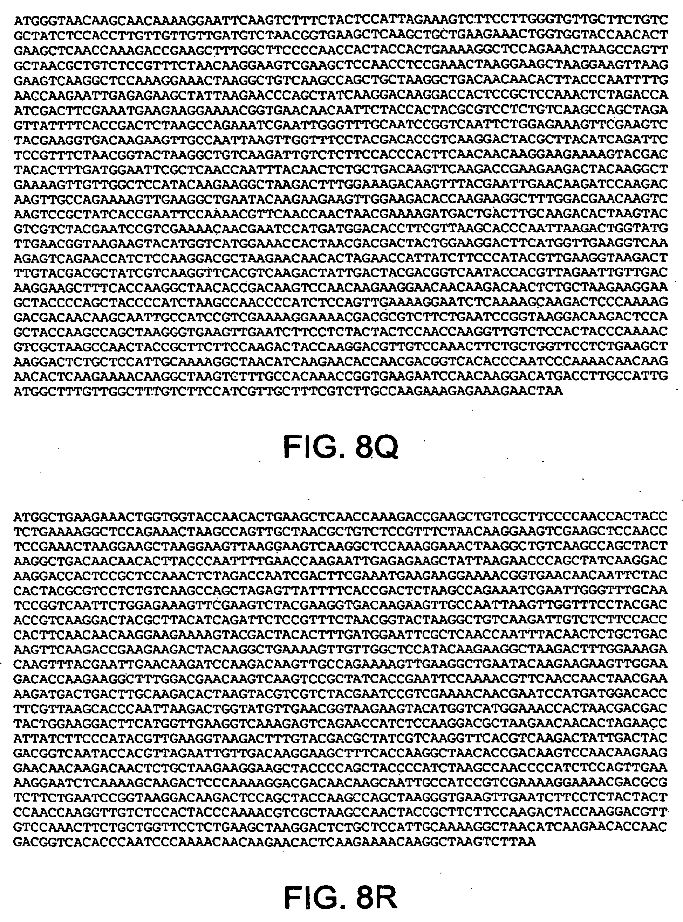

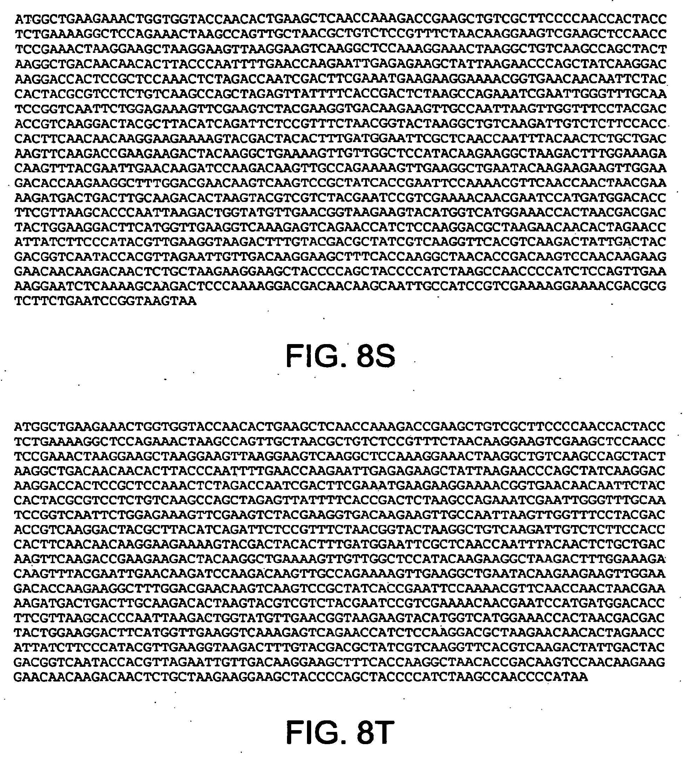

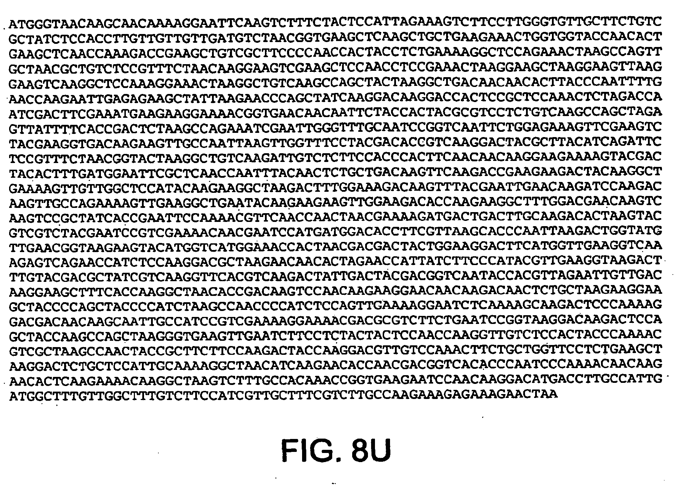

Figures 8A-8U provide examples of different nucleic acid sequences encoding for ORF0657n related polypeptides. Figure 8A provides a nucleic acid sequence (SEQ ID NO: 29) encoding for SEQ ID NO: 2 plus a carboxyl His-Tag. Figure 8B provides a nucleic acid sequence (SEQ ID NO: 30) encoding SEQ ID NO: 4 plus a carboxyl His-Tag. Figure 8C provides a yeast optimized nucleic acid sequence (SEQ ID NO: 31) encoding SEQ ID NO: 28 without a carboxyl His-Tag. Figure 8D provides a yeast optimized nucleic acid sequence (SEQ ID NO: 32) encoding SEQ ID NO: 3. Figure 8E provides a yeast optimized nucleic acid (SEQ ID NO: 33) sequence encoding SEQ ID NO: 1. Figures 8F-8M provide yeast optimized nucleic acid sequences (SEQ ID NOs: 34, 35, 36, 37, 38, 39, 40, and 41) encoding SEQ ID NO: 7 containing an amino terminus methionine. Figures 8N-8U provide yeast optimized nucleic acid sequences (SEQ ID NOs: 46-53) encoding different ORF0657n related polypeptides based on SEQ ID NO: 17 or SEQ ID NO: 20.

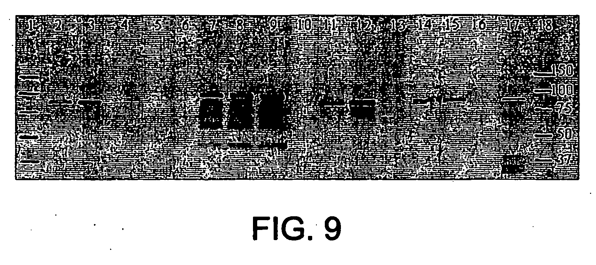

Figure 9 shows a Western blot comparing intracellular expression of ORF0657n related polypeptides in *S*. *cerevisiae.* Lanes 1 and 18, MW size standards. Lanes 2 and 3, 50 and 100 ng, respectively of purified ORF0657nH produced in *E. coli* (SEQ ID NO: 4 plus a carboxyl His-Tag) Lane 5 contains 500 ng protein of cell lysate from *S. cerevisiae* vector control transformant Lanes 7, 8, and 9 contain 1.0, 2.0, and 4.0 µg cell lysate protein from *S. cerevisiae* transformant producing SEQ ID NO: 28 without the carboxyl His-Tag. Lanes 11 and 12 contain 50 and 100 ng protein, respectively, of cell lysate from *S*. *cerevisiae* transformant producing ORF0657nH (SEQ ID NO: 3). Lanes 14 and 15 contain 250 and 500 ng protein, respectively, of cell lysate from *S. cerevisiae* transformant producing ORF0657nG (SEQ ID NO: 44). Lane 17 contains 250 ng cell lysate protein from ORF0657nG (SEQ ID NO: 44 plus a carboxyl His-Tag) *E. coli* producer. Lanes 4, 6, 10, 13, and 16 are empty.

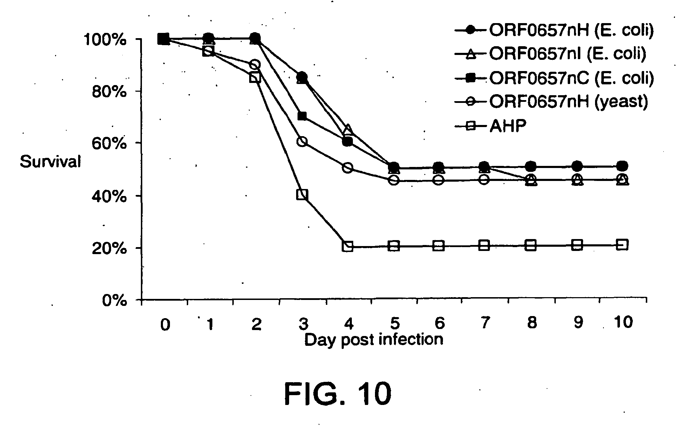

Figure 10 illustrates protective immunity data for ORF0657n related polypeptides produced in *E. coli* and yeast. ORF0657nH (*E. coli*)" corresponds to SEQ ID NO: 4 with a carboxyl His-Tag "ORF0657nI (*E. coli*)" corresponds to SEQ ID NO: 5 with a carboxyl His-Tag. "ORF0657nC (*E. coli*)" corresponds to SEQ ID NO: 28. "ORF0657nH (yeast)" corresponds to SEQ ID NO: 3.

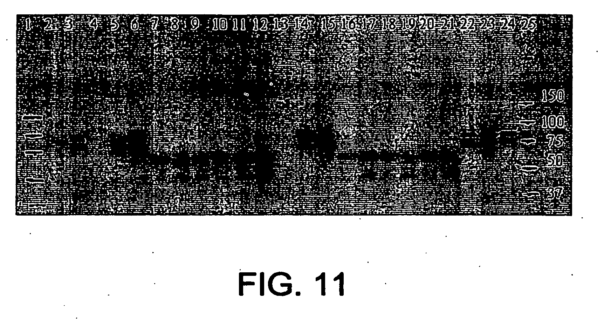

Figure 11 shows an exemplary Western blot of intracellular expression of ORF0657nI in *S*. *cerevisiae*. Lanes 1 and 25: MW size standards. Lanes 2, 3, and 24: 25, 50, and 100 ng, respectively, of purified ORF0657nH region (SEQ ID NO: 4 with a carboxyl His-Tag) produced in *E. coli*. Lanes 4-23 contain protein cell lysate from yeast transformants. Lanes 13-21 represent duplicate cell-lysates prepared from the same fermentation sample as the lysates in lanes 4-12. Lanes 4 and 13 contain 200 ng protein from vector control transformant containing pGAL110. Lanes 5 and 14 contain 100 ng protein from transformant 1-1 which produces ORF0657nH region (SEQ ID NO: 3). Lanes 6 and 15 contain 200 ng protein from transformant 1-1. Lanes 7 and 16 contain 100 ng protein and lanes 8 and 17 contain 200 ng of protein from transformant I1. Lanes 9 and 18 contain 100 ng of protein and lanes 10 and 19 contain 200 ng protein from transformant I2. Lanes 11 and 20 contain 100 ng protein and lanes 12 and 21 contain 200 ng protein from transformant I3. Lanes 22 and 23 contain 100 and 200 ng protein cell-lysate prepared previously from a prior fermentation of transformant 1-1.

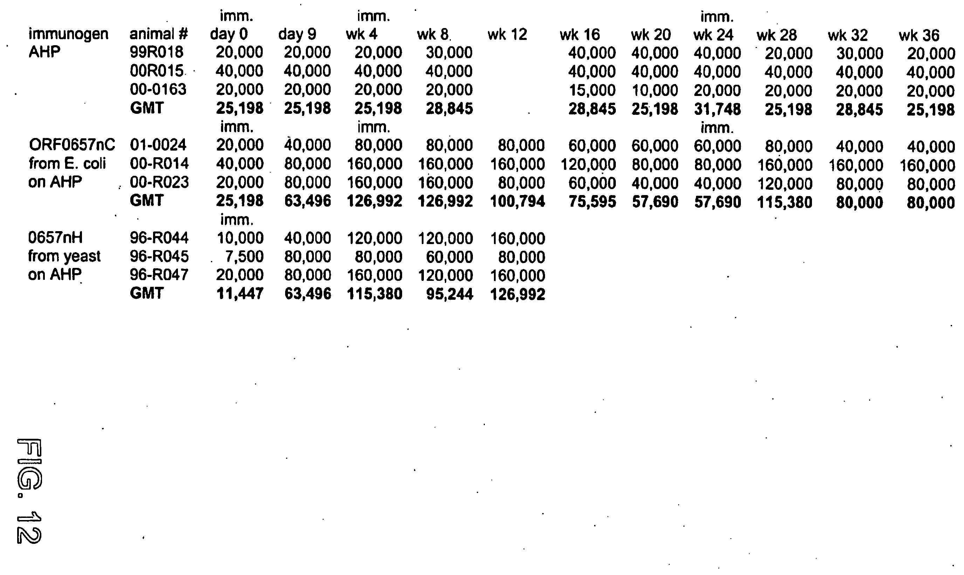

Figure 12 provides Rhesus monkeys immunization data. Rhesus monkeys were immunized with either yeast produced ORF0657n related polypeptide (ORF0657nH, SEQ ID NO: 3) or *E coli*-expressed ORF0657n related polypeptide (full-length ORF0657nC, SEQ ID NO: 28) formulated with or without AHP The monkeys in the vaccine group received 50 mcg ORF0657n related polypeptides via the intramuscular route.

### DETAILED DESCRIPTION OF THE INVENTION

ORF0657n related polypeptides including fun-length and shorter length derivatives containing the ORF0657nI region were found to provide protective immunity against *S. aureus* using an animal model. Figure la illustrates the location of different ORF0657n related polypeptide regions providing protective immunity against an *S. aureus* infection and regions that did not provide protective immunity. In Figure 1a, ORF0657n refers to a full-length sequence corresponding to SEQ ID NO: 2, ORF0657nI refers to a region corresponding to SEQ ID NO: 1 (without the amino terminus methionine), and ORF0657nH refers to a region corresponding to SEQ ID NO: 3 (without the amino terminus methionine).

An ORF0657n "related" polypeptide contains a region structurally related to a full-length ORF0657n or a fragment thereof. ORF0657n related polypeptides are polypeptides having at least about90% sequence identity to a corresponding region of a naturally occurring ORFO657n. The reference ORF0657n illustrated in Figure 1 corresponds to ORF0657n from *S. aureus* COL (SEQ ID NO: 2).

Percent identity to a reference sequence is determined by aligning the polypeptide sequence with the reference sequence and determining the number of identical amino acids. This number, is divided by the total number of amino acids in the reference sequence and then multiplied by 100 and rounded to the nearest whole number.

Figure la helps illustrate the importance of a core region comprising an amino acid sequence structurally related to SEQ ID NO: 1. SEQ ID NO: 1 comprises amino acids 42-486 of ORF0657n COL. SEQ ID NO: 1 also contains an amino terminus methionine to facilitate expression. Polypeptide fragments made up of SEQ ID NO: 2 amino acids 461-609, amino acids 82-486, or amino acids 42-196 were not protective.

Different amino acid and nucleic acid sequences are referred to throughout the application. Table 1 provides a summary of some of the polypeptide sequences indicating the Figure 1 region and additional modifications. Table 2 provides a summary of some of the nucleic acid sequences.

**Table 1**

| SEQ ID NOs: | ORF0657n region | Further modification/additional information |
|---|---|---|
| 1 | ORF0657nI | Amino terminus methionine |
| 2 | Full-length | |
| 3 | ORF0657nH | Amino terminus methionine |
| 4 | ORF0657nH | Amino terminus methionine-glycine |
| 5 | ORF0657nI | Amino terminus methionine-glycine |
| 6 | ORF0657nH | |
| 7 | ORF0657nH | |
| 8 | ORF0657nH | |
| 9 | ORF0657nH | |
| 10 | ORF0657nH | |
| 11 | ORF0657nH | |
| 12 | ORF0657nH | |
| 13 | ORF0657nH | |
| 14 | ORF0657nH | |
| 15 | ORF0657nH | |
| 16 | ORF0657nH | |
| 17 | ORF0657nH | |
| 18 | ORF0657nH | |
| 19 | ORF0657nH | |
| 20 | ORF0657nH | |
| 21 | ORF0657nH | |
| 22 | ORF0657nH | |
| 23 | ORF0657nH | |
| 24 | ORF0657nH | |
| 25 | ORF0657nH | |
| 26 | ORF0657nH | |
| 27 | ORF0657nH | |
| 28 | Full-length | SEQ ID NO: 2 modified to contain a glycine after the amino terminus methionine and a carboxyl His-Tag |
| 42 | ORF0657nI+ | Amino acids 1-481 of SEQ ID NO: 3 |
| 44 | ORF0657nG | Amino terminus methionine plus amino acids 42-645 of SEQ ID NO: 2 |
| 54-63 | ORF0657nH | From different *S. aureus* |
| 106 and 107 | Full-length | From different *S*. *aureus* |

**Table 2**

| SEQ ID NO: | Region | Other information |
|---|---|---|
| 29 | Full-length | Encodes SEQ ID NO: 2 (nucleotides 1-1935) + a carboxyl His-Tag |
| 30 | ORF0657nH | Encodes SEQ ID NO: 4 (nucleotides 1-1710) + a carboxyl His-Tag |
| 31 | Full-length | Encodes SEQ ID NO: 28 without a carboxyl His-Tag and is codon optimized for yeast expression (amino acids 1-646 of SEQ ID NO: 28) |
| 32 | ORF0657nH | Encodes SEQ ID NO: 3 and is codon optimized for yeast expression |
| 33 | ORF0657nI | Encodes SEQ ID NO: 1 and is codon optimized for yeast expression |
| 34 | ORF0657nH | Encodes SEQ ID NO: 7 containing an amino terminus methionine and is codon optimized for yeast expression |
| 35 | ORF0657nH | Encodes SEQ ID NO: 7 containing an amino terminus methionine and is codon optimized for yeast expression . |
| 36 | ORF0657nH | Encodes SEQ ID NO: 7 containing an amino terminus methionine and is codon optimized for yeast expression |
| 37 | ORF0657nH | Encodes SEQ ID NO: 7 containing an amino terminus methionine and is codon optimized for yeast expressions |
| 38 | ORF0657nH | Encodes SEQ ID NO: 7 containing an amino terminus methionine and is codon optimized for yeast expression |
| 39 | ORF0657nH | Encodes SEQ ID NO: 7 containing an amino terminus methionine and is codon optimized for yeast expression |
| 40 | ORF0657nH | Encodes SEQ ID NO: 7 containing an amino terminus methionine and is codon optimized for yeast expression |
| 41 | ORF0657nH | Encodes SEQ ID NO: 7 containing an amino terminus methionine and is codon optimized for yeast expression |
| 43 | ORF0657nI⁺ | Encodes SEQ ID NO: 42 and is codon optimized for yeast expression |
| 45 | ORF0657nG | Encodes SEQ ID NO: 44 containing an amino terminus methionine and is codon optimized for yeast expression |
| 46 | ORF0657nH | Encodes SEQ ID NO: 17 containing an amino terminus methionine and is codon optimized for yeast expression |
| 47 | ORF0657nI⁺ | Encodes the SEQ ID NO: 17 I⁺ region, is codon optimized for yeast expression, and encodes a methionine initiation codon |
| 48 | ORF0657nI | Encodes the SEQ ID NO: 17 I region, is codon optimized for yeast expression, and encodes a methionine initiation codon |
| 49 | Full-length | Encodes for full length ORF0657n containing SEQ ID NO: 17 (SEQ ID NO: 106) modified to contain a glycine after the amino terminus methionine and is codon optimized for yeast expression |
| 50 | ORF0657nH | Encodes SEQ ID NO: 20, is codon optimized for yeast expression, and encodes a methionine initiation codon |
| 51 | ORF0657nI⁺ | Encodes SEQ ID NO: 20 I⁺ region, is codon optimized for yeast expression, and encodes a methionine initiation codon |
| 52 | ORF0657nI | Encodes the SEQ ID NO: 20 I region, is codon optimized for yeast expression, and encodes a methionine initiation codon |
| 53 | Full length | Encodes for full length ORF0657n containing SEQ ID NO: 20 (SEQ ID NO: 107) modified to contain a glycine after the amino terminus methionine and is codon optimized for yeast expression |

### SEQ ID NO: 1 Related Polypeptides

A polypeptide region structurally related to SEQ ID NO: 1 contains an amino acid identity of at least 90% to SEQ ID NO: 1. Polypeptides containing a region structurally related to SEQ ID NO: 1 can be designed based on the guidance provided herein to obtain polypeptides protective against *S*. *aureus.*

Using SEQ ID NO: 1 as a frame of reference, alterations can be made taking into account the amino acid sequence of different naturally occurring ORF0657n polypeptides and known properties of amino acids. Alterations include one or more amino acid additions, deletions, and/or substitutions. The overall effect of different alterations can be evaluated using techniques described herein to confirm the ability of a particular polypeptide to provide protective immunity.

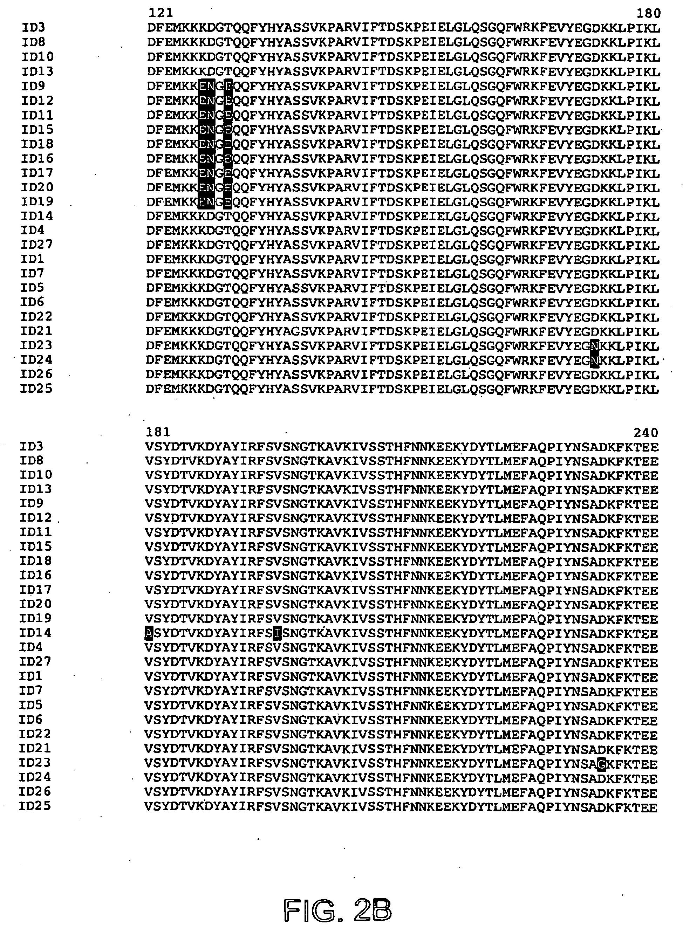

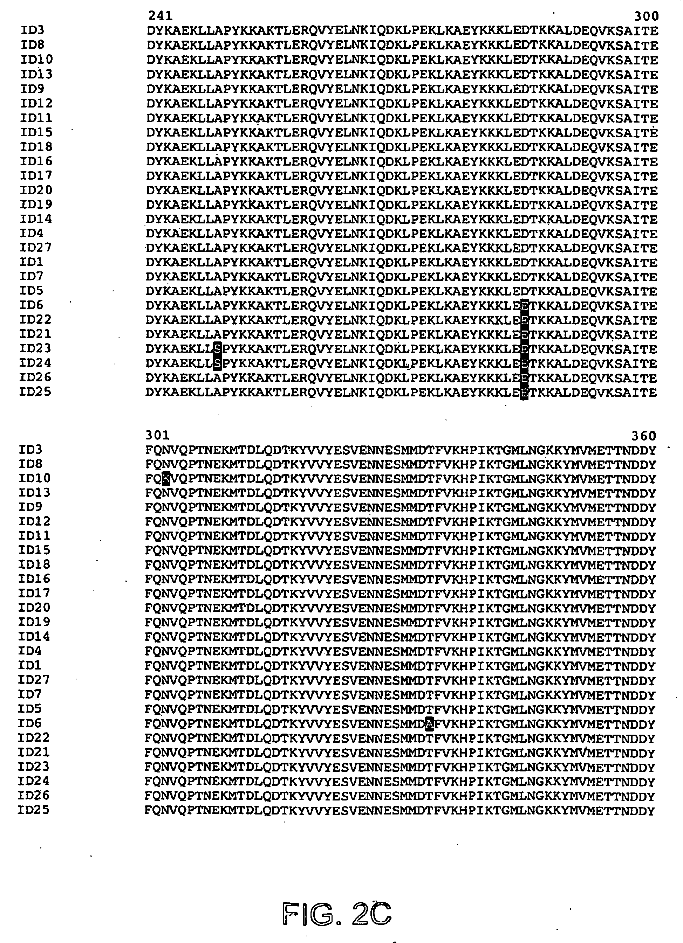

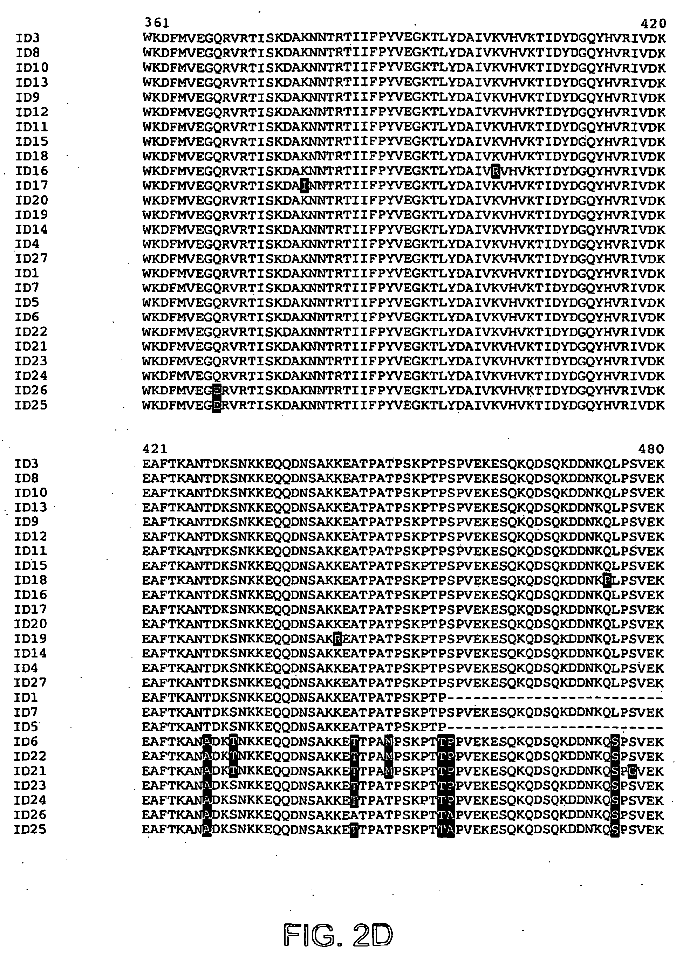

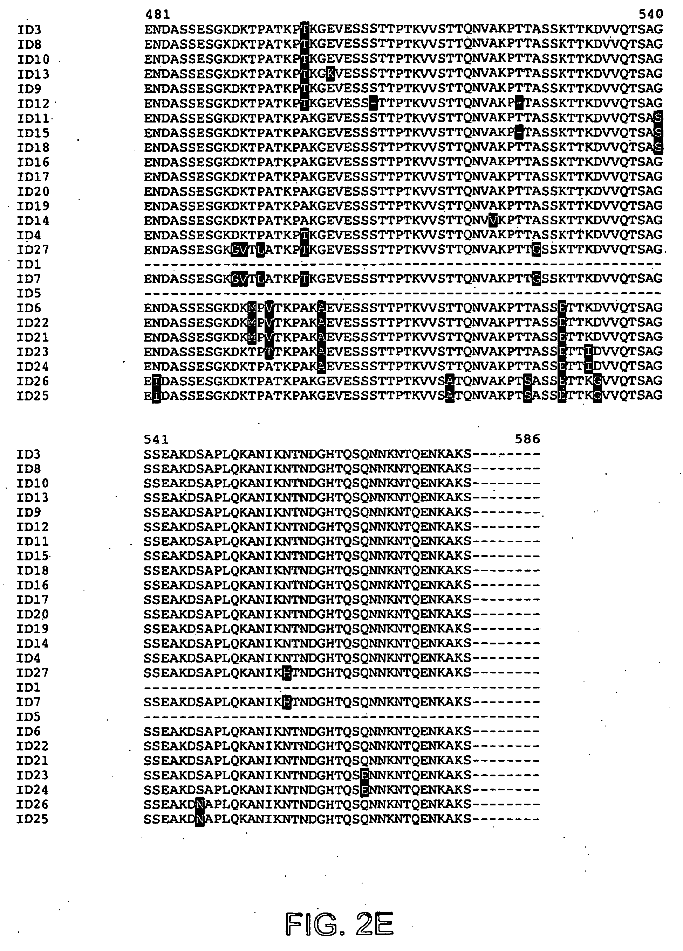

ORF0657n was found to be well conserved across a collection of pathologically and taxonomically diverse *S. aureus* clinical isolates. (See Example 5 *infra.*) Figure 2 provides an amino acid sequence comparison for different sequences containing a SEQ ID NO: 1. The illustrated sequence comparison is for the ORF0657nH region. The ORF0657nH region includes the smaller protective ORF0657nI region.

Figure 2 provides a sequence comparison of SEQ ID NO: 1 and 3-27. The comparison illustrates amino acid differences between *S. aureus* clinical isolates that can be used to guide the design of potential alterations to *S. aureus* related polypeptides such as SEQ ID NOs: 1 and 3. In addition, alterations can be made taking into account known properties of amino acids. SEQ ID NOs: 1, 3-6 and 8-26 illustrate naturally occurring sequences that start at position number 3, position numbers 1 and 2 illustrate the addition of methionine or methionine-glycine to the amino terminus to some sequences. SEQ ID NOs: 11-26 were obtained from different clinical isolates. SEQ ID NOs: 7 and 27 are variants of SEQ ID NO: 4 ORF0657nH region that contains five amino acid substitutions in a region outside the SEQ ID NO: 1 core region.

Additional ORF0657n sequences can be used in the sequence comparison to help guide alterations. Examples of additional *S. aureus* ORF0657nH region sequences are provided by SEQ ID NOs: 54-63, the full length sequence for SEQ ID NOs: 17 and 20 are provided by SEQ ID NOs: 106 and 107.

Generally, in substituting different amino acids to retain activity it is preferable to exchange amino acids having similar properties. Factors that can be taken into account for an amino acid substitution include amino acid size, charge, polarity, and hydrophobicity. The effect of different amino acid R-groups on amino acid properties are well known in the art. (See, for example, Ausubel, Current Protocols in Molecular Biology, John Wiley, 1987-2002, Appendix 1C.)

In exchanging amino acids to maintain activity, the replacement amino acid should have one or more similar properties such as approximately the same charge and/or size and/or polarity and/or hydrophobicity. For example, substituting valine for leucine, arginine for lysine, and asparagine for glutamine are good candidates for not causing a change in polypeptide function.

Alterations to achieve a particular purpose include those designed to facilitate production or efficacy of the polypeptide; or cloning of the encoded nucleic acid. Polypeptide production can be facilitated through the use of an initiation codon (*e.g*., coding for methionine) suitable for recombinant expression. The methionine may be later removed during cellular processing. Cloning can be facilitated by, for example, the introduction of restriction sites which can be accompanied by amino acid additions or changes.

Efficacy of a polypeptide to induce an immune response can be enhanced through epitope enhancement. Epitope enhancement can be performed using different techniques such as those involving alteration of anchor residues to improve peptide affinity for MHC molecules and those increasing affinity of the peptide-MHC complex for a T-cell receptor. (Berzofsky et al., Nature Review 1:209-219, 2001.)

In different embodiments, with respect to the SEQ ID NO: 1 related polypeptide region, the region is at least 90%, at least 94%, or at least 99% identical to SEQ ID NO: 1; differs from SEQ ID NO: 1 by 0, 1, 2, 3, 4, 5, 6, 7, 8,9, 10, 11, 12, 13, 14, 15, 16, 17, 18, 19, 20, 21, 22, 23, 24, or 25 alterations, or up to 50 alterations or consists of an OPR0657nI related region selected from the group consisting of:
amino acids 1-442 of SEQ ID NOs: 11, 15, 16, 18, or 54;
amino acids 1-443 of SEQ ID NO: 63
amino acids 1-444 of SEQ ID NOs: 57 or 59;
amino acids 1-445 of SEQ ID NOs: 7, 8, 9, 10, 12, 13, 14 17, 19, 20, 55, 56 or 58;
amino acids 1-446 of SEQ ID NOs: 23 or 24,
amino acids 1-446 or 2-446 of SEQ ID NOs: 1 or 3;
amino acids 1-447 of SEQ ID NOs: 25,or 26; or
amino acids 1-447, 2-447, or 3-4,47 of SEQ ID NOs: 4, 5, or 27;
amino acids 1-449 of SEQ ID NOs: 61 or 62;
amino acids 1-453 of SEQ ID NO: 60; and
amino acids 1-454 of SEQ ID NOs: 6, 21, or 22.

Additional amino acids may be presents. The additional amino acids can be at the carboxyl or amino terminus. In different embodiments 1, 2, 3, 5, 6, 7, 8, 9, 10, 11, 12, 13, 14, 15, 16, 17, 18, 19, or 20 additional amino acids are present. In preferred embodiments methionine is present at the amino terminus; or methionine-glycine is present at the amino terminus.

In an embodiment of the present invention, the polypeptide consists of an amino acid sequence at least 90% identical to SEQ ID NO: 42 or a fragment thereof comprising an amino acid sequence structurally related to SEQ ID NO: 1. In different embodiment with respect to SFQ ID NO: 42, the polypeptide is at least 94% or a least 99% identical to SEQ ID NO: 42; differs from SEQ .ID NO: 42 by 0, 1, 2, 3, 4, 5, 6, 7, 8, 9, 10, 11, 12, 13, 14, 15, 16, 17, 18, 19, 20, 21, 22, 23, 24, or 25 alterations, up to 50 alterations, or up to 65 alteration; or consists of SEQ ID NO: 42 or an OFR0657nl⁺ related region selected from the group consisting of:
amino acids1-477 of SEQ ID NOs: 11, 15, 16, 18, or 54;
amino acids 1-478 of SEQ ID NO: 63;
amino acids 1-479 of SEQ ID NOs: 57 or 59;
amino acids 1-480 of SEQ ID NOs: 7, 8, 9, 10, 12, 13, 14, 17, 19, 20, 55, 56, or 58;
amino acids 1-481 of SEQ ID NOs: 23 or 24;
amino acids 1 -481 or 2-481 of SEQ ID NOs: 1 or 3;
amino acids 1-482 of SEQ ID NOs: 25 or 26;
amino acids 1-482, 2-482, or 3-482 of SEQ ID NOs: 4, 5, or 27;
amino acids1-484 of SEQ ID NOs: 61 or 62;
amino acids 1-488 of SEQ ID NO: 60; and
amino aids 1-489 of SEQ ID NOs: 6, 21, or 22;

In mother embodiment of the present inventions, the polypeptide consists of an amino acid sequence least 90% identical to SEQ ID NO: 3 or a fragment thereof comprising an amino acid, sequence structurally related to SEQ ID NO: 1 In different embodiments, with respect to SEQ ID NO: 3, the polypeptide is at least 94%, or at least 99% identical to SEQ ID NO: 3; differs from SEQ ID NO: 3 by 0, 1, 2, 3, 4, 5, 6, 7, 8, 9, 10, 11, 12, 13, 14, 1 5, 16, 17, 18, 19, 20, 21, 22, 23, 24, or 25 alterations, up to 50 alterations, or up to 65 alterations; or consist of an amino acid sequence selected from the group consisting of SEQ ID NOs: 3, 4, 6, 7, 8, 9, 10, 11, 12, 13, 14, 15, 16, 17, 18, 19, 20, 21, 22, 23, 24, 25, 26, 27, 54, 55. 55, 57, 55, 59, 60, 61, 62, and 63.

In an additional embodiment the polypeptide consists of a polypeptide of SEQ ID NO: 2 modified by the insertion of a glycine after the initial methionine, or such a polypeptide without the initial methionine.

In an additional embodiment the polypeptide is a purified polypeptide. A "purified polypeptide" is present in an environment lacking one or more other polypeptides with which it is naturally associated and/or is represented by at least about 10% of the total protein present. In different embodiments, the purified polypeptide represents at least about 50%, at least about 75%, or at least about 95% of the total protein in a sample or preparation.

In an additional embodiment the polypeptide is "substantially purified." A substantially purified polypeptide is present in an environment lacking all, or most, other polypeptides with which the polypeptide is naturally associated. For example a substantially purified *S. aureus* polypeptide is present in an environment lacking all, or most, other *S. aureus* polypeptides. An environment can be, for example a sample or preparation.

Reference to "purified" or "substantially purified" does not require a polypeptide to undergo any purification and may include for example, a chemically synthesized polypeptide that has not been purified.

Polypeptide stability can be enhanced by modifying the polypeptide carboxyl or amino terminus. Examples of possible modification include amino terminus protecting groups such as acetyl, propyl, succinyl, benzyl, benzyloxycarbonyl or *t*-butyloxycarbonyl; and carboxyl terminus protecting groups such as amide, methylamide, and ethylamide.

In an embodiment of the present invention the protective polypeptide is part of an immunogen consisting of the polypeptide and one or more additional regions or moieties covalently joined to the polypeptide at the carboxyl terminus or amino terminus. Each region or moiety should be independently selected from a region or moiety having at least one of the following properties: enhances the immune response, facilitates purification, or facilitates polypeptide stability. Polypeptide stability can be enhanced, for example, using groups such as polyethylene glycol that may be present on the amino or carboxyl terminus.

Polypeptide purification can be enhanced by adding a group to the carboxyl or amino terminus to facilitate purification. Examples of groups that can be used to facilitate purification include polypeptides providing affinity tags. Examples of affinity tags include a six-histidine tag, trpE, glutathione and maltose-binding protein.

The ability of a polypeptide to produce an immune response can be enhanced using groups that generally enhance an immune response. Examples of groups that can be joined to a polypeptide to enhance an immune response against the polypeptide include cytokines such as IL-2. (Buchan et al., 2000. Molecular Immunology 37:545-552.)

### Polypeptide Production

Polypeptides can be produced using standard techniques including those involving chemical synthesis and those involving purification from a cell producing the polypeptide. Techniques for chemical synthesis of polypeptides are well known in the art. (See *e.g*., Vincent, Peptide and Protein Drug Delivery, New York, N.Y., Decker, 1990.) Techniques for polypeptide purification from a cell are illustrated in the Examples provided below. Additional examples of purification techniques are well known in the art: (See for example, Ausubel, Current Protocols in Molecular Biology, John Wiley, 1987-2002.)

Obtaining polypeptides from a cell is facilitated using recombinant nucleic acid techniques to produce the polypeptide. Recombinant nucleic acid techniques for producing a polypeptide involve introducing, or producing, a recombinant gene encoding the polypeptide in a cell and expressing the polypeptide.

A recombinant gene contains nucleic acid encoding a polypeptide along with regulatory elements for polypeptide expression. The recombinant gene can be present in a cellular genome or can be part of an expression vector.

The regulatory elements that may be present as part of a recombinant gene include those naturally associated with the polypeptide encoding sequence and exogenous regulatory elements not naturally associated with the polypeptide encoding sequence. Exogenous regulatory elements such as an exogenous promoter can be useful for expressing a recombinant gene in a particular host or increasing the level of expression. Generally, the regulatory elements that are present in a recombinant gene include a transcriptional promoter, a ribosome binding site, a terminator, and an optionally present operator. A preferred element for processing in eukaryotic cells is a polyadenylation signal.

Expression of a recombinant gene in a cell is facilitated through the use of an expression vector. Preferably, an expression vector in addition to a recombinant gene also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses.

Due to the degeneracy of the genetic code, a large number of different encoding nucleic acid sequences can be used to code for a particular polypeptide. The degeneracy of the genetic code arises because almost all amino acids are encoded by different combinations of nucleotide triplets or "codons". Amino acids are encoded by codons as follows:
A=Ala=Alanine: codons GCA, GCC, GCG, GCU
C=Cys=Cysteine: codons UGC, UGU
D=Asp=Aspartic acid: codons GAC, GAU
E=Glu=Glutamic acid: codons GAA, GAG
F=Phe=Phenylalanine: codons UUC, UUU
G=Gly=Glycine: codons GGA, GGC, GGG, GGU
H=His=Histidine: codons CAC, CAU
I=Ile=Isoleucine: codons AUA, AUC, AUU
K=Lys=Lysine_{:} codons AAA, AAG
L=Leu=Leucine: codons UUA, UUG, CUA, CUC, CUG, CUU
M=Met=Methionine: codon AUG
N=Asn=Asparagine: codons AAC, AAU
P=Pro=Proline: codons CCA, CCC, CCG, CCU
Q=Gln=Glutamine: codons CAA, CAG
R=Arg=Arginine: codons AGA, AGG, CGA, CGC, CGG, CGU
S=Ser-Serine: codons AGC, AGU, UCA, UCC, UCG, UCU
T=Thr=Threonine: codons ACA, ACC, ACG, ACU
V=Val=Valine: codons GUA, GUC, GUG, GUU
W=Trp=Tryptophan: codon UGG
Y=Tyr=Tyrosine: codons UAC, UAU

Suitable cells for recombinant nucleic acid expression of ORF0657n related polypeptides are prokaryotes and eukaryotes. Examples of prokaryotic cells include *E. coli;* members of the *Staphylococcus* genus, such as *S. aureus;* members of the *Lactobacillus* genus, such as *L. plantarum;* members of the *Lactococcus* genus, such as *L. lactis;* and members of the *Bacillus* genus, such as *B. subtilis.* Examples of eukaryotic cells include mammalian cells; insect cells; yeast cells such as members of the *Saccharomyces* genus (*e.g., S. cerevisiae*)*,* members of the *Pichia* genus (*e.g., P. pastoris*)*,* members of the *Hansenula* genus (*e.g., H. polymorpha*)*,* members of the *Kluyveromyces* genus (*e.g., K lactis* or *K. fragilis*) and members of the *Schizosaccharomyces* genus (*e.g., S. pombe*)*.*

Techniques for recombinant gene production, introduction into a cell, and recombinant gene expression are well known in the art. Examples of such techniques are provided in references such as Ausubel, Current Protocols in Molecular Biology, John Wiley, 1987-2002, and Sambrook et al., Molecular Cloning, A Laboratory Manual, 2nd Edition, Cold Spring Harbor Laboratory Press, 1989.

If desired, expression in a particular host can be enhanced through codon optimization. Codon optimization includes use of more preferred codons. Techniques for codon optimization in different hosts are well known in the art.

ORF0657n related polypeptides may contain post translational modifications, for example, N-linked glycosylation, O-linked glycosylation, or acetylation. Reference to "polypeptide" or an "amino acid" sequence of a polypeptide includes polypeptides containing one or more amino acids having a structure of a post-translational modification from a host cell, such as a mammalian, insect or yeast host cell.

Post translational modifications can be produced chemically or by making use of suitable hosts. For example, in *S*. *cerevisiae* the nature of the penultimate amino acid appears to determine whether the N-terminal methionine is removed. Furthermore, the nature of the penultimate amino acid also determines whether the N-terminal amino acid is N^{α}-acetylated (Huang et al., Biochemistry 26:8242-8246, 1987). Another example includes a polypeptide targeted for secretion due to the presence of a secretory leader (*e.g*., signal peptide), where the polypeptide is modified by N-linked or O-linked glycosylation. (Kukuruzinska et al., Ann. Rev. Biochem. 56:915-944, 1987.)

### Yeast Expression

ORF0657n related polypeptides are preferably expressed in yeast using encoding nucleic acid containing codons optimized for yeast expression. Expression in yeast can be performed using a recombinant gene encoding a ORF0657n related polypeptide and regulatory regions for yeast expression. Depending upon the employed expression system produced protein can remain intracellular or can be excreted outside the cell.

Preferably, the promoter for recombinant gene expression is an inducible promoter, such as a promoter from the yeast galactose gene cluster (a GAL promoter such as *GAL1, GAL7, GAL10, MEL1)* or the acid phosphatase *PHO5* promoter or the alcohol dehydrogenase II *ADH2* promoter or the copper-regulated metallothionein CUP1 promoter. Examples of "constitutive" promoters that might be used are *GAP (TDH), PGK* or *TPI* promoters. (Romanos et al., YEAST 8:423-488, 1992.)

The yeast host cell used for recombinant expression can be selected or engineered to facilitate recombinant gene expression. Mutation such as *mnn9, prb1* and/or *pep4* mutations are typically desirable. To enhance expression from GAL promoters, over expression of *GAL4* transcription factor can be achieved (Hopper *et al.,* U.S. Patient 5,068,185).

Codon optimization for a particular host is performed by replacing codons having a low or moderate usage level with codons having a high expression level. The percentage of optimal codons present in an encoding sequence can vary. In different embodiments the number of optimal codons (including codons initially present and codons introduced) is at least 50%, ate least 75%, at least 95%, or 100% of the total number of codons.

Codon optimization can be performed as follows:
1. For a particular codon, compare the wild-type codon frequency to overall codon frequency of use by yeast genes.
2. If the codon is not one of those commonly employed by yeast, replace it with an optimal codon for high expression in yeast cells.
3. Repeat steps (1) and (2) for different codons until achieving the desired level of codon optimization.
4. Inspect the new coding sequence for undesired sequences generated such as unwanted restriction enzyme sites, splice sites, promoters, undesirable palindrome or repeat sequences, transcription terminator sequences, and high frequency of GC bases. Remove undesired sequences using an alternative codon.

Alternative codon usage is provided by Lathe, J. Molec. Biol., 183:1-12, 1985. Codon usage in different yeast hosts is well known in the art. For example, Sharp et al., Yeast 7:657-678, 1991, describes synonymous codon usage in *Saccharomyces cerevisiae.*

Figures 8C-8M provide yeast optimized nucleic acid sequences. Figure 8C provides a yeast optimized nucleic acid sequence (SEQ ID NO: 31) encoding SEQ ID NO: 28 without a carboxyl His-Tag. Figure 8D provides a yeast optimized nucleic acid sequence (SEQ ID NO: 32) encoding SEQ ID NO: 3. Figure 8E provides a yeast optimized nucleic acid (SEQ ID NO: 33) sequence encoding SEQ ID NO: 1. Figures 8F-8M provide yeast optimized nucleic acid sequences (SEQ ID NOs: 34-41) encoding SEQ ID NO: 7 containing a methionine amino terminus. Figures 8N-8U provide yeast optimized nucleic acid sequences (SEQ ID NOs: 46-53) encoding different ORF0657n related polypeptides based on SEQ ID NO: 17 or SEQ ID NO: 20.

Yeast expression can be achieved using optimized sequences, and sequences not optimized for yeast expression (*e.g.,* nucleotides 1-1935 or 124-1458 of SEQ ID NO: 29, or nucleotides 1-1710 of SEQ ID NO: 30). Techniques for yeast expression using optimized and non-optimized sequences are illustrated in the Examples *infra.*

ORF0657n is a surface protein containing a 36-amino acid C-terminal cell wall sorting signal with a conserved "LPXTG" motif. (Schneedwind et al. 1993, EMBO, 12: 4803-4811,1993). Proteins containing a cell wall sorting signal are tethered to the cell wall envelope by a transpeptidation mechanism catalyzed by sortase, a membrane-bound protein (Mazmanian et al, Science 299:906-909, 2001). For tethering, the surface protein must also contain an N-terminal signal peptide for export into the secretory pathway. In the secretory pathway, the signal peptide is removed and the cell wall sorting signal facilitates retention in the secretory pathway. Sortase then cleaves between the threonine and the glycine of the LPXTG motif and catalyzes formation of an amide bond between the carboxyl-group of threonine and the amino-group of peptidoglycan cross-bridges.

Expression in yeast was found to be significantly increased by removing the cell wall sorting sequence. In different embodiments, the polypeptide encoding constructs lacks a functional cell wall sorting encoding sequence, and more preferably, lacks functional cell wall sorting and signal peptide encoding sequences. Corresponding preferred ORF0657n related polypeptides lack a functional a cell wall sorting sequence, or both the cell wall sorting and signal peptide sequences.

In different embodiments, at least substantially all of the cell wall sorting sequence or both the cell wall sorting and signal peptide sequences are not present in the polypeptide or nucleic acid encoding the polypeptide. In different embodiments, protein expression is increased at least about 10 fold, at least about 15 fold, or at least about 20 fold by removing at least substantially all of the cell wall sorting sequence or both the cell wall sorting and signal peptide sequences. More preferably, the encoding construct also contains one or more codons optimized for yeast *(e.g., S. cerevisiae)* expression.

Examples of approximate regions for ORF0657n cell wall sorting and signal peptide sequences can be illustrated with respect to SEQ ID NO: 2. Amino acids 1-42 contains the signal peptide sequence. Amino acids 609-645 contains the cell wall sorting sequence.

### Adjuvants

Adjuvants are substances that can assist an immunogen in producing an immune response. Adjuvants can function by different mechanisms such as one or more of the following: increasing the antigen's biologic or immunologic half-life; improving antigen delivery to antigen-presenting cells; improving antigen processing and presentation by antigen-presenting cells; and inducing production of immunomodulatory cytokines. (Vogel, Clinical Infectious Diseases 30(suppl.3):S266-270, 2000.)

A variety of different types of adjuvants can be employed to assist in the production of an immune response. Examples of particular adjuvants include aluminum hydroxide, aluminum phosphate, or other salts of aluminum, calcium phosphate, DNA CpG motifs, monophosphoryl lipid A, cholera toxin, *E. coli* heat-labile toxin, pertussis toxin, muramyl dipeptide, Freund's incomplete adjuvant, MF59, SAF, immunostimulatory complexes, liposomes, biodegradable microspheres, saponins, nonionic block copolymers, muramyl peptide analogues, polyphosphazene, synthetic polynucleotides, IFN-γ, IL-2, IL-12, and ISCOMS. (Vogel Clinical Infectious Diseases 30(suppl 3):S266-270, 2000, Klein et al., Journal of Pharmaceutical Sciences 89:311-321, 2000, Rimmelzwaan et al., Vaccine 19:1180-1187, 2001, Kersten Vaccine 21:915-920, 2003,O'Hagen Curr. Drug Target Infect. Disord., 1:273-286, 2001.)

### Patients For Inducing Protective Immunity

A "patient" refers to a mammal capable of being infected with *S. aureus.* A patient can be treated prophylactically or therapeutically. Prophylactic treatment provides sufficient protective immunity to reduce the likelihood, or severity, of a *S. aureus* infection. Therapeutic treatment can be performed to reduce the severity of a *S. aureus* infection.

Prophylactic treatment can be performed using a vaccine containing an immunogen described herein. Such treatment is preferably performed on a human. Vaccines can be administered to the general population or to those persons at an increased risk of *S. aureus* infection.

Persons with an increased risk of *S*. *aureus* infection include health care workers; hospital patients; patients with a weakened immune system; patients undergoing surgery; patients receiving foreign body implants, such as a catheter or a vascular device; patients facing therapy leading to a weakened immunity; persons with burn or wound injury; and persons in professions having an increased risk of burn or wound injury. (The Staphylococci in Human Disease, Crossley and Archer (ed.), Churchill Livingstone Inc. 1997.)

Non-human patients that can be infected with *S. aureus* include cows, pigs, sheep, goats, rabbits, horses, dogs, cats and mice. Treatment of non-human patients is useful in protecting pets and livestock, and in evaluating the efficacy of a particular treatment.

### Anamnestic Response

ORF0657n related polypeptides were found to produce a rapid and effective immune response in Rhesus monkeys after a single dose. (See Example 17 *Infra.*) The observed response was consistent with an anamnestic response.

The production of an anamnestic response provides significant advantages such as the ability to provide effective immunity using a single dose and providing the effective immunity in a short period of time. In different embodiments the anamnestic response results in at least a 3-fold, at least a 5-fold, or at least a 6-fold, increase in geometric mean titres over pre-existing titers; and the fold increase is produced within 3, 5, 7, 9, 14, or 21 days.

The ability to quickly generate an effective immune response provides cost savings over multi-dose vaccinations and can be used to vaccinate a patient having an increased risk of *S. aureus* infection. Persons with an increased risk of *S. aureus* infection include health care workers; hospital patients; patients with a weakened immune system; patients undergoing surgery; patients receiving foreign body implants, such as a catheter or a vascular device; patients facing therapy leading to a weakened immunity; persons with burn or wound injury; and persons in professions having an increased risk of burn or wound injury. (The Staphylococci in Human Disease, Crossley and Archer (ed.), Churchill Livingstone Inc. 1997.) In different embodiments a patient is vaccinated immediately or within 3, 5, 7, 9, 14, or 21 days of a medical procedure.

### Combination Vaccines

ORF0657n related polypeptides providing protective immunity can be used alone, or in combination with other immunogens, to induce an immune response. Additional immunogens that may be present include: one or more additional *S. aureus* immunogens, such as those referenced in the Background of the Invention *supra;* one or more immunogens targeting one or more other *Staphylococcus* organisms such as *S*. *epidermidis, S. haemolyticus, S. warneri,* or *S. lugunensis;* and one or more immunogens targeting other infectious organisms.

### Animal Model System

An animal model system was used to evaluate the efficacy of a polypeptide to produce a protective immune response against *S. aureus.* Two obstacles encountered in setting up a protective animal model were: (1) very high challenge dose needed to overcome innate immunity and (2) death rate too fast to detect a protective response. Specifically, mice succumbed to infection within 24 hours after bacterial challenge which did not provide sufficient time for the specific immune responses to resolve the infection. When the dose was lowered, both control and immunized mice survived the infection.

These obstacles were addressed by using a slow kinetics lethality model involving *S. aureus* prepared from cells in stationary phase, appropriately titrated, and administered intravenously. This slow kinetics of death provides sufficient time for the specific immune defense to fight off the bacterial infection (*e.g*., 10 days rather 24 hours).

*S. aureus* cells in stationary phase can be obtained from cells grown on solid medium. They can also be obtained from liquid, however the results with cells grown on solid medium were more reproducible. Cells can conveniently be grown overnight on solid medium. For example, *S. aureus* can be grown from about 18 to 24 hours under conditions where the doubling time is about 20-30 minutes.

*Staphylococcus* can be isolated from solid or liquid medium using standard techniques to maintain *Staphylococcus* potency. Isolated *Staphylococcus* can be stored, for example, at -70°C as a washed high density suspension (> 10⁹ colony forming units (CFU)/mL) in phosphate buffered saline containing glycerol.

The *Staphylococcus* challenge should have a potency providing about 80 to 90% death in an animal model over a period of about 7 to 10 days starting on the first or second day. Titration experiments can be performed using animal models to monitor the potency of the *Staphylococcus* inoculum.The titration experiments can be performed about one to two weeks Prior to an inoculation experiment.

Initial potency for titration experiments can be based on previous experiments. For the animal model strain Becker a suitable *S. aureus* challenge was generally found in the range of 5 x 10⁸ to 8 x 10⁸ CFU/mL.

### Administration

Immunogens can be formulated and administered to a patient using the guidance provided herein along with techniques well known in the art. Guidelines for pharmaceutical administration in general are provided in, for example, Vaccines Eds. Plotkin and Orenstein, W.B. Sanders Company, 1999; Remington's Pharmaceutical Sciences 20th Edition, Ed. Gennaro, Mack Publishing, 2000; and Modern Pharmaceutics 2nd Edition, Eds. Banker and Rhodes, Marcel Dekker,Inc., 1990.

Pharmaceutically acceptable carriers facilitate storage and administration of an immunogen to a patient. Pharmaceutically acceptable carriers may contain different components such as a buffer, sterile water for injection, normal saline or phosphate buffered saline, sucrose, histidine, salts and polysorbate.

Immunogens can be administered by different routes such as subcutaneous, intramuscular, or mucosal. Subcutaneous and intramuscular administration can be performed using, for example, needles or jet-injectors.

Suitable dosing regimens are preferably determined taking into account factors well known in the art including age, weight, sex and medical condition of the patient; the route of administration; the desired effect; and the particular compound employed. The immunogen can be used in multi-dose vaccine formats. It is expected that a dose would consist of the range of 1.0 µg to 1.0 mg total polypeptide. In different embodiments of the present invention the range is 0.01 mg to 1.0 mg and 0.1 mg to 1.0 mg.

The timing of doses depends upon factors well known in the art. After the initial administration, if needed for a particular individual, one or more booster doses may subsequently be administered to maintain or boost antibody titers. An example of a dosing regime would be day 1, 1 month, a third dose at either 4, 6 or 12 months, and additional booster doses at distant times as needed.

### Generation of Antibodies

An ORF0657 related polypeptide able to induce protective immunity can be used to generate antibodies and antibody fragments that bind to the polypeptide or to *S. aureus.* Such antibodies and antibody fragments have different uses including use in polypeptide purification, *S. aureus* identification, or in therapeutic or prophylactic treatment against *S. aureus* infection.

Antibodies can be polyclonal or monoclonal. Techniques for producing and using antibodies are well known in the art. Examples of such techniques are described in Ausubel, Current Protocols in Molecular Biology, John Wiley, 1987-1998, Harlow et al., Antibodies, A Laboratory Manual, Cold Spring Harbor Laboratory, 1988, and Kohler et al., Nature 256:495-497, 1975.

### EXAMPLES

Examples are provided below further illustrating different features of the present invention. The examples also illustrate useful methodology for practicing the invention. These examples do not limit the claimed invention.

### Example 1: Use of ORF0657n Region to Provide Protective Immunity

This example illustrates the ability of full-length ORF0657n region to provide protective immunity.

### ORF0657n Cloning and Expression

PCR primers were designed to amplify the gene encoding ORF0657n starting at the first asparagine residue and ending prior to the stop codon at the terminal asparagine residue. These PCR primers also had additional *Nco*I (forward primer) and *Xho*I (reverse primer) sites to facilitate cloning into the expression vector.

The encoded protein was designed to be expressed from the pET28 vector with the terminal His residues and the stop codon encoded by the vector. In addition, a glycine residue was added to the protein after the methionine initiator. The resulting amplified DNA sequence encodes a carboxyl His-Tag ORF0657n (SEQ ID NO: 28).

PCR amplified sequences were ligated into the pET28 vector (Novagen) using the *Nco*I/*Xho*I sites that had been engineered into the PCR primers and introduced into *E. coli* DH5a (Invitrogen) by heat shock. The transformation mixture was grown overnight on Luria-Bertani (LB) agar plates with 100 µg/mL kanamycin at 37°C. Colonies were selected, grown in LB with 30 µg/mL kanamycin, DNA minipreps made (Promega), and insert integrity determined by restriction digestion and PCR. Four minipreps with correct insert size were sequenced using the primers M13F (SEQ ID NO: 65), M13R (SEQ ID NO: 66), ORF0657nF (SEQ ID NO: 67), and ORF0657nR (SEQ ID NO: 68). A clone was selected containing no DNA changes from the desired sequence. *E. coli* HMS174(DE3) cells (Novagen) were transformed and grown on LB plates containing kanamycin (30 µg/mL); 3 colonies (UnkC-1, UnkC-2 and UnkC-3) were selected for expression testing. Liquid LB (kanamycin) cultures were incubated at 37°C, 250 rpm until the A₆₀₀ was between 0.6 and 1.0 and then induced by the addition of IPTG to a final concentration of 1 mM followed by three hours further incubation. Cultures were harvested by centrifugation at 5000 x g for 5 minutes at 4°C. Cells were resuspended in 500 µL lysis buffer (Bug Buster, with protease inhibitors, Novagen). An equal volume of loading buffer (supplemented with β-mecapto ethanol to 5% final volume) was added prior to heating the samples at 70°C for 5 minutes. Extracts were run on Novex 4-20% Tris-Glycine gels and proteins were visualized (Coomassie Blue stained) and blotted onto nitrocellulose and probed with anti-HIS6 antibodies (Zymed).

### ORF0657n Purification

Direct scale-up of the above small scale procedure into stirred tank fermenters (75 liter scale) with a 50 liter working volume was achieved. Inoculum was cultivated in a 250 mL flask containing 50 mL of Luria-Bertani (LB) medium (plus kanamycin) and inoculated with 1 mL of frozen seed culture and cultivated for 3 hours. One mL of this seed was used to inoculate a 2 liter flask containing 500 mL of LB medium (plus kanamycin) and incubated for 16 hours. A large scale fermenter (75 liter scale) was cultivated with 50 liters of LB medium (plus kanamycin). The fermentation parameters of the fermenter were: pressure = 5 psig, agitation speed = 300 rpms, airflow = 15 liters/minute and temperature = 37°C. Cells were incubated to an optical density (OD) of 0.8 optical density units, at a wavelength of 600 nm, and induced with Isopropyl-β-K-Thiogalactoside (IPTG) at a concentration of 1 mM. Induction time, with IPTG, was three hours. Cells were harvested by lowering the temperature to 15°C, concentrated by passage through a 500KMWCO hollow fiber cartridge, and centrifuged at 9,000 times gravity at 4°C for 20 minutes. Supernatants were decanted and the recombinant *E*. *coli* wet cell pellets were frozen at -70°C.

Recombinant *E*. *coli* cells (19.2 g wet cell weight) were suspended in Lysis Buffer (50 mM Tris-HCl, pH 8.0, 0.1 M NaCl, 2 mM MgCl₂, 10 mM imidazole, 0.1 % Tween^{™} -80, and 6 M guanidine-HCl) at 8 mL per gram of cell wet weight. Protease Inhibitor Cocktail for use with poly-(Histidine)-tagged proteins (Sigma, P8849) was added to the suspension at 0.05 mL per gram of cell paste. Additionally, Lysozyme was added to 1 mg/mL,and Benzonase^{™} (EM Ind.) was added to 1 µL/mL. Cell lysis was accomplished by passing the suspension through a microfluidizer at 14,000 PSI (Microfluidics Model 110S) four times at 4°C. Cell debris was pelleted at 11,000 x g for 30 minutes at 4°C, and the supernatant retained.

Proteins bearing a His-Tag were purified from the supernatant. The supernatant was mixed with 20 mL of Ni⁺-NTA agarose (Qiagen) at 4°C with gentle inversion for 2 hours. The mixture was poured into an open column (1.5 cm x 20 cm) and the non-bound fraction was collected in bulk. The column was washed with Wash Buffer (20 mM Tris-HCl, pH 8.0,0.15 M NaCl, 0.1% Tween^{™}-80). His-Tagged ORF0657n was eluted with a step gradient of 300 mM imidazole, 20 mM Tris-HCl, pH 7.5, 0.15 M NaCl, 0.1% Tween^{™}-80.

Fractions containing His-Tag ORF0657n (SEQ ID NO: 28) were detected by Coomassie stained SDS-PAGE and pooled. Pooled fractions were filtered through a 0.2 micron filter to remove particulate material, and were applied on a size-exclusion column (Sephacryl S-300 26/60 column, Amersham Biosciences) and eluted at 1 mL/min with 10 mM MOPS pH 7.1, 150 mM NaCl. Fractions containing His-Tag ORF0657n were detected by Coomassie stained SDS-PAGE and Western blotting (anti-tetra His Mab, Qiagen). Endotoxin was removed by filtration through a Zeta-Plus™ Biofilter (CUNO). Protein concentration was determined by BCA (Pierce). Purity was determined by densitometry of Coomassie stained gels.

### Example 2: Preparation of S. aureus Challenge

*S. aureus* was grown on Tryptic Soy Agar (TSA) (Becton Dickinson, Sparks, MD) plates at 37°C overnight. The bacteria were washed from the TSA plates by adding 5 mL of PBS onto a plate and gently resuspending the bacteria with a sterile spreader. The bacterial suspension was spun at 6000 rpm for 20 minutes using a Sorvall RC-5B centrifuge (DuPont Instruments). The pellet was resuspended in 16% glycerol and aliquots were stored frozen at -70°C.

Prior to use, inocula were thawed, appropriately diluted and used for infection. Each stock was titrated at least 3 times to determine the dose appropriate for inducing slow kinetics of death in naive mice. The potency of the bacterial inoculum (ability to kill 80 to 90% of the mice) was constantly monitored to assure reproducibility of the model. Ten days before each challenge experiment, a group of 10 control animals (immunized with adjuvant alone) were challenged and monitored.

### Example 3: Protection Studies Using His-Tagged ORF0657n Related Polypeptides

Twenty-five BALB/c mice were immunized with three doses of His-Tag ORF0657n (SEQ ID NO: 28), 20 µg per dose, on aluminum hydroxyphosphate adjuvant (450 µg per dose). Aluminum hydroxyphosphate adjuvant (AHP) is described by Klein et al., Journal of Pharmaceutical Sciences 89, 311-321, 2000. The doses were administered as two 50 µL intramuscular injections on days 0, 7 and 21. The mice were bled on day 28, and their sera were screened by ELISA for reactivity to His-Tag ORF0657n.

On day 35 of the experiment the mice were challenged by intravenous injection of *S. aureus* grown, at a dose (7.3 x 10⁸ CFU/mL) determined in titration experiments to cause death over a period of approximately 2 to 7 days. Survival in this lethal model with slow kinetics of death was evaluated against a control set of mice that had just been sham-immunized with AHP. The mice were monitored over a 14 day period for survival (Figure 3A). At the end of the experiment 11 mice survived the ORF0657n immunized group compared to three surviving in the AHP control group.

Figures 3B and 3C illustrate protection using polypeptides corresponding to the ORF0657nH and ORF0657nI regions. Figure 3B illustrates protection with SEQ ID NO: 4 containing a carboxyl His-Tag. Figure 3C illustrates results with SEQ ID NO: 5 containing a carboxyl His-Tag.

### Example 4: Obtaining ORF0657n Sequences

ORF0657n has been implicated to have a role in *S. aureus* iron acquisition. (Andrade et al., Genome Biology 3(9):47.1-47.5, 2003.) ORF0657n sequences, some of which are from different sources, have been given different designations in different references. (For example, see, Etz et al., PNAS USA, 99:6573-6578, 2002 (LPXTGVI); Baba et al., The Lancet 359:1819-1827, 2002 (MW1011); Kuroda, et al., The Lancet 357, 1225-1240, 2001 (SA0976); Andrade et al., Genome Biology 3(9):47.1-47.5, 2003 (S_aur2); Mazmanian et al., Science 299:906-909, 2003 (isdB); Mazmanian et al., Molecular Microbiology 40:1049-1057, 2001. (sasJ); and Taylor et al., Mol. Microbiol. 43:1603-1614, 2002 (sirH).

A polypeptide sequence corresponding to a ORF0657n protein sequence appears to be provided in different patent publications. (Meinke *et al.,* International Publication Number WO 02/059148, published August 1, 2002, Wang *et al.,* International Publication Number WO 02/077183, published October 3, 2002, Masignani *et al.,* International Publication Number WO 02/094868, published November 28, 2002, Foster *et al.,* International Publication Number WO 02/102829, published December 27, 2002, and Foster *et al.,* International Publication Number WO 03/011899, published February 13, 2003.)

Genomic DNA was obtained from different *S. aureus* clinical isolates. Clinical isolates were added to 3 mL of Difco Tryptic Soy Broth (Becton Dickinson, Sparks, MD) and incubated overnight at 37°C and 150 rpm. The overnight cultures were centrifuged in 1.5 mL Eppendorf tubes at 14,000 rpm for 5 minutes. The broth was decanted and the pellets resuspended in 500 µL re-suspension buffer (25% sucrose, 10 mM Tris pH 7.5). A 5 µL aliquot of a 2 mg/ml lysostaphin (Sigma-Aldrich, St. Louis, MO) solution was added to each resuspended pellet. Suspensions were then incubated at 37°C for 1 hour.

At the end of the incubation period, 250 µL of 2% SDS was added to each tube and vortexed until the viscosity of the solution noticeably decreased. 250 µL phenol-chloroform-isoamyl solution (25:24:1, v/v) (Gibco/Invitrogen Corporation, Grand Island, NY) were added. The mixture was vortexed for 30 seconds and centrifuged for 5 minutes at 14,000 rpm. The top aqueous phase was removed and the precipitation steps were repeated until barely any interface remained. 0.1 volume of 3 M NaOAc, pH 4.8, was added to each tube and mixed. One volume of isopropanol was then added and mixed again. The tubes were left to incubate 5 minutes at room temperature and then centrifuged at 14,000 rpm for 15 minutes. The supernatant was decanted and tubes were allowed to dry upside-down on tissue. The pellets were resuspended in 50 µL sterile H₂O.

The isolated DNA was used as a template for PCR. The gene was amplified using a forward primer (ORF0657nF, SEQ ID NO: 67) and reverse primer (ORF0657nR, SEQ ID NO: 68). PCR products were sequenced using standard Big Dye protocols.

### Example 5: Comparison of ORF0657n From Different S. aureus Isolates

ORF0657n was found to be well conserved across a collection of pathologically and taxonomically diverse *S. aureus* clinical isolates. Table 3 provides a summary of the percent identity among different isolates including clinical isolates.

**Table 3**

| **Strain** | **MLST** | **Source** | **Country** | | **CP** | **ORF0657n % ID** |
|---|---|---|---|---|---|---|
| CL-1 | 1 | Hyper-virulent community blood isolate | UK | MSSA | 8 | 99 |
| MW2 | 1 | Hyper-virulent community blood isolate | USA | MRSA | 8 | 99 |
| CL-2 | 1 | Left forearm wound | USA | MSSA | 8 | 99 |
| CL-3 | 1 | Arm wound | USA | MSSA | 8 | 99 |
| CL-4 | 5 | Left foot | USA | MRSA | 5 | 99 |
| CL-5 | 5 | Wound | USA | MRSA | 5 | 100 |
| Mu 50 | 5 | Pus, Surgical wound infection | Japan | VISA | 5 | 100 |
| N315 | 5 | Pharyngeal smear | Japan | MRSA | 5 | 100 |
| CL-6 | 5 | CDC3 | USA | MRSA/ VISA | 5 | 97 |
| CL-7 | 8 | Blood | Germany | MRSA | 5 | 98 |
| NCTC8325 | 8 | MSSA lab strain | USA | MSSA | 5 | 99 |
| CL-8 | 8 | Right leg wound | USA | MRSA | 5 | 99 |
| CL-90 | 9 | Nares | UK | MSSA | 5 | 97 |
| CL-10 | 9 | Blood | UK | MSSA | 5 | 97 |
| CL-11 | 12 | Blood | NL | MSSA | 8 | 97 |
| CL-12 | 12 | Nares | UK | MSSA | 8 | 97 |
| CL-13 | 15 | Left finger skin | USA | MSSA | 8 | 99 |
| CL-14 | 15 | Pus left anaresle | USA | MSSA | 8 | 99 |
| CL-15 | 15 | Blood | UK | MSSA | 8 | 99 |
| CL-16 | 22 | Blood | Canada | MSSA | 5 | 99 |
| CL-17 | 22 | Barnin MRSA | Germany | MRSA | 5 | 98 |
| CL-18 | 25 | Blood | Canada | MRSA | 5 | 97 |
| CL-19 | 25 | Blood | UK | MSSA | 5 | 97 |
| CL-20 | 25 | Nares | UK | MSSA | 5 | 97 |
| CL-21 | 30 | Source not known mecIV | Sweden | MRSA | 8 | 94 |
| CL-22 | 30 | Source not known mecIV | Germany | MRSA | 8 | 94 |
| CL-23 | 36 | Epidemic MRSA blood | UK | MRSA | 8 | 94 |
| CL-24 | 45 | Left leg stump pus | USA | MRSA | 8 | 95 |
| CL-25 | 45 | Nares | USA | MRSA | 8 | 95 |
| Becker | 45 | Prototype capsule 8 strain | USA | MSSA | 8 | 95 |
| COL | 88 | MRSA lab strain | USA | MRSA | 5 | 100 |
| CL-26 | 97 | Blood | NL | MSSA | 5 | 98 |
| CL-27 | 97 | Blood | Canada | MSSA | 5 | 99 |
| CL-28 | 97 | Nares | UK | MSSA | 5 | 99 |
| CL-29 | 121 | Nares, community disease | UK | MSSA | 8 | 96 |
| CL-30 | 121 | Blood | UK | MSSA | 8 | 96 |

| | | | | | | |
|---|---|---|---|---|---|---|
| CP, capsule type; MLST, Multilocus sequence typing taxonomic groupings; MRSA methicillin resistant *S.aureus;* MSSA, methicillin sensitive *S. aureus* "CL" indicates clinical isolate. "ID" indicates identity. "VISA" indicates vancomycin intermediate *S. aureus.* | | | | | | |

Percent identity (% ID) was determined by aligning the polypeptide sequence with SEQ ID NO: 2 and determining the number of identical amino acids. This number was divided by the total number of amino acids (of SEQ ID NO: 2) and then multiplied by 100 and rounded to the nearest whole number.

### Example 6: Protection Against Different S. aureus Clinical Isolates

The efficacy of ORF0657n as immunogen against different *S. aureus* clinical isolates was evaluated using His-Tag ORF0657n as an immunogen (SEQ ID NO: 28). A subset of taxonomically diverse isolates described in Table 3 were used as challenge inocula. The ORF0657n sequences in these different isolates differed from the employed vaccine OFR0657n sequence.

The challenge strains obtained were prepared using the techniques described in Example 2. Mice were immunized and challenged using the techniques described in Example 3, and monitored for 10 days. The challenge innocula routinely used in the models far exceeds those typically encountered in human infections.

Protection was demonstrated against both methicillin sensitive and resistant strains. The results are shown in Figures 4A-4H.

### Example 7: Codon Optimization of SEO ID NO: 28 for Yeast Expression

The nucleic acid sequence encoding amino acids 1-646 of SEQ ID NO: 28 was codon optimized for expression in yeast. A codon-optimized sequence for amino acids 1-646 of SEQ ID NO: 28 is shown in Figure 8C (SEQ ID NO: 31).

SEQ ID NO: 29 without the carboxyl His-Tag encoding region was used as the starting construct for optimization. The overall codon usage of the encoding sequence prior to optimization was: 28% of the codons (179) were very rare or never used by highly expressed yeast genes and 20% (126) were moderately rare.

Codon optimization of nucleic acid encoding amino acids 1-646 of SEQ ID NO: 28 was performed to replace codons having a low or moderate expression with codons highly expressed in yeast. In addition, a glycine codon was added to the second position. Codon optimization was performed using the software program MacDNAsis Pro V3.0. The employed parameter table used for backtranslating proteins indicates highly expressed *S. cerevisiae* codons. In MacDNAsis Pro, the function used is "Translate > [Protein -->DNA]". The output is entitled "Amino Acid Conversion."

In some cases, there is more than one highly expressed codon for a given amino acid. For example, serine is encoded by either "TCT" or "TCC". In these cases, approximately equal numbers of the two different codons were employed. Table 4 provides a codon table for highly expressed *S. cerevisiae* codons.

**Table 4**

| Amino Acid | Optimal Codon(s) | | Amino Acid | Optimal Codon(s) |
|---|---|---|---|---|
| Phe | TTC | | His | CAC |
| Leu | TTG | | Gln | CAA |
| Ile | ATT, ATC | | Asn | AAC |
| Met | ATG | | Lys | AAG |
| Val | GTT, GTC | | Asp | GAC |
| Ser | TCT, TCC | | Glu | GAA |
| Pro | CCA | | Cys | TGT |
| Thr | ACT, ACC | | Trp | TGG |
| Ala | GCT | | Arg | AGA |
| Tyr | TAC | | Gly | GGT |

All of the codons that were not optimal were changed to codons found in highly expressed yeast genes with the exception of two alanine codons that were changed to codons found in moderately expressed genes (GCG beginning at nucleotides 505 and 1546) of SEQ ID NO: 31.

The overall sequence encoding ORF0657n was prepared by the annealing and extension of 25 oligomers (SEQ ID NOs: 69-93) designed to encode the final desired sequence. The oligomers were 85-110 bp in length. The oligomers were alternating, ORF0657n coding sequence. Each oligomer had a complementary overlap of 25-29 bp with the adjoining oligomer and the duplex had a Tm of 80-84°C. This was calculated manually by assigning a GC base-pair a value of 4°C, and an AT base-pair as 2°C.

Seven separate extension reactions were performed using three or four adjoining overlapping oligomers and sense and antisense PCR primers (23-26) nt in length with duplex Tm=70-72°C. Native Pfu DNA polymerase (STRATAGENE, La Jolla, CA) was used for the PCR reactions in a "touch down" strategy as follows: 95°C, 90 seconds, one cycle; 95°C, 30 seconds, 55°C, 30 seconds, 68°C, 3 minutes for 5 cycles, immediately followed by a second series of reactions; 95°C, 30 seconds, 52°C, 30 seconds, 68°C, 3 minutes for 20-cycles. The reaction was completed by incubation at 68°C for 7 minutes. As a result of these PCR reactions, seven colinear fragments of the gene were created (referred to herein for convenience as 1, 2, 3, 4, 5, 6, and 7).

The fragments were isolated by agarose gel electrophoresis and products of the appropriate size were excised and purified using the GENE CLEAN^{®} II method (QBIOgene, Carlsbad, CA) as recommended by the manufacturer. Colinear fragments 1, 2, and 3, colinear fragments 4 and 5, and colinear fragments 6 and 7 were combined in subsequent PCR reactions with the appropriate primers to yield fragments A, B, and C, respectively. The complete gene for ORF0657n was then assembled by an additional PCR reaction in which fragments A, B, and C were combined using distal sense and antisense primers. The final PCR product was gel-isolated and cloned into pCR^{®}-Blunt II-TOPO^{®} (INVITROGEN, Carlsbad, CA) as recommended by the manufacturer. The DNA sequence of several independent clones was obtained and errors identified.

Errors were corrected on different segments of three independent clones, pUC3, pUC4, and pUC6, either sequentially using the QUIK-CHANGE Site-directed Mutagenesis Kit, or simultaneously, with the QUIK-CHANGE Site-directed Multi Mutagenesis Kit (STRATAGENE, La Jolla, CA) according to the manufacturer's recommendations. The final corrected sequence was obtained by swapping repaired restriction fragments from the three clones. A repaired 1.1-kb *Xmn*I fragment of clone pUC4 was swapped for the corresponding *Xmn*I fragment of pUC3 to construct pUnkC 13. A repaired 456-bp *Acc*I fragment of pUC6 was swapped for the corresponding fragment of pUnkC 13 to construct pUnkCR1 and the DNA sequence was verified.

### Example 8: Construction of Saccharomyces cerevisiae Strains for Recombinant Gene Expression

This example illustrates techniques that can be employed to obtain *Saccharomyces cerevisiae* strains for recombinant gene expression. The creation of strains designated 1260 and 1309 is described below. As the genetic background of a strain can greatly influence the properties of a strain for heterologous protein expression, it was desired to construct yeast strains with differing genetic backgrounds which also contained several desirable genetic markers: (1) *mnn9* mutation to prevent hyperglycosylation of secreted proteins, (2) *prb1* and/or *pep4* protease mutations to reduce problems with proteolysis, and (3) overexpression of the *GAL4* transcription factor in order to increase expression from *GAL* promoters.

### Construction of S. cerevisiae Strain Designated 1260

A starting *S. cerevisiae* strain was constructed in the following manner. The *S. cerevisiae* strain Y379-5D (MATα, *cyh2, nib1, rho⁻,* cir°) (Livingston, Genetics 86:73-84, 1977) was crossed with strain DC04 (MATα, *adel, adeX, leu2-04,* cir°) (Broach et al., Cell 21:501-508, 1980). The resulting diploid strain was sporulated and tetrads were dissected by standard procedures. One of the haploid spores gave rise to the strain 2150-2-3 (MATa, *adel, leu2-04,* cir°). The α-mating type *S. cerevisiae* strain LB-347-1C (MATα, *mnn9)* is *S. cerevisiae* strain X2180-1B (MATα, *SUC2, mal, mel, gal2, CUP1;* ATCC Number 204504) containing a *mnn9* mutation. LB-347-1C was mated with the type strain 2150-2-3 (MATa, *leu2-04, ade1)* by mixing the strain on a YEHD complete media agar plate (Carty et al., J. Ind. Micro 2:117-121, 1987). To select for diploids, the mated strains were replica-plated onto minimal media without leucine and containing 2% sucrose as the sole carbon source. After isolating single colonies, the diploids were sporulated, and asci were dissected by standard techniques. The KHY-107 strain was isolated as a single haploid spore and characterized as *ADE1, leu2* and *mnn9* (by Schiff stain technique). A frozen stock in glycerol was established and stored at -70°C.

KHY-107 (cir⁺) was grown from the -70°C stock and transformed with the yeast vector pC1/1, which contains the yeast *LEU2*-d gene and is related to pJDB219 (Beggs, Nature 275:104-109, 1978), except that the pMB9 DNA is replaced by pBR322 DNA. An isolated transformant was grown on selective liquid medium (lacking leucine and containing 1 M sorbitol), then grown through multiple passages in rich medium (containing 1 M sorbitol) to lose both pC1/1 and 2µ DNA. Colonies that had lost pC1/1 (unable to grow on medium lacking leucine) were checked by DNA-DNA hybridization for the presence of 2µ DNA. An isolated clone, KHY-107(cir°)-1, showing no 2µ DNA, was chosen and established as a frozen stock (-70°C) in glycerol.

KHY-107(cir°)-1 was made *ura3* by gene disruption. A plasmid was constructed containing the *S. cerevisiae URA3* gene disrupted by 2 copies of the Aph3'I gene from Tn903. The 5'-URA3-Aph3'I-URA3-3' cassette was excised from the vector and used to transform KHY-107(cir°)-1. Transformants that had integrated the disrupted ura3 cassette were selected on 5-fluoro-orotic acid plates (Kaiser, C. et al., Methods in Yeast Genetics -1994 Edition, Cold Spring Harbor Laboratory Press, (Cold Spring Harbor, New York; 1994) pages 214-215), and subsequently shown to be unable to grow on medium lacking uracil. One isolated clone, KHY-107ura3(PN₂), was chosen and established as a frozen (-70°C) stock in glycerol.

KHY-107ura3(PN₂) was grown on complex medium (containing 1 M sorbitol), then plated onto synthetic medium containing canavanine instead of arginine and canavanine-resistant (can^{R}) mutants were obtained. Spontaneous can^{R} mutants were streaked for isolated colonies on solid synthetic medium containing canavanine instead of arginine. Isolated colonies were shown by standard genetic complementation tests to be *can1.* One isolated *can1* colony, DMY10, was chosen and preserved as a frozen (-70°C) stock in glycerol.

To overexpress the yeast *GAL4* transcription factor, the *GAL10p-GAL4* fusion gene was integrated into the *HIS3* gene of DMY10. The *5'-HIS3-GAL10p-GAL4-URA3-HIS3-3'* cassette was excised from pKHint-C (Schultz et al., Gene 61:123-133,1987) and used to transform DMY10. Transformants that had integrated the cassette were selected on solid synthetic medium lacking uracil, and subsequently shown to be unable to grow on medium lacking histidine. The integration of the cassette only at the *HIS3* locus was verified by Southern hybridization. One isolated integrant, DMY10int-3, was chosen and established as a frozen (-70°C) stock in glycerol. This strain was designated CF52.

Plasmid FP8ΔH bearing the *S. cerevisiae PRBI* gene (Moehle et al., Genetics 115:255-263, 1987) was digested with *Hin*dIII plus *Xho*I and the 3.2-kbp DNA fragment bearing the *PRB1* gene was gel-purified and made blunt-ended by treatment with T4 DNA polymerase. The plasmid pUC18 was digested with *Bam*HI*,* gel-purified and made blunt-ended by treatment with T4 DNA polymerase. The resulting vector fragment was ligated with the above *PRB1* gene fragment to yield the plasmid pUC18-PRB1. Plasmid YEp6 (Struhl et al., Proc. Natl. Acad. Sci., USA 76:1035, 1979) which contains the *HIS3* gene, was digested with *Bam*HI*.* The resulting 1.7-kbp *Bam*HI fragment bearing the functional *HIS3* gene was gel-purified and then made blunt-ended by treatment with T4 DNA polymerase. The pUC18-PRB1 vector was digested with *EcoR*V plus *Nco*I which cut within the *PRB1* coding sequence and removes the protease B active site and flanking sequence. The 5.7- kbp *Eco*RV-*Nco*I fragment bearing the residual 5' and 3'-portions of the *PRB1* coding sequence in pUC18 was gel-purified, made blunt-ended by treatment with T4 DNA polymerase, dephosphorylated with alkaline phosphatase, and ligated with the blunt-ended *HIS3* fragment described above. The resulting plasmid (designated pUC18-prb1::HIS3, stock #1245) contains the functional *HIS3* gene in place of the portion of the *PRB1* gene which had been deleted above.

The *PRB1* gene disruption vector (pUC18-prb1::HIS3) was digested with *Sac*I plus *Xba*I to generate a linear *prb1::HIS3* disruption cassette and used for transformation of strain CF52 by the lithium acetate method (Ito et al., J Bacteriol. 153:163, 1983). His⁺ transformants were selected on synthetic agar medium lacking histidine and restreaked on the same medium for clonal isolates. Genomic DNA was prepared from a number of the resulting His⁺ isolates, digested with *Eco*RI*,* and then electrophoresed on 0.8% agarose gels. Southern blot analyses conformed the presence of the desired *prb1Δ::HIS3* gene disruption.

One of the isolates containing the desired *prb1Δ::HIS3* disruption was selected for further use and was designated strain #1260. Frozen stocks in glycerol were prepared for strain #1260 for storage at -70°C. The resulting genotype of strain 1260 is as follows: *MATa, leu2-2,112, mnn9, ura3Δ, can1, his3Δ::GAL10p-GAL4-URA3, prb1Δ::HIS3,* cir°. *Construction of S. cerevisiae Strain Designated 1309*

The *S. cerevisiae* strain BJ1995 (*MATα, leu2, trp1, ura3-52, prb1-*1122*, pep4*-3, *gal*2) has been described previously (Jones, E. W., Tackling the Protease Problem in Saccharomyces cerevisiae, Methods in Enzymology 194 (1991), pages 428-453). A cir° derivative of BJ1995 which lacked the endogenous 2µ DNA plasmid was isolated using the procedure disclosed for the construction of strain 1260. The resulting cir° isolate was designated strain 91 and was preserved as a frozen stock (-70°C) in glycerol.

A derivative of strain 91 was then constructed which overproduces the *GAL4* transcription factor in order to increase transcription from GAL promoters. The plasmid pKHint-C (Schultz et al., Gene 61:123-133, 1987) was digested with *Bam*HI and the resulting *5'-HIS3-GAL10p-GAL4-URA3-HIS3-3'* cassette was used to transform strain 91. Transformants that had integrated the cassette were selected on solid synthetic medium lacking uracil, and subsequently shown to be unable to grow on medium lacking histidine. The desired integration of the cassette at the *HIS3* locus was verified by Southern hybridization using a probe for the yeast *HIS3* gene. One isolated integrant, BJ1995cir°int #22, was chosen and established as a frozen (-70°C) stock in glycerol. This isolate was designated strain 1282.

A derivative of strain 1282 containing a disruption of the *MNN9* gene was then isolated in the following series of steps. In order to disrupt the *MNN9* gene, it was necessary to first clone the *MNN9* gene from *S. cerevisiae* genomic DNA for the purpose of preparing a gene disruption vector. This was accomplished by standard PCR technology. A 5' sense primer and 3' antisense primer for PCR of the full-length *MNN9* coding sequence were designed based on the published sequence for the yeast *MNN9* gene (MacKay *et al.,* European Publication Number EP0314096, International Publication Date May 3, 1989). The following oligodeoxynucleotide primers containing flanking *Hind*III sites (underlined) were used: sense primer (SEQ ID NO: 94): 5'-CTT AAA GCT TAT GTC ACT TTC TCT TGT ATC G-3' antisense primer (SEQ ID NO: 95): 5'-TGA TAA GCT TGC TCA ATG GTT CTC TTC CTC-3' The initiating methionine codon for the *MNN9* gene is highlighted in bold print.

PCR was conducted using genomic DNA from *S*. *cerevisiae* as a template. The resulting 1.2-kbp PCR fragment carrying the *MNN9* gene was digested with *Hind*III, gel-purified, and ligated with *Hind*III-digested, alkaline-phosphatase treated pUC13. The resulting plasmid was designated p1183. The yeast *TRP1* gene was isolated from YRp7 (Struhl et al., Proc. Natl. Acad. Sci. USA 76:1035-1039, 1979) as a 0.85-kb *Eco*RI*-Bgl*II fragment which was made flush-ended and then inserted into the *Pml*I site of p1183. The *Pml*I site is in the middle of the *MNN9* coding sequence, thereby insertion of the *TRP1* gene at that site results in a gene disruption. The resulting plasmid pUC13-mnn9::TRP1 (pH11885-239-1) was then digested with *Hpa*I plus *Eco*RI and the 5'*-mnn9-TRP1-mnn9*-3' cassette was used for transformation of strain 1282 by the lithium acetate method. (Ito et al., J Bacteriol. 153:163, 1983.)

Trp⁺ transformants were selected on synthetic medium agar plates lacking tryptophan and restreaked on the same plates for single colony isolates. Genomic DNA was prepared from a number of the resulting Trp⁺ isolates, digested with *Hind*III, and then electrophoresed on 0.8% agarose gels. Southern blot analyses confirmed the presence of the desired *mnn9::TRP1* gene disruption. One of these isolates containing the desired *mnn9::TRP1* disruption was selected for further use and was designated strain 1309. Frozen stocks in glycerol were prepared for strain 1309 for storage at -70°C. The genotype for strain 1309 is as follows: *MATα, leu2, mnn9::TRP1, trp1, ura3*-52, *his3Δ::GAL10p-GAL4-URA3, prb1-*1122*, pep4*-3, *gal2,* cir°.

### Example 9: Expression of Full-length ORF0657n Region

Full-length ORF0657n was expressed in *E*. *coli* (SEQ ID NO: 28; His-Tag ORF0657n) and *S. cerevisiae* (amino acids 1-646 of SEQ ID NO: 28) and the expression products were compared.

### S. cerevisiae Expression

DNA encoding the ORF0657n protein with codons optimized for yeast expression was amplified from the final corrected clone, pUnkCR1 (Example 7), using the following sense and antisense primers, respectively, UnkCY-F (SEQ ID NO; 96), 5'AAC CGG TTT GGA TCC CAC AAA ACA AAA TGG GTA ACA AGC AAC AAA AGG AAT TC3' and UnkCY-R (SEQ ID NO: 97), 5'AAC CGG TTT GGA TCC TTA GTT CTT TCT CTT TCT TGG CAA GAC3' containing flanking *Bam*HI restriction sites. The sense primer contains a 5' untranslated sequence and ATG codon and the antisense primer contains TAA as the stop codon. The resulting 1.9-kb product was gel-isolated and cloned into the TOPO TA vector pCR2.1 (INVITROGEN CORPORATION, Carlsbad, CA) according to the manufacturer to construct plasmid UnkC-B1. The DNA sequence of the insert was subsequently verified. The *Bam*HI fragment was gel-isolated and subcloned in the proper orientation into a yeast vector (pGAL110, Fig. 5A) to construct pRUnkC-pGAL110. The sequence of the insert was verified by DNA sequencing.

Plasmid DNA from the pRUnkC-pGAL110 was used to transform *S. cerevisiae* strains containing a *leu2* mutation to leucine prototrophy (Leu⁺) by using a spheroplast transformation protocol (Hinnen et al., Proc. Natl. Acad. Sci. U S A, 75:1929-33, 1978). The construction of strains 1260 and 1309 is provided in Example 8, *supra.*

Transformants were selected on synthetic agar medium lacking leucine and containing 1 M sorbitol. The top and bottom synthetic agar medium lacking leucine and containing 1 M sorbitol were obtained from REMEL, Lenexa, KS (cat #09459 and 92155, respectively). Clonal Leu⁺ isolates were obtained by serial growth on SD minus leucine plates (KD MEDICAL, Columbia MD).

For screening multiple transformants, 5.0 mL production cultures were grown in culture tubes rotated slowly at 30°C. Subsequently, production was confirmed for selected transformants in 25-ml cultures in a 125-mL flask. For both cases, five-mL seed cultures were grown at 30°C in 5X minus leucine medium containing 4.0% glucose and 0.1 M sorbitol for 18-24 hours to OD₆₀₀ of 1.5-3.0/mL. 5X minus leucine medium contains the following components per liter: Yeast Nitrogen Base without added amino acids or ammonium sulfate, 8.5 g; adenine, 0.40 g; L-tyrosine, 0.25 g; uracil, 0.20 g, succinic acid, 10.0 g, ammonium sulfate, 5.0 g and 50 ml of Leucine-Minus Solution #3. Leucine-Minus Solution #3 contains per liter of distilled water, L-arginine, 2 g; L-histidine, 1.0 g; L-isoleucine, 6 g; L-lysine 4.0 g; L-methionine, 1.0 g; L-phenylalanine, 6.0 g; L-tryptophan, 4.0 g. The pH of the medium was adjusted to 5.3 with 50% sodium hydroxide.

For production in tubes, a 0.3 mL aliquot of the seed culture was transferred to either 5.0 mL of 5X minus leucine medium containing 2% glucose, 4% galactose or YEHDG medium for 72 hours to a final OD₆₀₀ of 5-16.0/mL. YEHDG medium contains per liter: L-Hy-Soy peptone-Sheffield, 10 g; Yeast extract, 20 g; L-dextrose, 16 g; D(+) galactose, 40 g. For production in flasks, a 1.5-mL aliquot of the seed culture was transferred to 25-mL of medium and grown as described above with shaking at 220 rpm.

After harvesting 10 OD units per sample, the cell pellets were broken with glass beads in 0.3 mL lysis buffer (0.1 M sodium phosphate buffer, pH 7.2, 0.5 M NaCl, 2 mM PMSF). The lysate was recovered by centrifugation, the unbroken cells/beads were washed with 0.3 mL of lysate buffer and the clarified supernatants were combined. Protein concentration was determined by the BIO-RAD Protein Assay Dye Reagent system (BIO-RAD, Hercules, CA) according to the manufacturer's instructions. The cell lysates were analyzed for the expression of ORF0657n by immunoblot analysis after electrophoresis on 4-20% gradient Tris-Glycine gels (INVITROGEN, Carlsbad, CA) in 1X Tris-glycine SDS buffer (BIO-RAD) under reducing and denaturing conditions. The samples contained 20 µg of total cellular protein. The gels were electroblotted onto 0.45 micron nitrocellulose membrane filters (Optitran from Schleicher and Schuell, Keene, NH). To estimate protein size, prestained standards between 6.4 and 203 kDa (Broad Range Prestained SDS-PAGE Standard, BIO-RAD) were run in parallel with the lysates.

### E. coli Standard

His-Tag ORF0657n (SEQ ID NO: 28) purified from the *E*. *coli* producing culture (*E. coli* host HMS 174(DE3), NOVAGEN, Madison, WI), induced to produce ORF0657n, and a cell lysate were employed as standards. *E. coli* was transformed with an expression vector expressing His-Tag ORF0657n. The *E*. *coli* culture was grown overnight in LB broth containing 30 µg/mL kanamycin at 37°C. The next day, 60 µL of overnight culture was used to inoculate 6.0 mL LB plus 30 µg per mL kanamycin. The culture was grown at 37°C for approximately 3 hours until the OD₆₀₀ was between 0.4-1.0. Expression was induced with 1 mM IPTG for 2 hours at 37°C. The cells were harvested and the cell pellet was stored at -80°C.

The *E. coli* lysate was prepared using Bugbuster Protein Extraction Reagent (NOVAGEN, Madison, WI) following the manufacturer's protocol. Proteins were immunodetected by Western blot using a murine monoclonal antibody ("designated "2H2B8") to ORF0657n as primary antibody and goat anti-mouse IgG (H+L) horseradish peroxidase-linked whole antibody (ZYMED LABORATORIES, South San Francisco, CA) as the secondary antibody. Mab 2H2B8 was generated by immunization of mice with purified *E. coli* produced full-lengh ORF0657n. Mab 2H2B8 was selected by ELISA and was shown to be specific for ORF0657n. The filters were processed using the BIO-RAD HRP Conjugate Substrate Kit.

### Expression Products From E. coli and S. cerevisiae

From the initial screening of yeast transformants, one transformant each of yeast strains 1260 and 1309 were chosen as the best producers of full-lengh ORF0657n region (SEQ ID NO: 28 without the carboxyl His-Tag). An example of their production of full-length ORF0657n region after a 72 hour fermentation in YEHDG medium compared to ORF0657n region (SEQ ID) NO: 28) production in *E. coli* is shown in Figures 6A and 6B.

The major protein defected by Western blot analysis with mAb 2H2B8 was ∼105-110-kDa as shown in Fig. 6A. (strain 1260) and 6B (strain 1309): lanes 5 and 6. The ∼105-110-kDa protein corresponded in size to the largest protein detected in the sample of purified recombinant ORF0657n produced in *E. coli* (SEQ ID NO: 28; lane 2) or an extract of the induced *E. coli* ORF0657n-producing culture (the latter not included in gels shown in Fig. 6). The 105-110 -kDa *E. coli* and *S. cerevisiae* produced proteins are of higher molecular weight than the predicted size in our gel-electrophoresis system. It should be noted that the predominant species detected in the *E. coli* controls was smaller, ∼95 kDa and comigrated with a minor amount of protein produced in yeast. The ∼105-110 kDa protein is believed to correspond to the full-length ORF0657n that was expressed with the *E. coli* secretory leader in both *E. coli* and in *S. cerevisiae*, and the 95-kDa proteins is thought to be a degradation product. No detection was observed with an extract of a control transformant obtained with vector pGAL110 alone (lanes 3 and 4).

An estimate of the amount of ORF0637n region (SEQ ID NO: 28 without the carboxyl His-Tag) produced by the transformants of 1260 and 1309 was ∼10 micrograms ORF0657n/mL YEHDG medium with ORF0657n region comprising -2% of the total protein as determined by semi-quantitative Western blot with 100 ng of purified ORF0657n (SEQ ID NO: 28) as standard. Both the amounts expressed and % ORF0657n region (SEQ ID NO: 28 without the carboxyl His-Tag) of the total protein were greater at 72 hours compared to 48 hours in borh strains (data not presented). The titer of ORF0657n region (SEQ ID NO: 28 without the carboxyl His-Tag) produced by transformants of 1260 in 5X leucine medium containing 2% glucose, 4% galactose, was ∼8.0 µg/mL and the % total protein was 2.0.

### Example 10: Expression of Secreted Codon-Optimized Sequence Encoding an ORF0657nH Region

Vector pKH4-3B (Carty et al., Biotechnol. Lett. 12:879-884, 1990) contains the yeast alpha factor (MFα1) pre-pro secretory leader. Fusion of a desired protein to this leader results in a translation product that is cleaved first by signal peptidase of *S. cerevisiae*. Subsequently the Kex2 protease cleavers after the "Lys Arg residue" in the secretory leader and the mature protein is released. The *S. cerevisae* inducible *GAL10* promoter was used to drive protein expression.

The vector pKH4-3B was used to express a codon optimized gene encoding an altered SEQ ID NO: 3. SEQ ID NO: 3 was altered by removing the amino terminus methionine and adding an amino terminus leader sequence. The codon optimized SEQ ID NO: 3 encoding region is provided by nucleotides 4-1710 of SEQ ID NO: 32, and the leader encoding sequence is provided by vector pKH4-3B prepared as described below.

An in frame fusion of the alpha factor pre-pro secretory leader of pKH4-3B to ORF0657nH region of SEQ ID NO: 3 was made. This was accomplished by digestion of vector pKH4-3B with *Sph*I followed by treatment with T4 DNA polymerase to remove the *Sph*I-overhang, creating a blunt end at the 5' end with the appropriate codon of the leader. Subsequestly, the vector was digested with *Bam*HI to create a 3' cloning site. This construct requires a PCR product with a 5' blunt end corresponding to the second codon of rebuilt ORF0657nH region (SEQ ID NO: 32), and a 3' end with a stop codon and *Bam*HI restriction site.

DNA encoding the ORF0657nH region with codons optimized for yeast expression was amplified from pUnkCR 1 (Example 7) using the following sense and antisense primers, respectively, UCKHS2 (SEQ ID NO: 98), 5'GCTGAAGAAACTGGTGGTACCAAC3' and UCKHA2, (SEQ ID NO: 99) 5'GTCACGGATCCTTAAGACTTAGCCTTGTTTTCTTGAGTGTTC3', The 5' end of the sense primer, GCT, encodes alanine, the predicted N-terminus of the ORF0657nH region. The antisense primer contains a TAA stop codon and a *Bam*HI site (underlined). The resulting 1.7-kb PCR product was gel isolated and cloned into pH4-3B (prepared as described above) to construct pUS38. The entire DNA sequence of the insert and partial sequence of the vector was verified, and showed that desired fusion to the secretory was made. Appropriate cleavage by Kex2p of the initial protein expressed from this construct would result in a protein corresponding to SEQ ID NO: 3 lacking the N-terminal methionine,

Plasmid pUS38 was used to transform *S. cerevisiae* strains 1260 and 1309 to leucine protorophy (Leu⁺) by using a spheroplast transformation protocol (Hinnen et al., Proc. Natl. Acad. Sci. U.S.A. 75:1939-33, 1978), Transformants were selected synthetic agar medium lacking leucine and containing 1 M sorbitol. The top and bottom synthetic agar medium lacking leucine and containing 1 M sorbitol were obtained from REMEL, Lenexa, KS (cat #09459 and 92155, respectively). Clonal Leu⁺ isolates were obtained by serial growth on SD minus leucine plates (KD MEDICAL, Columbia MD).

For screening multiple transformants, five-mL seed cultures were grown at 30°C in 5X minus leucine medium containing 4.0% glucose and 0.1 M sorbitol medium for 18-24 hours to OD₆₀₀ of 1.5-3.0/mL. 5X minus leucine medium contains the following components per liter: Yeast Nitrogen Base w/o amino acids or ammonium sulfate, 8.5 g; adenine, 0.40 g; L-tyrosine, 0.25 g; uracil, 0.20 g, succinic acid, 10.0 g, ammonium sulfate, 5.0 g and 50 mL of Leucine-Minus Solution #3. Leucine-Minus Solution #3 contains per liter of distilled water, L-arginine, 2 g; L-histidine, 1.0 g; L-isoleucine, 6 g; L-lysine 4.0 g; L-methionine, 1.0 g; L-phenylalanine, 6.0 g; L-tryptophan, 4.0 g. The pH of the medium was adjusted to 5.3 with 50% sodium hydroxide. A 0.3 mL aliquot was transferred to either 5.0 mL of 5X minus leucine medium containing 2% glucose plus 4% galactose or YEHDG medium for 72 hours to a final OD600 of 10-20.0/mL. YEHDG medium contains per liter: L-Hy-Soy peptone-Sheffield, 10 g; Yeast extract, 20 g; L-dextrose, 16 g; D(+) galactose, 40 g.

The fermentations were harvested by removing the yeast cells and analyzing the supernatants directly for the expression of ORF0657nH region (SEQ ID NO:3) by Western blot analysis or by Coomassie staining of SDS-PAGE gels. For immunoblot analysis, 10 to 25 microliter samples were subjected to electrophoresis on 4-15% gradient Tris-HCl gels (BIORAD, Hercules, CA) in 1X Tris glycine SDS buffer (BIORAD, Hercules, CA) under reducing and denaturing conditions. The gels were electroblotted onto 0.2 micron PVDF membrane filters. Proteins were immunodetected with the monoclonal antibody 2H2B8 as primary antibody and goat anti-mouse IgG (H+L) horseradish peroxidase-linked whole antibody (ZYMED LABORATORIES, South San Francisco, CA) as the secondary antibody. Mab 2H2B8 is described in Example 9. The filters were processed using the WESTERN LIGHTNING^{™} Chemiluminesence Reagent Plus kit (PERKIN ELMER, Wellesley, MA).

For analysis of expression of recombinant yeast ORF0657nH region (SEQ ID NO: 3) by Coomassie staining of SDS-PAGE gels, samples were subjected to electrophoresis on 4-15% gradient Tris-HCl gels (BIO-RAD) in 1X Tris glycine SDS buffer (BIO-RAD) under reducing and denaturing conditions. The gels were stained with Bio-Safe Coomassie, a Coomassie G250 stain according to the manufacturer's protocol (BIO-RAD).

As a qualitative standard, a cell lysate from the *E. coli* culture producing ORF0657nH (SEQ ID NO: 4 with a carboxyl His-Tag) was employed. The predicted amino acid sequence of *E. coli*-expressed ORF0657nH region is identical to the predicted amino acid sequence of *S. cerevisiae* internally-expressed ORF0657nH region (SEQ ID NO: 3) except for the presence of glycine following the N-terminal methionine in the *E. coli* construct.

To produce ORF0657nH region in *E. coli,* the producing culture was grown overnight in LB broth containing 50 µg/mL kanamycin at 37°C. A pET28 encoding SEQ ID NO: 4 with a carboxyl His-Tag was used to obtain expression of protein. The next day, 500 µL of overnight culture was used to inoculate 5.0 mL LB broth plus 50 µg per mL kanamycin. The culture was grown at 37°C for approximately 3 hours to an OD₆₀₀ of 0.6. Expression was induced with 1 mM IPTG for 3.5 hours at 37°C. The cells were harvested and the cell pellet was stored at -80°C. The *E. coli* lysate was prepared using Bugbuster Protein Extraction Reagent (NOVAGEN, Madison, WI) following the manufacturer's protocol.

Protein from a cell-lysate of *S. cerevisiae* transformant 1-1, expressing internal ORF0657nH region (SEQ ID NO: 3) described and prepared as indicated in Example 11 *infra,* was also included as a standard. To estimate protein size, prestained standards between 10 and 250 kDa (BIO-RAD) were run in parallel with the samples on SDS-PAGE gels for both Coomassie staining and Western evaluation.

Production of the desired species was obtained in 5X minus leucine medium containing 2% glucose plus 4% galactose. Transformants containing pUS38 of both strains 1260 and 1309 secreted an ∼80-kDa protein that comigrated very closely with yeast internally expressed ORF0657nH region (from nucleic acid encoding SEQ ID NO: 3) and *E. coli* expressed ORF0657nH (from nucleic acid encoding SEQ ID NO: 4 with a carboxyl His-Tag) that was detected by Western and Coomassie staining. In a typical experiment, 500 ng of these control lysates and 25 microliters of medium supernatant were subjected to electrophoresis. The detection was specific; the 80-kDa protein was not detected in a supernatant of a transformant containing vector alone by either Western blot or Coomassie staining. The secreted -80-kDa protein could correspond to mature non-glycosylated ORF0657nH region (SEQ ID NO: 3) or alternatively, it could contain a few glycosyl residues. A higher molecular weight species in the supernatants was detected by the antibody as well as two lower molecular weight proteins, all of which were stained by Coomassie Blue. The higher molecular weight species could contain unprocessed leader and /or could be glycosylated. The low MW species are likely to be degradation products.

An estimate of the 80-kDa species amount secreted by transformants of both strains 1260 and 1309 was -2 µg per mL culture medium. This was determined by comparing the Western signal at 80 kDa to that of the sample of the yeast cell lysate containing ORF0657nH region (SEQ ID NO: 3), suggesting that ORF0657nH region comprises at least 50% of the total protein. The combined titer of the remaining species of ORF0657n region was estimated to be approximately 50 µg per mL culture medium.

### Example 11: Intracellular Expression of ORF0657nH regions (SEO ID NO: 3) in S. cerevisiae

A very high level production of discretely-sized protein was obtained by intracellular expression of SEQ ID NO: 3 in *S. cerevisiae.* Expression was achieved using a yeast optimized encoding nucleic acid sequence.

DNA encoding ORF0657nH region with codons optimized for yeast expression (SEQ ID NO: 32) was amplified from pUnkCR1 (Example 7), using the following sense and antisense primers, respectively, (SEQ ID NO: 100) 5'GGGG GGATCC CACAAAACAAA ATG GCT GAA GAA ACT GGT GG3' and (SEQ ID NO: 101) 5'GGG GGG GGATCC TTA AGA CTT AGC CTT GTT TTC TTG AGT3' with flanking BamHI restriction sites (underlined). The sense primer contains a 5' untranslated leader sequence and ATG codon, and the antisense primer contains TAA as the stop codon.

The resulting 1.7-kb product was gel-isolated and cloned into the TOPO TA vector pCR2.1 (INVITROGEN CORPORATION, Carlsbad, CA), according to the manufacturer's directions, to construct plasmid pCR_iUC-S. The DNA sequence of the insert was subsequently determined from two independent clones, pCR_iUCS2.2 and pCR_iUCS-2.4. A single PCR error was found in each of the inserts, located on different *Hin*dIII fragments of each plasmid. A plasmid with the correct sequence was obtained by swapping the 1.3-kb *Hin*dIII fragment of pCR_iUC-S2.2 with the correct sequence, for the corresponding fragment of pCR_iUC-S2.4.

The 1.3-kb fragment of pCR_iUC-S2.2 and the 4.3-kb fragment of pCR_iUC-S2.4 were gel-isolated and ligated. A clone with the desired fragments was selected, pCR_iUC-S, and the appropriate orientation and nucleotide sequence verified by DNA sequencing. Subsequently, the 1.7-kb *Bam*HI fragment was subcloned into pGAL110 (Fig. 5A) to construct piUCS-S(-).

Plasmid DNA from piUC-S(-) was used to transform *S*. *cerevisiae* strains 1260 and 1309 to leucine prototrophy (Leu⁺) by using a spheroplast transformation protocol. (Hinnen et al., Proc. Natl. Acad. Sci. U S A, 75:1929-33, 1978.) Transformants were selected on synthetic agar medium lacking leucine and containing 1 M sorbitol. The top and bottom synthetic agar medium lacking leucine and containing 1 M sorbitol were obtained from REMEL, Lenexa, KS (cat #09459 and 92155, respectively). Clonal Leu⁺ isolates were obtained by serial growth on SD minus Leu plates (KD MEDICAL, Columbia MD).

Multiple transformants were screened for production of ORF0657nH region using the fermentation conditions described in Example 9. The cell lysates were analyzed for the production of ORF0657nH region by Western blot analysis or by analysis of SDS-PAGE gels stained with Coomassie blue.

For Western blot analysis, samples were subjected to electrophoresis on 4-15% gradient Tris-HCl Criterion gels (BIO-RAD, Hercules, CA) in 1X Tris glycine SDS buffer (BIORAD) under reducing and denaturing conditions. The gels were electroblotted onto 0.2-micron PVDF membrane filters. Proteins were immunodetected by Western blot using a monoclonal antibody to full-length ORF0657n (SEQ ID NO: 28), 2H2B8, as primary antibody and goat anti-mouse IgG (H+L) horseradish peroxidase-linked whole antibody (ZYMED LABORATORIES, South San Francisco, CA) as the secondary antibody. Mab 2H2B8 was described in Example 9. The filters were processed using the WESTERN LIGHTNING^{Tm} Chemiluminesence Reagent Plus kit (PERKIN ELMER, Wellesley, MA).

For analysis of expression of recombinant yeast ORF0657nH (encoded by SEQ ID NO: 32) by Coomassie staining of SDS-PAGE gels, samples were subjected to electrophoresis on 4-15% gradient Tris-HCl Criterion gels (BIO-RAD) in 1X Tris glycine SDS buffer (BIORAD) under reducing and denaturing conditions. The gels were stained with Bio-Safe Coomassie, a Coomassie G250 stain (BIO-RAD) according to the manufacturer's protocol.

The samples of yeast cell-lysate subjected to electrophoresis contained 0.5 and 1.25 µg of total cellular yeast protein, respectively, for Western blot and Coomassie-stain. As a quantitative standard for the Coomassie staining, purified BSA (100X, NEW ENGLAND BIOLABS, Beverly, MA) was used. Purified *E. coli* full-length recombinant ORF0657n region (SEQ ID NO: 28) was used as a quantitative standard for the Westerns. As a qualitative standard, a cell lysate from the *E. coli* culture producing ORF0657nH region (SEQ ID NO: 4 with a carboxyl His-Tag), induced to produce ORF0657nH, was employed for both evaluations.

To produce ORF0657nH region (SEQ ID NO: 4 plus a carboxyl His-Tag) in *E. coli,* the producing culture was grown overnight in LB broth containing 50 µg/mL kanamycin at 37°C. The next day, 500 µL of overnight culture was used to inoculate 5.0 mL LB broth plus 50 µg per mL kanamycin. The culture was grown at 37°C for approximately 3 hours to an OD₆₀₀ of 0.6. Expression was induced with 1 mM IPTG for 3.5 hours at 37°C. The cells were harvested and the cell pellets were store at -80°C. The *E. coli* lysate was prepared using Bugbuster Protein Extraction Reagent (NOVAGEN, Madison, WI) following the manufacturer's protocol. To estimate protein size, prestained standards between 10 and 250 kDa were run in parallel with the lysates (BIO-RAD).

From the initial screening, one transformant of yeast strain 1260, designated 1-1, was chosen as the best producer of ORF0657nH region (SEQ ID NO: 3). An example of the production of ORF0657nH region after a 72 hour fermentation in complex medium YEHDG in shake flasks is shown in Figures 7A and 7B. The major protein detected by either Coomassie staining (A) or by the antibody (B) was -85 kDa (see lanes 5, 6, and 7) and comigrated with *E. coli* ORF0657nH (SEQ ID NO: 4 with a carboxyl His-Tag; lane 2). The MW of both the *E. coli* expressed ORF0657n and the yeast-expressed ORF0657n was larger than the predicted size in our gel electrophoresis system. Detection of the -85-kDa protein was specific; no detection was observed in a cell lysate from a transformant containing vectorpGAL110 alone (lane 3). Significantly more ORF0657nH region (SEQ ID NO: 3) was produced compared to full-length ORF0657n region (SEQ ID NO: 28 without a carboxyl His-Tag), compare lanes 5, 6, and 7 to lane 4. The transformant containing full-length ORF0657n was fermented at the same time as the transformant containing ORF0657nH region(SEQ ID NO: 3) and under the identical conditions. Little evidence of degradation of mature yeast-expressed ORF0657nH region was observed.

To evaluate protein stability, a sample from a cell lysate of transformant 1-1 that had been frozen for several days and subsequently unthawed was included on the gel (Figure 7, lane 7). It can be seen that the integrity and amount of this sample is similar to the fresh samples (lanes 5 and 6). The lack of degradation and stability of the ORF0657nH region (SEQ ID NO: 3) was confirmed by Western blot using a goat polyclonal antibody raised against full-length *E. coli* ORF0657n (SEQ ID NO: 28) (data not shown).

An estimate of the amount of ORF0657nH region (SEQ ID NO: 3) from the Western blot and Coomassie gel was ∼360 µg/per mL YEHD medium and the % total protein was estimated to be -50. The amount produced in defined medium was 225 µg/mL culture medium and the % of the total protein was also -50. The amount of ORF0657nH region (SEQ ID NO: 3) compared to the amount of full-length ORF0657n region (SEQ ID NO: 28 without a carboxyl His-Tag) was higher (350 vs 10 µg/mL, respectively) and the % total protein was also higher (50 vs 2.0).

### Example 12: Intracellular Expression of ORF0657nG Region (SEQ ID NO: 44) in S. cerevisiae

Intracellular expression of ORF0657n related polypeptides was evaluated in the following example using a DNA construct encoding ORF0657nG region (SEQ ID NO: 44). ORF0657nG (SEQ ID NO: 44) is a truncated version of SEQ ID NO: 2, that lacks the amino-terminal signal sequence. SEQ ID NO: 44 retains the N-terminal methionine and amino acids 42-645 of SEQ m NO: 2.

Yeast optimized DNA encoding ORF0657nG region (SEQ ID NO: 44) was amplified from pUnkCR1 (Example 7), using the following sense and antisense primers, respectively, 5'GGGGGGATCCCACAAAACAAAATGGCTGAAGAAACTGGTGG3' (SEQ ID NO: 102) and 5'GGGGGGGATCCTTAGTTCTTTCTCTTTCTTGG3' (SEQ ID NO: 103) with flanking *Bam*HI restriction sites. The sense primer contains a 5' untranslated leader sequence and ATG codon, and the antisense primer contains TAA as the stop codon. The resulting 1.8-kb product was gel-isolated and cloned into the TOPO TA vector pCR2.1 according to the manufacturer's directions (INVTTROGEN CORPORATION, Carlsbad, CA), to construct plasmid pCR_iUC-L4. DNA sequence analysis confirmed that a single deletion occurred at the 5'-end.

A clone with the correct sequence was obtained by swapping the 1.3-kb *Hin*dIII-*Hin*dIII fragment of pCR_iUC-S2.2 containing the correct sequence (see Example 11), for the corresponding fragment of pCR_i UC-L4. The 1.3-kb fragment of pCR_iUCS2.2 and the 4.4-kb fragment of pCR_iUC-LA were gel-isolated and ligated. A clone with the desired fragments was selected, pCR_iUC-L, and the appropriate orientation and nucleotide sequence verified by DNA sequencing. Subsequently, the 1.8-kb *Bam*HI fragment was subcloned into pGAL110 to construct piUC-L(-) and the DNA sequence was verified.

Plasmid DNA from piUC-L(-) was used to transform *S*. *cerevisiae* strain 1260 to leucine prototrophy (Leu⁺) as described in Example 9. Several yeast transformants were screened for intracellular production of ORF0657nG region (SEQ ID NO: 44) using the conditions for fermentation and cell breakage described in Example 11. 0.25-0.5 µg of cell-lysate protein were analyzed for the production of ORF0657nG region by Western blot analysis as described in Example 11. Purified *E. coli* ORF0657nH region (SEQ ID NO: 4 with a carboxyl His-Tag) was used as a quantitative standard and a cell lysate from the *E. coli* culture producing ORF0657nG region (SEQ ID NO: 44 with a carboxyl His-Tag), induced to produce ORF0657nG region, was used as a qualitative standard. The samples were subjected to electrophoresis on 4-15% gradient Tris-HCl Criterion gels (BIO-RAD, Hercules, CA) in 1X Tris glycine SDS buffer (BIO-RAD) under reducing and denaturing conditions. The gels were electroblotted onto 0.2-micron PVDF membrane filters (BIO-RAD).

Proteins were immunodetected by Western blot using a polyclonal antibody to purified *E. coli* produced full-length ORF0657n (SEQ ID NO: 28 without the carboxyl His-Tag) as primary antibody and goat anti-mouse IgG (H+L) horseradish peroxidase-linked whole antibody (ZYMED LABORATORIES; South San Francisco, CA) as the secondary antibody. The polyclonal antibody was generated by immunization with purified *E. coli*-produced full-length ORF0657n region (SEQ ID NO: 28). The filters were processed using the WESTERN LIGHTNING^{Tm} Chemiluminesence Reagent Plus kit (PERKIN ELMER, Wellesley, MA).

For comparison purposes, a ORF0657nG region (SEQ ID NO: 44 with a carboxyl His-Tag) was cloned in an *E. coli* expression vector as described in Example 1 and expressed. To produce the ORF0657nG region in *E. coli,* the producing culture was grown overnight in LB broth containing 50 µg1mL kanamycin at 37°C. The next day, 500 µL of overnight culture was used to inoculate 5.0 mL LB broth plus 50 µg per mL kanamycin. The culture was grown at 37°C for approximately 3 hours to an OD₆₀₀ of 0.6. Expression was induced with 1 mM IPTG for 3.5 hours at 37°C. The cells were harvested and the cell pellets were stored at -80°C. The *E. coli* lysate was prepared using Bugbuster Protein Extraction Reagent following the manufacturer's protocol (NOVAGEN, Madison, WI). To estimate protein size, prestained standards between 10 and 250 kDa (BIO-RAD) were run in parallel with the lysates.

From the initial screening, one transformant of yeast strain 1260, designated iUC-L3, was chosen as a good producer of ORF0657nG region after a 72 hour fermentation in complex medium YEHDG in culture tubes as described in Example 11. The major protein detected by either Coomassie staining or by the antibody was -85 kDa and comigrated with *E. coli* expressed ORF0657nG region (SEQ ID NO: 44 with a carboxyl His-Tag). The MW of both the *E. coli* expressed ORF0657nG region and the yeast-expressed ORF0657nG region was larger than the predicted size in our gel electrophoresis system. Detection of the -85-kDa protein was specific; no detection was observed in a cell lysate from a transformant containing vector pGAL110 alone. The average titer of ORF0657nG determined from three Western blot experiments was 30 µg/per mL of YEHDG medium and the % total protein was estimated to be 10.0 (see Example 13 *infra*).

### Example 13: Removing the Cell Wall Sorting Signal Increases Intracellular Expression

The effect of removing the ORF0657n carboxyl cell sortase C-terminal encoding region alone, and in combination with removing the N-terminal signal region was examined using codon-optimized ORF0657n genes SEQ ID NOs: 31, 32, and 45. SEQ ID NO: 31 encodes full-length ORF0657n region. SEQ ID NO: 32 encodes ORF0657nH region , which lacks both an N-terminal signal sequence and C-terminal cell wall sorting region. SEQ ID NO: 45 encodes ORF0657nG region lacking just the signal peptide sequence.

One transformant each expressing full-length ORF0657n region (SEQ ID NO: 28 without the carboxyl His-Tag; transformant rUnkCl), ORF0657nH region (SEQ ID NO: 3; transformant 1-1), and ORF0657nG region (SEQ ID NO: 44 transformant iUC-L3) was fermented, cell lysates were prepared and analyzed as described in Example 11. A representative Western blot is shown in Figure 9. The amount of protein cell lysate loaded on the gel from the different transformants was not the same in order to compensate for the differing levels of expression of the three constructs encoding ORF0657n regions. In each case, at least two different amounts of protein cell lysate from each ORF0657n-containing transformant were loaded.

Typical results demonstrating that ORF0657nH region (SEQ ID NO: 3) was expressed significantly better than full-length ORF0657n region (SEQ ID NO: 28 without the carboxyl His-Tag) or ORF0657nG region (SEQ ID NO: 44) are shown in Figure 9. A substantial signal was detected with only 50 and 100 ng cell lysate protein from the ORF0657nH region - producer as shown in lanes 11 and 12. In contrast, 1.0 and 2.0 µg cell lysate protein of the full-length ORF0657nC region -producer, lanes 7 and 8, respectively, was used. A good signal was detected for ORF0657nG region with less protein, 250 and 500 ng protein were loaded as shown in lanes 14 and 15.

Table 4 presents a comparison of the average titer of each of the three ORFs and % total protein determined from three independent Western blots. Cell lysates were prepared fresh for each Western blot. The averages include results from two independent fermentations. The amount of specific ORF0657n was determined relative to the purified *E. coli* ORF0657nH (SEQ ID NO: 4 plus a carboxyl His-Tag) standard.

**Table 4**

| ORF0657n Produced | Titer, µg/mL YEHDG Medium | % Total Protein |
|---|---|---|
| C | 10.0 | 4.0 |
| G | 30.0 | 10.0 |
| H | 425.0 | 80.0 |

Removal of the signal peptide sequence enhanced % total protein and titer 2.5-3-fold as determined by comparing expression of full-length ORF0657n region (SEQ ID NO: 28 without the carboxyl His-Tag) and ORF0657nG region (SEQ ID NO: 44). Removal of both the signal peptide and cell wall sorting sequence enhanced expression 8-14-fold based compared to removal of just the signal peptide sequence (compare G and H region), and enhanced expression 20-42.5 fold compared to the full length protein (C to H region). The magnitude of the increase in expression upon removal of the cell wall sorting sequence was not expected.

### Example 14: Intracellular Expression of ORF0657nI region in S. cerevisiae

DNA encoding the ORF0657nI region with codons optimized for yeast expression (SEQ ID NO: 33) was amplified from pUnkCR1 (Example 7), using the following sense and antisense primers, respectively, 5'CTCCGGATCCCACAAAACAAAATGGCTGAAGAAACTGGT 3' (SEQ ID NO: 104) and 5'GCTGCCGGGATCCTTATGGGGTTGGCTTAGATGGGGTAG 3' (SEQ ID NO: 105) with flanking *Bam*HI restriction sites (underlined). The sense primer contains a 5' untranslated leader sequence and ATG codon, and the antisense primer contains TAA as the stop codon. The resulting 1.3-kb product was gel-isolated and cloned into pGAL110 (Fig. 5A) to construct piUC-I (Fig. 5B) and the DNA sequence was verified.

Plasmid DNA from piUC-I was used to transform *S. cerevisiae* strain 1260 to leucine prototrophy (Leu⁺) as described in Example 9. Three Leu⁺ transformants were screened for intracellular production of ORF0657nI region (SEQ ID NO: 1) using the small-scale conditions for fermentation (5.0 mL cultures) as described in Example 9.

Production was compared to that of ORF0657nH region (SEQ ID NO: 3) produced by *S. cerevisiae* transformant 1-1 (see Example 11). Cell lysates were prepared as described in Example 9, and 100 and 200 ng of yeast cell-lysate protein were analyzed for the production of ORF0657nI and ORF0657nH by semi-quantitative Western blot analysis as described in Example 11. Purified *E. coli* ORF0657nH (SEQ ID NO: 4 with a carboxyl His-Tag) was used as a quantitative standard. The samples were subjected to electrophoresis on 10-20% gradient Tris-HCl Criterion gels (BIO-RAD, Hercules, CA) in 1X Tris glycine SDS buffer (BIORAD) under reducing and denaturing conditions. The gels were electroblotted onto 0.2-micron PVDF membrane filters (BIO-RAD). Proteins were immunodetected by Western blot using a polyclonal antibody to purified *E. coli* full-length ORF0657n (SEQ ID NO: 28) as primary antibody and goat anti-mouse IgG (H+L) horseradish peroxidase-linked whole antibody (ZYMED LABORATORIES; South San Francisco, CA) as the secondary antibody. The polyclonal antibody was generated by immunization with purified *E. coli*-produced full-length ORF0657n (SEQ ID NO: 28). The filters were processed using the WESTERN LIGHTNING^{Tm} Chemiluminesence Reagent Plus kit (PERKIN ELMER, Wellesley, MA). To estimate protein size, prestained standards between 10 and 250 kDa (BIO-RAD) were run in parallel with the lysates.

Figure 11 presents results from a 72 hour fermentation in complex YEHDG medium in culture tubes. Strong detection of yeast-expressed ORF0657nH region (SEQ ID NO: 3) was obtained as shown in lanes 5, 6, 14, 15, 21 and 22. An -60-kDa protein in the transformants constructed to express ORF0657nI (SEQ ID NO: 1) was also readily detected as shown in lanes 7-12 and 16-21. Yeast-expressed ORF0657nI (SEQ ID NO: 1) comigrated with ORF0657nI (SEQ ID NO: 5 with a carboxyl His-Tag) expressed and purified from *E. coli* (data not presented). The MW of both the *E. coli* expressed ORF0657nI region and the yeast-expressed ORF0657nI region was larger than the predicted size in our gel electrophoresis system. The 60-kDa protein detected in transformants constructed to express ORF0657nI region (SEQ ID NO: 1) was specific; no protein was detected in a cell lysate from a vector control transformant (lanes 4 and 13).

The amount of ORF0657nH region (SEQ ID NO: 3) and ORF0657nI region (SEQ ID NO: 1) on the gel was estimated from the semi-quantitative Western blot by comparison with a known amount of purified ORF0657nH (SEQ ID NO: 4 with a carboxyl His-Tag). The titers and per cent total protein were calculated as the average determined from the duplicate lysates containing the two different amounts of protein on the gel. The volumetric titers of ORF0657nH region (SEQ ID NO: 3) determined from a freshly fermented sample and a frozen cell-lysate were comparable, ∼500 and ∼550 µg/mL culture medium, and 320 µg ORF0657nI region (SEQ ID NO: 1) was produced per mL culture medium (the titer for ORF0657nI region is for transformant I1). The per cent ORF0657nH region (SEQ ID NO: 3) of total protein was estimated as 78% and 80%, respectively, while ORF0657nI (SEQ ID NO: 1) comprised 45% of the total protein.

Thus, ORF0657nI region expressed well in *S. cerevisiae* with a titer about 1.5-fold lower than that of ORF0657nH (SEQ ID NO: 3) and the % total protein was about 1.3-fold lower. Good expression of ORF0657nI region in yeast was confirmed by Coomassie staining of an SDS-PAGE gel containing the cell lysates. Expression of ORF0657nI region in YEDHG medium was scalable: production in either 125 mL or 2L shake flasks was comparable to small-scale expression in culture tubes.

ORF0657nI region (SEQ ID NO: 1) also expressed well in defined 5X minus leucine medium (medium described in example 9). The titer was only ∼1.5-fold lower than the titer in complex medium YEHDG whereas the % total protein was comparable in both media. Integrity of ORF0657nI was good in both media with no significant degradation detected. Production of ORF0657nI was higher at 72 than 96 hours in both complex and 5X minus leucine medium, when tested in shake flask fermentations.

### Example 15: Large-Scale Fermentation of Yeast Strain Producing ORF0657nH (SEO ID NO: 3)

Frozen seed stock of yeast strain 1-1 (strain 1260 transformed with plasmid piUC-S(-); described in Example 11) was used for large-scale fermentation and purification. The vial of seed stock was thawed and 1.0 mL was used to inoculate a 250-mL Erlenmeyer flask containing 50 mL of a leucine-free selective medium (5X Leu⁻ medium, Bayne et al., Gene 66(2):235-44, 1988) containing 4% glucose. The flask was incubated at 28°C, 250 rpm on a rotary shaker. After 24 hours (residual glucose 23.5 g/L), a culture volume of 13 mL was added to a 2-L flask containing 877 mL of the same medium. Again, the flask was incubated at 28°C and agitated at 250 rpm. After 24 hours (residual glucose 4.04 g/L), the contents of the 2-L flask were used to inoculate a 20-L reactor containing a chemically defined medium (Oura, *Biotechnol. Bioengineer.* 16:1197, 1974) that was optimized for the employed strains. This medium contained 20 g/L glucose followed by 25 g/L galactose for induction. The reactor was operated at 28°C, 4.7 L/min, 15 psig, and 300 rpm. Under these conditions, dissolved oxygen levels were maintained at greater than 30% of saturation. Cellular growth was monitored by glucose consumption, optical density (A₆₀₀ nm, 1-cm cuvettes), dry cell weight, galactose utilization and ethanol production. Cultivation continued for 90 hours reaching an A₆₀₀ of 33.9 and a dry cell weight of 17.5 g/L.

The culture was harvested via hollow fiber tangential flow filtration (AMICON Hump01-43 cartridge) using an AMICON DC-10 harvest skid (NELLEPORE, Billerica, MA). The permeate was discarded and the cells were concentrated, diafiltered with PBS, and collected by centrifugation at 8000 rpm, 4°C for 20 minutes using a Sorvall Evolution RC (SLA-3000 rotor). Cells were stored at -70°C.

To evaluate production of ORF0657nH by strain 1-1 in large-scale fermentation, cell lysates were prepared from 10 OD₆₀₀ units of the harvested culture and evaluated as detailed in Example 11 (results not shown). Production of ORF0657nH from a shake flask fermentation (72 hour, complex YEHDG medium) was also assessed. The results of a Western blot analysis (performed as described in Example 11) demonstrated that the protein produced from the large-scale fermentation comigrated with ORF0657nH produced in a shake flask fermentation (results not shown). The titer of ORF0657nH from the large-scale (20-L) fermentation was estimated to be 739 µg per mL and the % total protein was estimated to be 55%, compared to 732 µg/mL and 58% of the total protein for the shake flask fermentation (using the semi-quantitative Western method described in Example 11). These results verify that the production of ORF0657n can be scaled up in yeast.

### Example 16: Protective Immunity of ORF0657n related Polypeptides Produced in Yeast

The ability of yeast produced ORF0657n related polypeptides to provide protective immunity was evaluated by expressing a polypeptide of SEQ ID NO: 3 in yeast. *E coli* expressed full-length ORF0657nC (SEQ ID NO: 28), ORF0657nH (SEQ ID NO: 4 with carboxyl-terminal His-Tag), ORF0657nI (SEQ ID NO: 5 with carboxyl-terminal His-Tag), and adjuvant alone were used as controls.

An ORF0657n related polypeptide of SEQ ID NO: 3 was obtained from yeast and used in an animal model to provide protective immunity. The polypeptide of SEQ ID NO: 3 was expressed as described in Example 11.

Frozen recombinant *S. cerevisiae* cells expressing ORF657nH (SEQ ID NO: 3) were resuspended at 5 mL per gram wet cell weight in 0.2 M MOPS, pH 7.0 with protease inhibitors (EDTA free). A lysate was prepared by four passes through a microfluidizer at 14,000 psi (Microfluidics Model 110S). The Lysate was clarified by centrifugation (10,000 X g, 20 minutes, 2-8°C) followed by coarse filtration (glass fiber pre-filter, Millipore) and fine filtration (0.2µm cellulose acetate, Whatman).

The Clarified Lysate was fractionated on a size-exclusion chromatography (SEC) column (Pharmacia HiPrep 26/60 Sephacryl S-300 HR, mobile phase: 0.2 M MOPS, pH 7.0). Fractions were analyzed by SDS-PAGE with Coomassie detection and Western blotting using ORF0657n protein specific anti-serum (raised against full-length ORF0657n, SEQ ID NO: 28) Fractions that contained product were pooled.

The SEC Product was sterile filtered through 0.2 µm cellulose acetate under aseptic conditions. Purity of the Sterile-Filtered Product was ≥ or higher and 94% by SDS-PAGE and Western blot. Sterile-Filtered Product was formulated at 0.2 mg/ml with AHP and Thimerosal.

The ability of ORF0657n related polypeptides produced in yeast to provide protective immunity is illustrated in Figure 10. Yeast expressed ORF0657nH (SEQ ID NO: 3) provided equivalent protective immunity as *E. coli* expressed polypeptides.

### Example 17: Anamnestic Response in Non-Human Primates

Three groups of Rhesus monkeys were immunized with either yeast produced ORF0657n related polypeptide (ORF0657nH, SEQ ID NO: 3) or *E coli*-expressed ORF0657 related polypeptide (full-length ORF0657n, SEQ ID NO: 28) formulated with or without AHP. The monkeys in the vaccine group received 50 mcg ORF0657n related polypeptides via the intramuscular route.

As shown in Figure 12, the vaccine group animals responded, after a single dose, with a - 3- to 6-fold increase in geometric mean titers over modest pre-existing titers, suggesting an anamnestic response. By contrast, geometric mean titers for animals in the control group remained the same (within the variability of the antibody assay). After a second dose of vaccine, geometric mean post-dose one titers in the vaccine and control groups changed very little compared to the post-dose titers.

To observe the development of antibody responses after only one dose, the group receiving yeast-expressed ORF0657H region (SEQ ID NO: 3) was followed out to 3 months (last time point tested). Antibody titers continued to rise after 9 days reaching levels that were not surpassed even after 2 or 3 doses of vaccine.

These observations suggest that one dose of the vaccine can elicit substantial and lasting antibody responses subsequent to natural priming of the immune system likely due to environmental exposure to *S. aureus.* A survey of pre-existing antibody titers in humans demonstrated the presence of modest antibody titers against ORF0657n in all samples tested (data not shown).

## Claims

1. A polypeptide immunogen comprising an amino acid sequence at least 90% identical to SEQ ID NO: 1, wherein said polypeptide provides protective immunity against *S. aureus* and wherein if one or more additional polypeptide regions are present said additional regions do not provide a carboxyl terminus containing amino acids 609-645 of SEQ ID NO: 2.

2. The polypeptide of claim 1, wherein said polypeptide consists of an amino acid sequence at least 90% identical to SEQ ID NO: 3 or a fragment thereof comprising an amino acid sequence at least 90% identical to SEQ ID NO: 1.

3. The polypeptide of claim 2, wherein said polypeptide consists of an amino acid sequence at least 94% identical to SEQ ID NO: 3, or a fragment thereof comprising an amino acid sequence at least 94% identical to SEQ ID NO: 1.

4. The polypeptide of claim 3, wherein said polypeptide consists of an amino acid sequence at least 94% identical to SEQ ID NO: 1, SEQ ID NO: 3 or SEQ ID NO: 42.

5. The polypeptide of claim 1, wherein said polypeptide is SEQ ID NO: 1, SEQ ID NO:3 or SEQ ID NO:42; or differs from said sequences by up to 25 amino acid alterations.

6. The polypeptide of claim 5, wherein said polypeptide is SEQ ID NO: 1, SEQ ID NO:3 or SEQ ID NO:42, or differs from said sequences by up to 10 amino acid alterations.

7. The polypeptide of claim 6, wherein said polypeptide is SEQ ID NO: 1, SEQ ID NO:3 or SEQ ID NO:42, or differs from said sequences by up to 5 amino acid alterations.

8. The polypeptide of claim 1 wherein said polypeptide is SEQ ID NO: 1, SEQ ID NO:3 or SEQ ID NO:42.

9. The polypeptide of claim 1 wherein said polypeptide consists of the amino acid sequence of SEQ ID NOs 1, 3, 7, 17, 20, or 42 and up to 20 additional amino acids.

10. The polypeptide of claim 5 wherein said polypeptide consists of the amino acid sequence of SEQ ID NOs 7, 17 or 20.

11. The polypeptide of any preceding claim lacking all, or most, other polypeptides with which the polypeptide is naturally associated.

12. An immunogen consisting of the polypeptides of any preceding claim, wherein said immunogen consists of said amino acid sequence and one or more additional regions or moieties covalently joined to said sequence at the carboxyl terminus or amino terminus, wherein each region or moiety is independently selected from a region or moiety having at least one of the following properties: enhances the immune response, facilitates purification, or facilitates polypeptide stability.

13. A composition able to induce a protective immune response in a patient comprising an immune logically effective amount of the immunogen of any one of claims 1-12 and a pharmaceutically acceptable carrier.

14. The composition of claim 13, wherein said composition further comprises an adjuvant.

15. A nucleic acid comprising a recombinant gene comprising a nucleotide sequence encoding the polypeptide immunogen of any one of claims 1-10.

16. The nucleic acid of claim 15, wherein the recombinant gene does not encode a cell signal peptide encoding sequence and does not encode a cell wall sorting signal sequence.

17. The nucleic acid of claim 16, wherein said recombinant gene contains one or more codons optimized for yeast expression.

18. The nucleic acid of claim 17, wherein said nucleotide sequence is at least 50% codon optimized for expression in yeast.

19. The nucleic acid of claim 16, wherein said nucleotide sequence is selected from the group consisting of: SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 46, SEQ ID NO: 47, SEQ ID NO: 48, SEQ ID NO: 49, SEQ ID NO: 50, SEQ ID NO: 51, SEQ ID NO: 52 and SEQ ID NO: 53.

20. The nucleic acid of claim 15 or 19, wherein said nucleic acid is an expression vector.

21. A recombinant cell comprising the nucleic acid of any one of claims 15-20.

22. A method of making a *S. aureus* polypeptide that provides protective immunity comprising the steps of
(a) growing the recombinant cell of claim 21 under conditions wherein the polypeptide of any one of claims 1 to 11 is expressed; and
(b) purifying said polypeptide.

23. The method of claim 22, wherein said recombinant cell is a *S*. *cerevisiae.*

24. An immunogen of any one of claims 1 to 12 for use in a method of treatment of the human body by therapy.

25. The immunogen of claim 24 for providing protective immunity against *S. aureus.*

26. The immunogen of claim 25, wherein said patient is a human.

27. The immunogen of claim 25, wherein said patient is treated prophylactically against *S. aureus* infection.

28. The immunogen of claim 24 for inducing an anamnestic response.

29. The immunogen of claim 28, wherein said anamnestic response results in at least a 3-fold increase in geometric titer over pre-existing titer within 3 days.

30. Use of an immunogen of any one of claims 1 to 12 for the manufacture of a medicament for use in a method as defined in any one of claims 25 to 29.

31. An immunogen of any one of claims 1 to 12 in combination with additional immunogens selected from: one or more additional *S*. *aureus* immunogens; one or more immunogens targeting one or more other *Staphylococcus* organisms; and one or more immunogens targeting other infectious organisms.

## Patentansprüche

1. Ein Polypeptid-Immunogen, umfassend eine Aminosäuresequenz, die zu wenigstens 90% identisch ist mit SEQ ID NR.: 1, wobei das Polypeptid Schutzimmunität gegen *S. aureus* verleiht, und wobei, wenn ein oder mehrere zusätzliche Polypeptidbereiche vorliegen, die zusätzlichen Bereiche kein Carboxylende bereitstellen, das die Aminosäuren 609 - 645 von SEQ ID NR.: 2 enthält.

2. Das Polypeptid gemäß Anspruch 1, wobei das Polypeptid aus einer Aminosäuresequenz, die zu wenigstens 90% identisch ist mit SEQ ID NR.: 3, oder einem Fragment davon, das eine Aminosäuresequenz umfasst, die zu wenigstens 90% identisch ist mit SEQ ID NR.: 1, besteht.

3. Das Polypeptid gemäß Anspruch 2, wobei das Polypeptid aus einer Aminosäuresequenz, die zu wenigstens 94% identisch ist mit SEQ ID NR.: 3, oder einem Fragment davon, das eine Aminosäuresequenz umfasst, die zu wenigstens 94% identisch ist mit SEQ ID NR.: 1, besteht.

4. Das Polypeptid gemäß Anspruch 3, wobei das Polypeptid aus einer Aminosäuresequenz besteht, die zu wenigstens 94% identisch ist mit SEQ ID NR.: 1, SEQ ID NR.: 3 oder SEQ ID NR.: 42.

5. Das Polypeptid gemäß Anspruch 1, wobei das Polypeptid SEQ ID NR.: 1, SEQ ID NR.: 3 oder SEQ ID NR.: 42 ist oder sich von diesen Sequenzen um bis zu 25 Aminosäure-Änderungen unterscheidet.

6. Das Polypeptid gemäß Anspruch 5, wobei das Polypeptid SEQ ID NR.: 1, SEQ ID NR.: 3 oder SEQ ID NR.: 42 ist oder sich von diesen Sequenzen um bis zu 10 Aminosäure-Änderungen unterscheidet.

7. Das Polypeptid gemäß Anspruch 6, wobei das Polypeptid SEQ ID NR.: 1, SEQ ID NR.: 3 oder SEQ ID NR.: 42 ist oder sich von diesen Sequenzen um bis zu 5 Aminosäure-Änderungen unterscheidet.

8. Das Polypeptid gemäß Anspruch 1, wobei das Polypeptid SEQ ID NR.: 1, SEQ ID NR.: 3 oder SEQ ID NR.: 42 ist.

9. Das Polypeptid gemäß Anspruch 1, wobei das Polypeptid aus der Aminosäuresequenz SEQ ID NR.: 1, 3, 7, 17, 20 oder 42 und bis zu 20 zusätzlichen Aminosäuren besteht.

10. Das Polypeptid gemäß Anspruch 5, wobei das Polypeptid aus der Aminosäuresequenz SEQ ID NR.: 7, 17 oder 20 besteht.

11. Das Polypeptid gemäß einem vorhergehenden Anspruch, dem sämtliche oder die meisten anderen Polypeptide fehlen, mit dem das Polypeptid natürlicherweise verbunden ist.

12. Ein Immunogen, bestehend aus dem Polypeptid gemäß einem vorhergehenden Anspruch, wobei das Immunogen aus der Aminosäuresequenz und einem oder mehreren zusätzlichen Bereichen oder Resten, kovalent an die Sequenz am Carboxylende oder Aminoende gebunden, besteht, wobei jeder Bereich oder Rest unabhängig ausgewählt ist aus einem Bereich oder Rest mit wenigstens einem der folgenden Eigenschaften: immunreaktionsfördemd, reinigungsunterstützend oder polypeptidstabilitätsfördernd.

13. Eine Zusammensetzung, die eine schützende Immunreaktion bei einem Patienten auslösen kann, umfassend eine immunologisch wirksame Menge des Immunogens gemäß einem der Ansprüche 1 - 12 und einen pharmazeutisch annehmbaren Träger.

14. Die Zusammensetzung gemäß Anspruch 13, wobei die Zusammensetzung ferner einen Hilfsstoff umfasst.

15. Eine Nukleinsäure, umfassend ein rekombinantes Gen, umfassend eine Nukleotidsequenz, die das Polypeptid-Immunogen gemäß einem der Ansprüche 1-10 kodiert.

16. Die Nukleinsäure gemäß Anspruch 15, wobei das rekombinante Gen keine zellsignalpeptidkodierende Sequenz kodiert und keine Zellwandsortierungssignalsequenz kodiert.

17. Die Nukleinsäure gemäß Anspruch 16, wobei das rekombinante Gen ein oder mehrere Kodone enthält, die für die Hefeexpression optimiert sind.

18. Die Nukleinsäure gemäß Anspruch 17, wobei die Nukleinsäuresequenz zu wenigstens 50% kodonoptimiert ist für die Expression in Hefe.

19. Die Nukleinsäure gemäß Anspruch 16, wobei die Nukleotidsequenz ausgewählt ist aus der Gruppe, bestehend aus: SEQ ID NR.: 30, SEQ ID NR.: 31, SEQ ID NR.: 32, SEQ ID NR.: 33, SEQ ID NR.: 34, SEQ ID NR.: 35, SEQ ID NR.: 36, SEQ ID NR.: 37, SEQ ID NR.: 38, SEQ ID NR.: 39, SEQ ID NR.: 40, SEQ ID NR.: 41, SEQ ID NR.: 46, SEQ ID NR.: 47, SEQ ID NR.: 48, SEQ ID NR.: 49, SEQ ID NR.: 50, SEQ ID NR.: 51, SEQ ID NR.: 52 und SEQ ID NR.: 53.

20. Die Nukleinsäure gemäß Anspruch 15 oder 19, wobei die Nukleinsäure ein Expressionsvektor ist.

21. Eine rekombinante Zelle, die die Nukleinsäure gemäß einem der Ansprüche 15-20 umfasst.

22. Ein Verfahren zur Herstellung eines *S. aureus*-Polypeptids, welches Schutzimmunität verleiht, umfassend die Schritte:
(a) Kultivieren der rekombinanten Zelle gemäß Anspruch 21 unter Bedingungen, bei denen das Polypeptid gemäß einem der Ansprüche 1 bis 11 exprimiert wird, und
(b) Reinigen des Polypeptids.

23. Das Verfahren gemäß Anspruch 22, wobei die rekombinante Zelle *S. cerevisiae* ist.

24. Ein Immunogen gemäß einem der Ansprüche 1 bis 12 zur Verwendung bei einem Verfahren zur therapeutischen Behandlung des menschlichen Körpers.

25. Das Immunogen gemäß Anspruch 24 zur Verleihung von Schutzimmunität gegen *S. aureus.*

26. Das Immunogen gemäß Anspruch 25, wobei der Patient ein Mensch ist.

27. Das Immunogen gemäß Anspruch 25, wobei der Patient prophylaktisch gegen eine *S. aureus*-Infektion behandelt wird.

28. Das Immunogen gemäß Anspruch 24 zur Herbeiführung einer anamnestischen Reaktion.

29. Das Immunogen gemäß Anspruch 28, wobei die anamnestische Reaktion zu einer wenigstens 3-fachen Erhöhung des geometrischen Titers gegenüber dem zuvor bestehenden Titer innerhalb von 3 Tagen führt.

30. Verwendung eines Immunogens gemäß einem der Ansprüche 1 bis 12 zur Herstellung eines Medikaments zur Verwendung bei einem wie in einem der Ansprüche 25 bis 29 definierten Verfahren.

31. Ein Immunogen gemäß einem der Ansprüche 1 bis 12 in Kombination mit zusätzlichen Immunogenen, ausgewählt aus: ein oder mehrere zusätzliches *S*. *aureus*-Immunogene; ein oder mehrere Immunogene, die auf einen oder mehrere andere *Staphylococcus*-Organismen ausgerichtet sind; und ein oder mehrere Immunogene, die auf andere infektiöse Organismen ausgerichtet sind.

## Revendications

1. Immunogène polypeptide comprenant une séquence d'acides aminés au moins 90% identique à la SEQ ID NO:1, où ledit polypeptide offre une immunité protectrice contre le *S. aureus* et où, si une ou plusieurs régions de polypeptide supplémentaires sont présentes, lesdites régions supplémentaires ne procurent pas une terminaison carboxyle contenant les acides aminés 609-645 de la SEQ ID NO:2.

2. Polypeptide selon la revendication 1, où ledit polypeptide est constitué d'une séquence d'acides aminés au moins 90% identique à la SEQ ID NO:3 ou à un fragment de celle-ci comprenant une séquence d'acides aminés au moins 90% identique à la SEQ ID NO:1.

3. Polypeptide selon la revendication 2, où ledit polypeptide est constitué d'une séquence d'acides aminés au moins 94% identique à la SEQ ID NO:3 ou à un fragment de celle-ci comprenant une séquence d'acides aminés au moins 94% identique à la SEQ ID NO:1.

4. Polypeptide selon la revendication 3, où ledit polypeptide est constitué d'une séquence d'acides aminés au moins 94% identique à la SEQ ID NO:1, à la SEQ ID NO:3 ou à la SEQ ID NO:42.

5. Polypeptide selon la revendication 1, où ledit polypeptide est la SEQ ID NO:1, la SEQ ID NO:3 ou la SEQ ID NO:42 ou diffère desdites séquences par jusqu'à 25 modifications d'acides aminés.

6. Polypeptide selon la revendication 5, où ledit polypeptide est la SEQ ID NO:1, la SEQ ID NO:3 ou la SEQ ID NO:42, ou diffère desdites séquences par jusqu'à 10 modifications d'acides aminés.

7. Polypeptide selon la revendication 6, où ledit polypeptide est la SEQ ID NO:1, la SEQ ID NO:3 ou la SEQ ID NO:42 ou diffère desdites séquences par jusqu'à 5 modifications d'acides aminés.

8. Polypeptide selon la revendication 1, où ledit polypeptide est la SEQ ID NO:1, la SEQ ID NO:3 ou la SEQ ID NO:42.

9. Polypeptide selon la revendication 1, où ledit polypeptide est constitué de la séquence d'acides aminés des SEQ ID NOs 1, 3, 7, 17, 20 ou 42 et de jusqu'à 20 acides aminés supplémentaires.

10. Polypeptide selon la revendication 5, où ledit polypeptide est constitué de la séquence d'acides aminés des SEQ ID NOs 7,17 ou 20.

11. Polypeptide selon l'une quelconque des revendications précédentes, manquant de la totalité, ou de la majeure partie, d'autres polypeptides avec lesquels le polypeptide est naturellement associé.

12. Immunogène constitué du polypeptide selon l'une quelconque des revendications précédentes, où ledit immunogène est constitué de ladite séquence d'acides aminés et d'une ou de plusieurs régions ou fractions supplémentaires jointes par covalence à ladite séquence au niveau de la terminaison carboxyle ou de la terminaison amino, dans lequel chaque région ou fraction est indépendamment choisie parmi une région ou une fraction présentant au moins une des propriétés suivantes: augmente la réponse immunitaire, facilite la purification ou facilite la stabilité du polypeptide.

13. Composition capable d'induire une réponse immunitaire protectrice chez un patient comprenant une quantité immunologiquement efficace de l'immunogène selon l'une quelconque des revendications 1-12 et un support pharmaceutiquement acceptable.

14. Composition selon la revendication 13, où ladite composition comprend en outre un adjuvant.

15. Acide nucléique comprenant un gène recombinant comprenant une séquence de nucléotides codant pour l'immunogène polypeptide selon l'une quelconque des revendications 1-10.

16. Acide nucléique selon la revendication 15, dans lequel le gène recombinant ne code pas pour une séquence codant pour un peptide signal cellulaire et ne code pas pour une séquence signal de tri de paroi cellulaire.

17. Acide nucléique selon la revendication 16, dans lequel ledit gène recombinant contient un ou plusieurs codons optimisés pour une expression dans une levure.

18. Acide nucléique selon la revendication 17, dans lequel ladite séquence de nucléotides est au moins 50% de codon optimisé pour une expression dans une levure.

19. Acide nucléique selon la revendication 16, dans lequel ladite séquence de nucléotides est choisie dans le groupe constitué de: SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:46, SEQ ID NO:47, SEQ ID NO:48, SEQ ID NO:49, SEQ ID NO:50, SEQ ID NO:51, SEQ ID NO:52 et SEQ ID NO:53.

20. Acide nucléique selon la revendication 15 ou 19, où ledit acide nucléique est un vecteur d'expression.

21. Cellule recombinante comprenant l'acide nucléique selon l'une quelconque des revendications 15-20.

22. Procédé pour la fabrication d'un polypeptide de *S. aureus* qui offre une immunité protectrice comprenant les étapes:
(a) de croissance de la cellule recombinante selon la revendication 21 dans des conditions dans lesquelles le polypeptide selon l'une quelconque des revendications 1 à 11 est exprimé; et
(b) de purification dudit polypeptide.

23. Procédé selon la revendication 22, dans lequel ladite cellule recombinante est un *S. cerevisiae.*

24. Immunogène selon l'une quelconque des revendications 1 à 12, pour une utilisation dans un procédé de traitement du corps humain par une thérapie.

25. Immunogène selon la revendication 24, pour offrir une immunité protectrice contre le *S. aureus.*

26. Immunogène selon la revendication 25, où ledit patient est un humain.

27. Immunogène selon la revendication 25, où ledit patient est traité prophylactiquement contre une infection de *S. aureus.*

28. Immunogène selon la revendication 24, pour induire une réponse immunitaire amnestique.

29. Immunogène selon la revendication 28, dans lequel la réponse immunitaire amnestique conduit à une augmentation d'au moins 3 fois dans le titre géométrique par rapport au titre pré-existant dans les 3 jours.

30. Utilisation d'un immunogène selon l'une quelconque des revendications 1 à 12 pour la fabrication d'un médicament pour une utilisation dans un procédé tel que défini dans l'une quelconque des revendications 25 à 29.

31. Immunogène selon l'une quelconque des revendications 1 à 12 en combinaison avec des immunogènes supplémentaires choisis parmi: un ou plusieurs immunogènes de *S. aureus* supplémentaires; un ou plusieurs immunogènes ciblant un ou plusieurs autres organismes *Staphylococcus;* et un ou plusieurs immunogènes ciblant d'autres organismes infectieux.
